(19) Europäisches Patentamt
European Patent Office
Office européen des brevets

(11) **EP 4 509 141 A1**

(12) **EUROPEAN PATENT APPLICATION**
published in accordance with Art. 153(4) EPC

(43) Date of publication:
**19.02.2025 Bulletin 2025/08**

(21) Application number: **23787722.0**

(22) Date of filing: **11.04.2023**

(51) International Patent Classification (IPC):
***A61K 47/60** (2017.01)* ***A61K 47/68** (2017.01)*
***A61K 47/65** (2017.01)* ***C07K 16/00** (2006.01)*
***A61P 35/00** (2006.01)*

(52) Cooperative Patent Classification (CPC):
**A61K 31/4745; A61K 45/00; A61K 47/60; A61K 47/65; A61K 47/68; A61P 35/00; C07K 16/00**

(86) International application number:
**PCT/CN2023/087691**

(87) International publication number:
**WO 2023/198079 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.04.2022 CN 202210382003**

(71) Applicant: **Bio-Thera Solutions, Ltd.**
**Guangzhou, Guangdong 510530 (CN)**

(72) Inventors:
- **TANG, Weijia**
  **Guangzhou, Guangdong 510530 (CN)**
- **SONG, Shuqiang**
  **Guangzhou, Guangdong 510530 (CN)**
- **MEI, Xingxing**
  **Guangzhou, Guangdong 510530 (CN)**
- **CHAO, Chuanqi**
  **Guangzhou, Guangdong 510530 (CN)**
- **YU, Jin-Chen**
  **Guangzhou, Guangdong 510530 (CN)**
- **LI, Shengfeng**
  **Guangzhou, Guangdong 510530 (CN)**

(74) Representative: **Engelhard, Markus**
**Boehmert & Boehmert**
**Anwaltspartnerschaft mbB**
**Pettenkoferstrasse 22**
**80336 München (DE)**

Remarks:
The complete document including Reference Table(s) and the Sequence Listing(s) can be downloaded from the EPO website

(54) **METHOD FOR TREATING HER2-POSITIVE SOLID TUMOR**

(57) Disclosed is a method for treating a HER2-positive solid tumor, which comprises administering to a patient with a HER2-positive solid tumor an effective amount of an antibody-drug conjugate or administering to a patient with a HER2-positive solid tumor an effective amount of an antibody-drug conjugate and another HER2-targeted antibody.

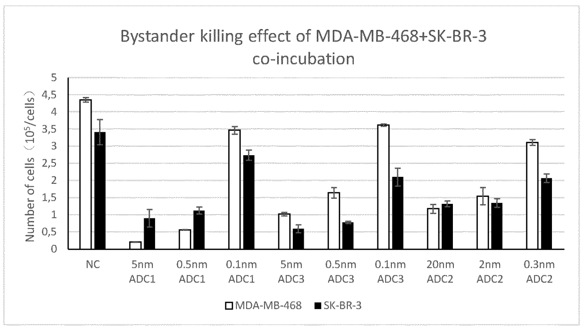


FIG. 1

EP 4 509 141 A1

## Description

### TECHNICAL FIELD

The present invention relates to the field of pharmaceutical therapeutic methods, in particular to a method for treating HER2-positive solid tumors with antibody drug conjugate.

### BACKGROUND

The targeted treatment of cancer, immunodeficiency, infectious diseases, etc. is currently the main focus of precision medicine. Over the years, there have been numerous reports of using cell surface receptor binding molecules as drug delivery vehicles to form conjugates with cytotoxin molecules for targeted delivery of cytotoxin molecules to attack various pathogenic cells (Allen, T.M. and Cullis, P.R., 2004 Science, 303(5665), 1818-22; Hu, Q.Y., et al. (2016), Chem Soc Rev 45(6): 1691-1719).

An antibody-drug conjugate consists of three parts: an antibody, a cytotoxin molecule, and a linker in between for linking the two (Thomas, A., et al. (2016), Lancet Oncol 17(6): e254-e262). The three components each serve unique functions: the antibody should be able to specifically bind to tumor cells, the cytotoxin molecule should be sufficiently active and have a broad spectrum for the tumor cells, and the linker should be uniquely functional, stable in the blood circulation and effective in releasing the cytotoxin molecule upon reaching the tumor cells (Chari, R.V. (2008), Acc Chem Res 41(1): 98-107). Good clinical results can be produced only when the three components are reasonably constructed (Singh, S.K., et al. (2015), Pharm Res 32(11): 3541-3571; Hamilton, G.S. (2015), Biologicals 43(5): 318-332).

### SUMMARY

The present invention provides a method for treating a HER2-positive solid tumor, which comprises administering to a patient with a HER2-positive solid tumor an effective amount of an antibody-drug conjugate; or use of an antibody-drug conjugate in the preparation of a pharmaceutical composition for treating a HER2-positive solid tumor; or use of an antibody-drug conjugate in the treatment of a HER2-positive solid tumor. The antibody-drug conjugate has a structure shown as formula I, or a stereoisomer, or a pharmaceutically acceptable salt or solvate thereof:

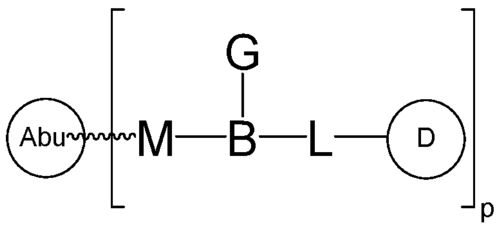

wherein,

Abu is an antibody or an antigen-binding unit thereof that binds to HER2;

D is drug,

M is

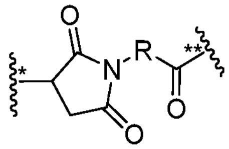

,

wherein * links to Abu, ** links to B, and R is selected from: $-(CH_2)_r-$, - $(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, - $(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$ and - $(CH_2CH_2O)_rC(O)NR^m(CH_2)_r-$; wherein each $R^m$ is

independently H, C1-C6 alkyl, C3-C8 carbocyclyl, phenyl or benzyl, and each r is independently 1, 2, 3, 4, 5, 6, 7, 8, 9 or 10;

B is

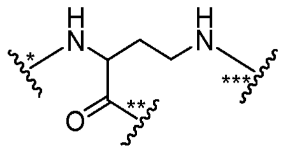

,

or is

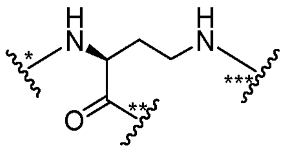

;

wherein * links to M, ** links to L, and *** links to G;

L is-$(AA)_i(FF)_f$-, wherein AA is an amino acid or polypeptide, and i is 1, 2, 3, 4, 5, 6, 7, 8, 9, 10, 11, 12, 13, 14, 15, 16, 17, 18, 19 or 20;

FF is

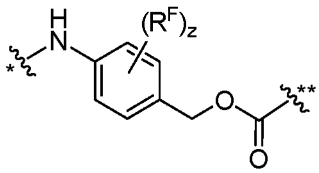

,

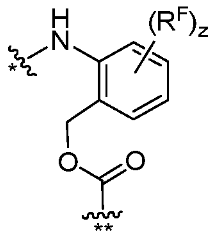

or

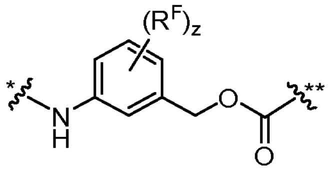

,wherein $R^F$ is C1-C6 alkyl, C1-C6 alkoxy, $-NO_2$ or halogen; z is 0, 1, 2, 3 or 4; wherein * links to AA, and ** links to D;

G is

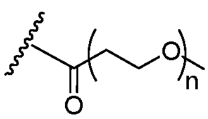

,

wherein n is 1 -24;

p is 1-10.

In one or more embodiments, the drug is an anti-cancer drug, a cytotoxic drug, a cell differentiation factor, a stem cell trophic factor, a steroid drug, a drug for treating autoimmune diseases, an antiinflammatory drug or a drug for treating infectious diseases.

In one or more embodiments, the drug is a tubulin inhibitor, a DNA damaging agent, or a DNA topoisomerase inhibitor.

In one or more embodiments, the tubulin inhibitor is selected from dolastatin, auristatins and maytansinoids (maytansine).

In one or more embodiments, the drug is an auristatin, selected from monomethyl auristatin E (MMAE), monomethyl auristatin F (MMAF), or auristatin F (AF).

In one or more embodiments, the drug is a DNA damaging agent, e.g., a calicheamicin, a duocarmycin, or the anthramycin derivative PBD (pyrrolobenzodiazepine).

In one or more embodiments, the drug is a DNA topoisomerase inhibitor or a salt thereof, e.g., irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, N [2-(dimethylamino)ethyl]-4-acridinecarboxamide dihydrochloride, or N-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan.

In one or more embodiments, the drug is

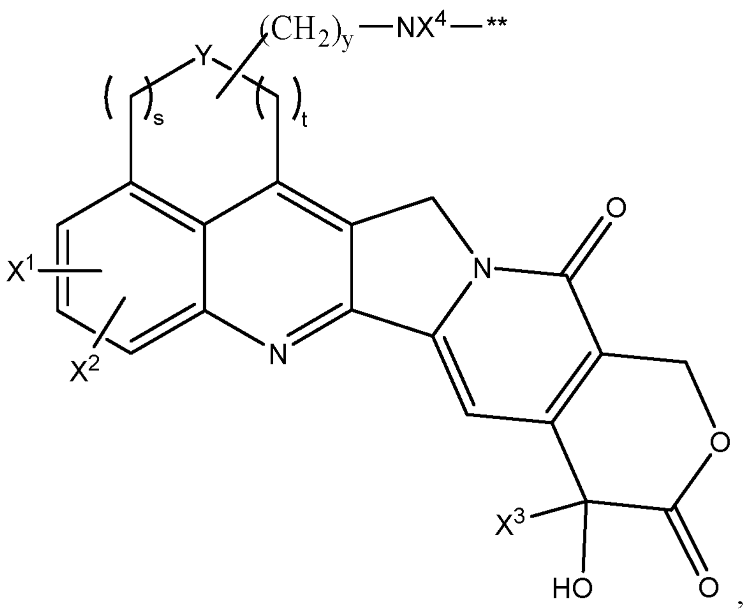

wherein
$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy, halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl,

carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-aryle ne-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_q$ $C(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)$ $NR^n(CH_2CH_2O)_q-CH_2-CH_3$ or $-(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** links to L;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.
In one or more embodiments, the heterocyclyl is azetidine, niverazine, morpholine, pyrrolidine, piperidine, imidazole, thiazole, oxazole or pyridine.
In one or more embodiments, the amino-protecting group is formyl, acetyl, trityl, t-butoxycarbonyl, benzyl, or p-methoxybenzyloxycarbonyl.
In one or more embodiments, the drug is

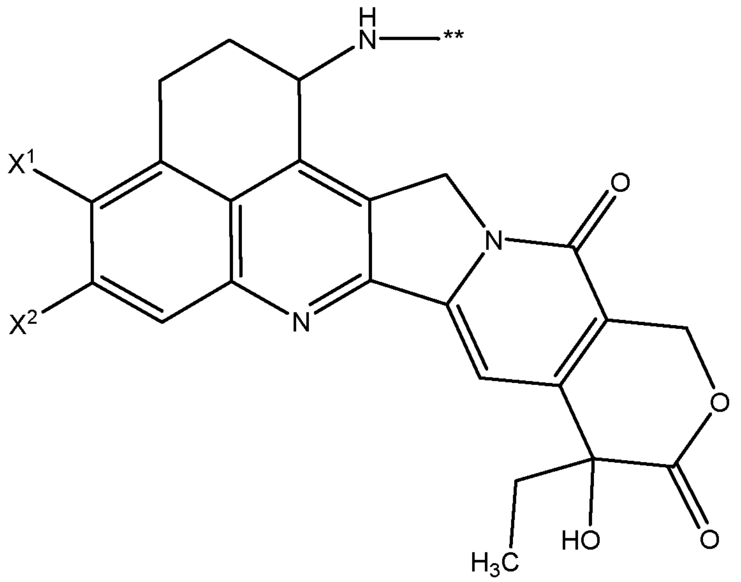

,

wherein $X^1$ and $X^2$ are each independently C1-C6 alkyl, halogen, or -OH; ** links to L.

In one or more embodiments, the drug is

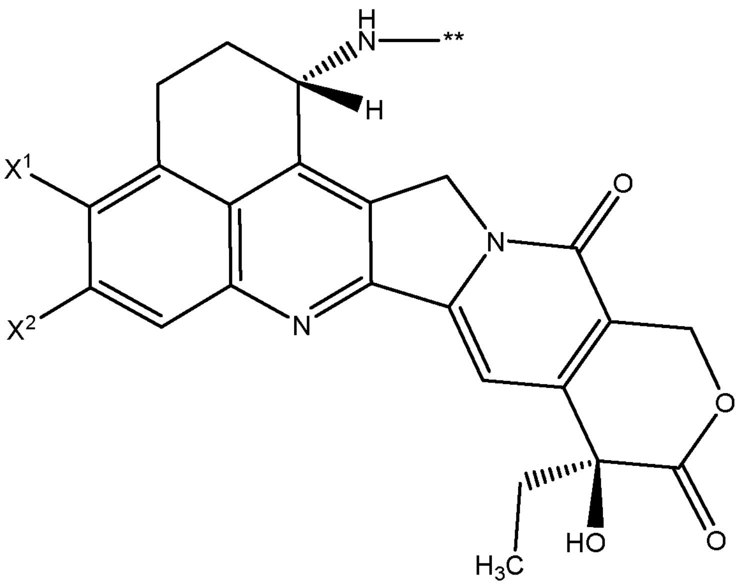

,

wherein $X^1$ and $X^2$ are each independently C1-C6 alkyl, halogen, or -OH; ** links to L.

In one or more embodiments, $X^1$ and $X^2$ are each $-CH_3$

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is $-(CH_2)_r-$, wherein r is 1 or 5.

In one or more embodiments, R is $-(CH_2)-$.

In one or more embodiments, AA is selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, AA is Val-Cit, i is 1.

In one or more embodiments, FF is

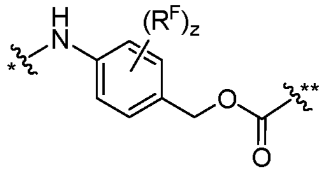 , 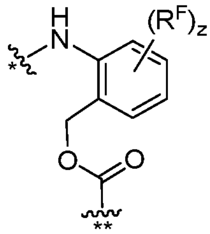

or

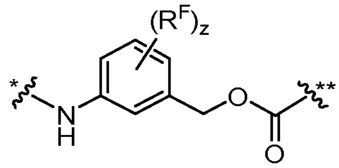 ,

wherein $R^F$ is C1-C6 alkyl, C1-C6 alkoxy, $-NO_2$, or halogen, wherein * links to AA, and ** links to D.

In one or more embodiments, the halogen is F, z is 0, 1, 2, 3 or 4.

In one or more embodiments, $R^F$ is $-CH_3$, F, $-NO_2$ or $-OCH_3$.

In one or more embodiments, z is 0.

In one or more embodiments, z is 1 or 2.

In one or more embodiments, FF is

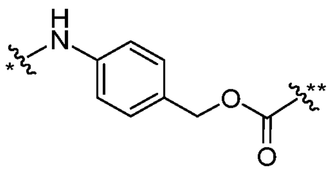 , 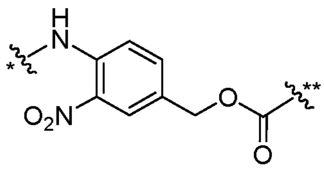 ,

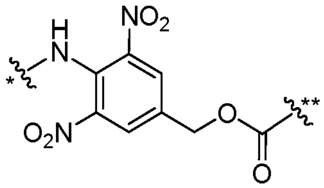 , 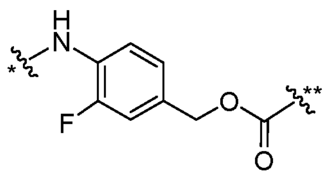 , 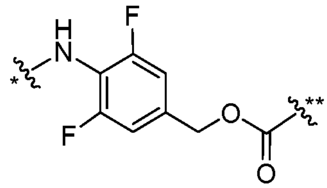 ,

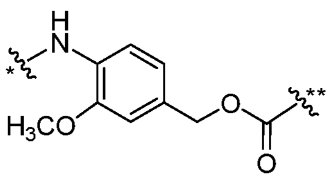 , 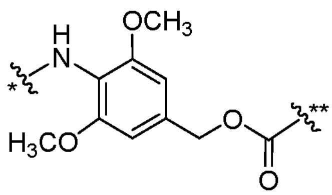 , 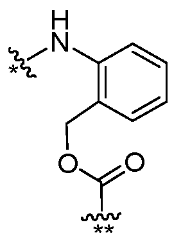 , 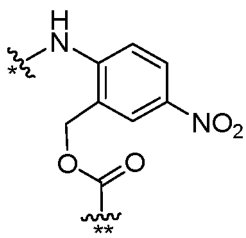 ,

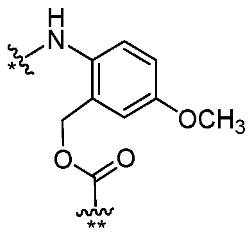 , 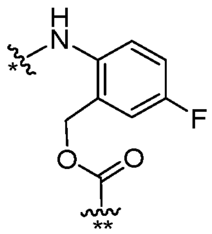 , 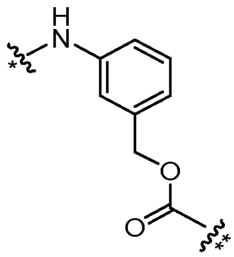 , 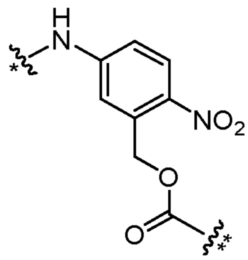 ,

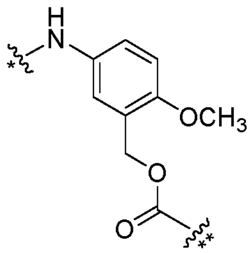 or 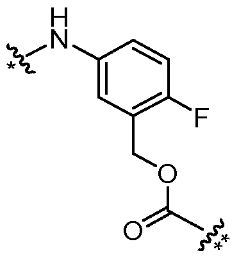 ;

wherein * links to AA, and ** links to D.

In one or more embodiments, f is 1.

In one or more embodiments, FF is

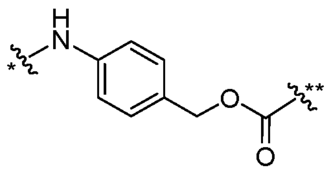 ,

and f is 1, wherein * links to AA, and ** links to D.

In one or more embodiments, L is

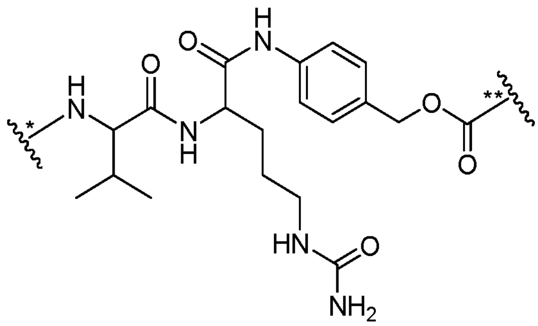 ,

wherein * links to B, and * * links to D

In one or more embodiments, L is

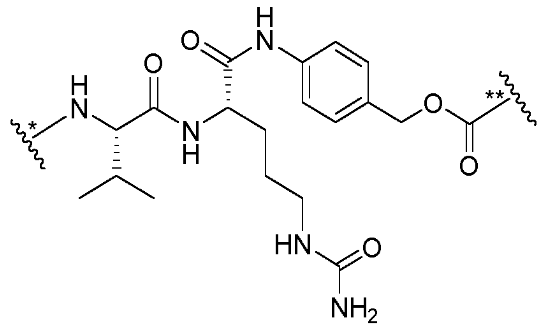

,

wherein * links to B, and ** links to D.

In one or more embodiments, G is

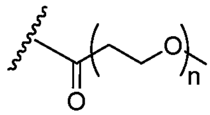

,

and n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, the Abu is trastuzumab.

In one or more embodiments, the antibody-drug conjugate has a structure shown as formula I-1, I-2, I-3, I-4, I-5, I-6, I-7, I-8, I-9, I-10, or I-11, or a stereoisomer, or a pharmaceutically acceptable salt or solvate thereof:

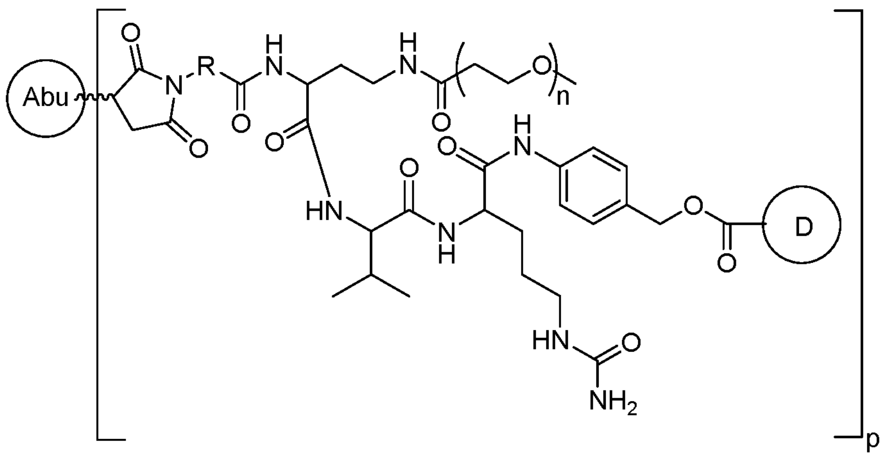

formula I-1

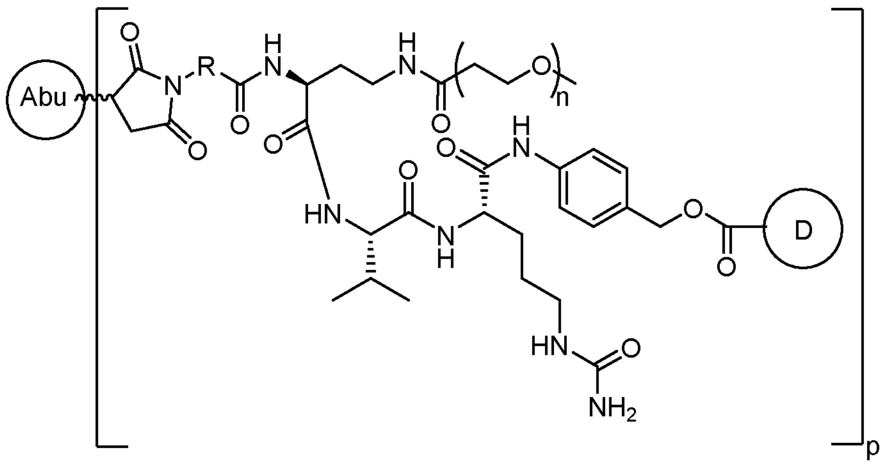

formula I-2

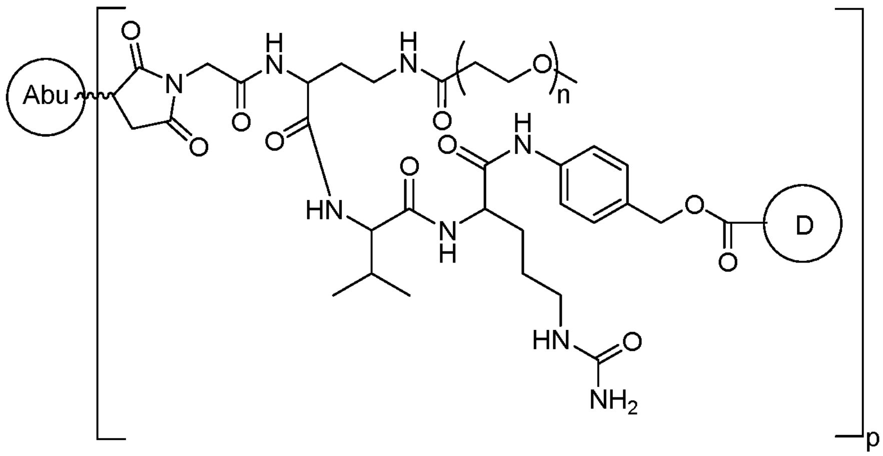

formula I-3

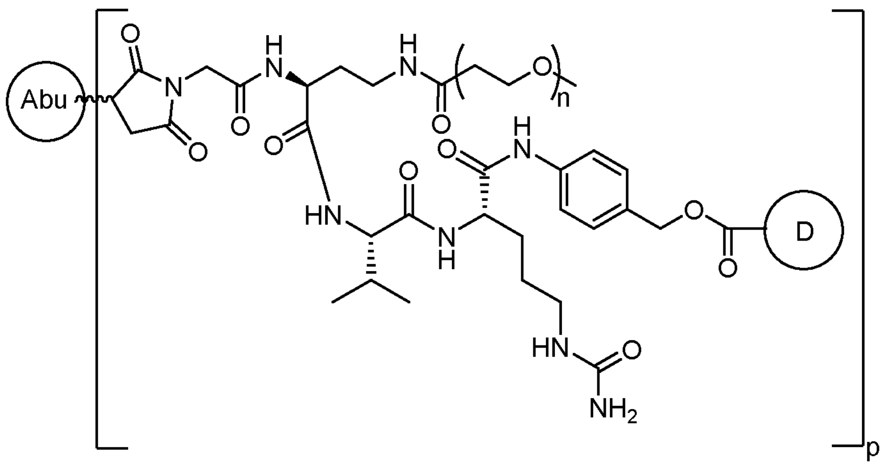

formula I-4

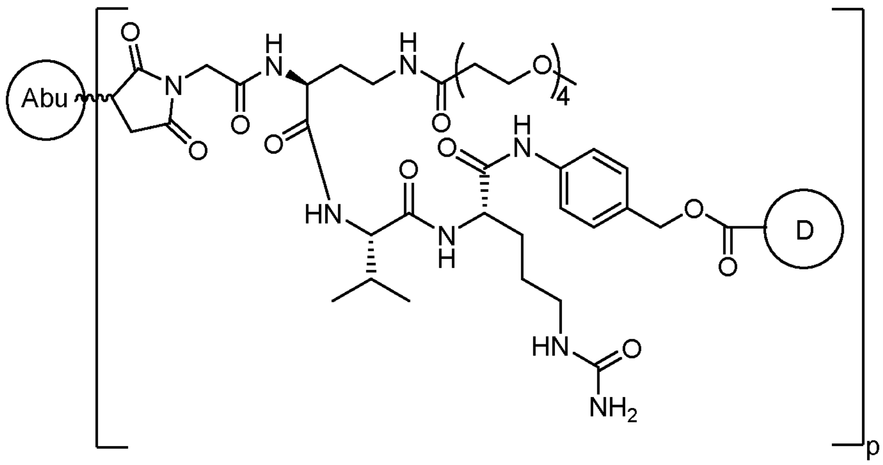

formula I-5

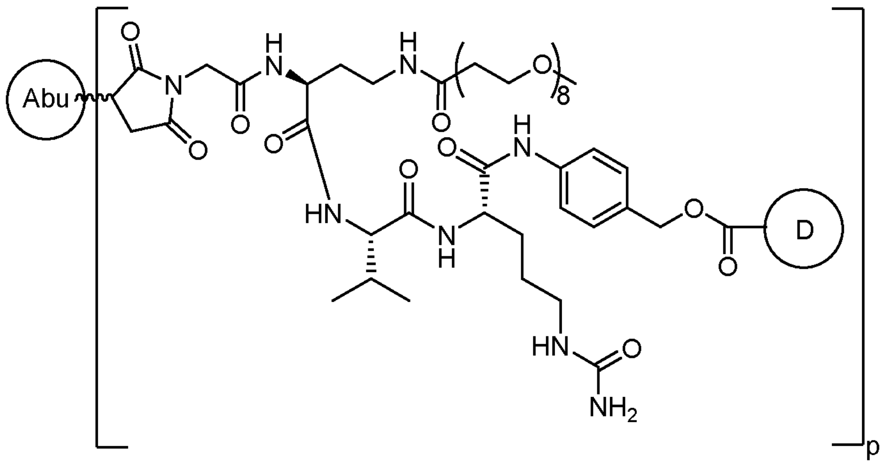

formula I-6

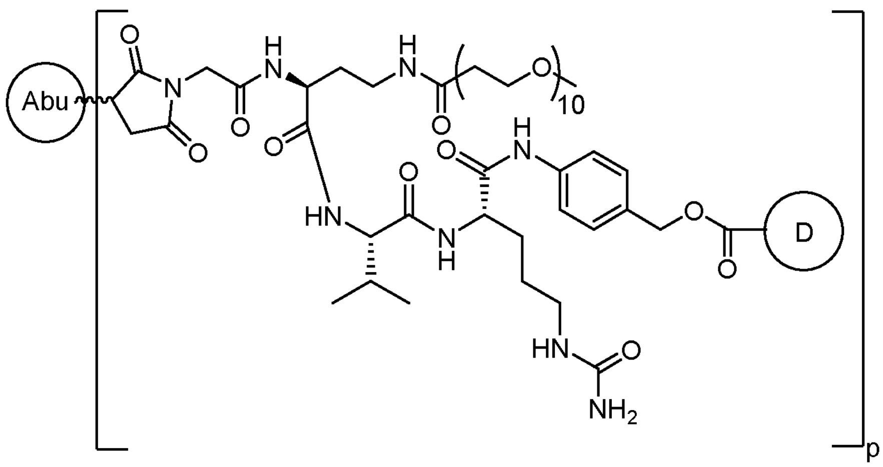

formula I-7

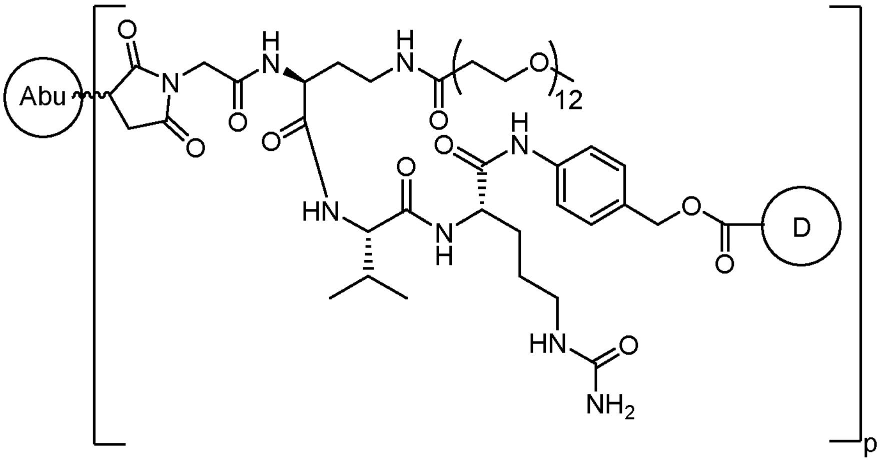

formula I-8

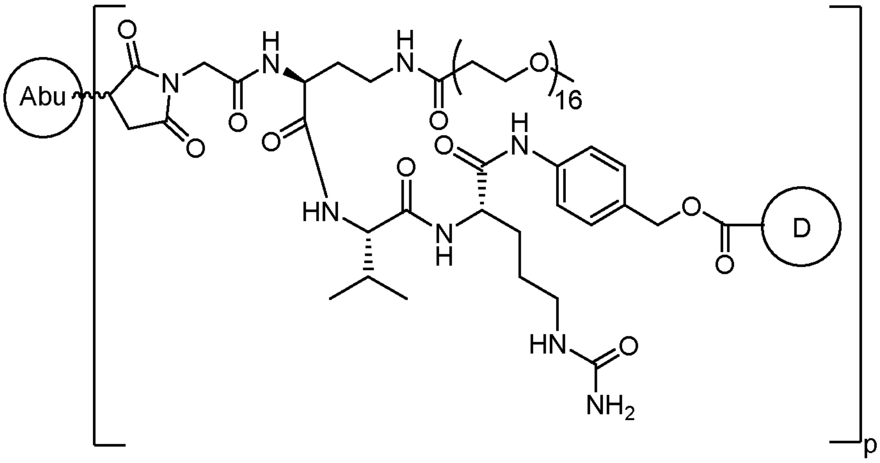

formula I-9

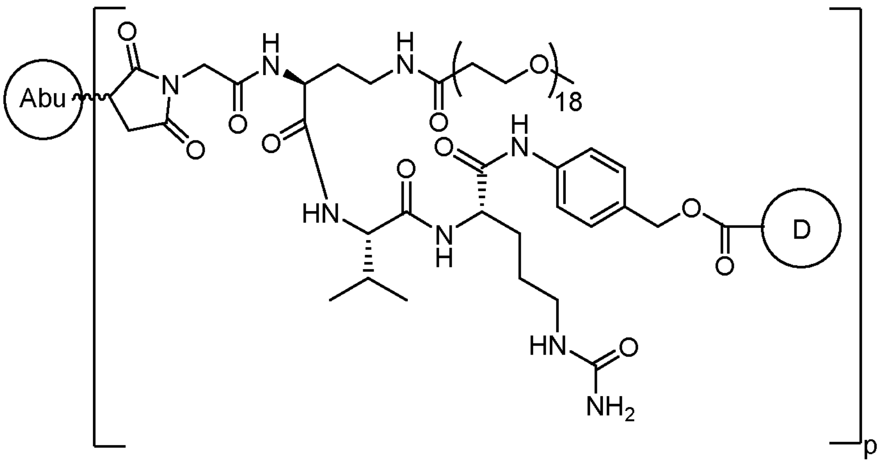

formula I-10

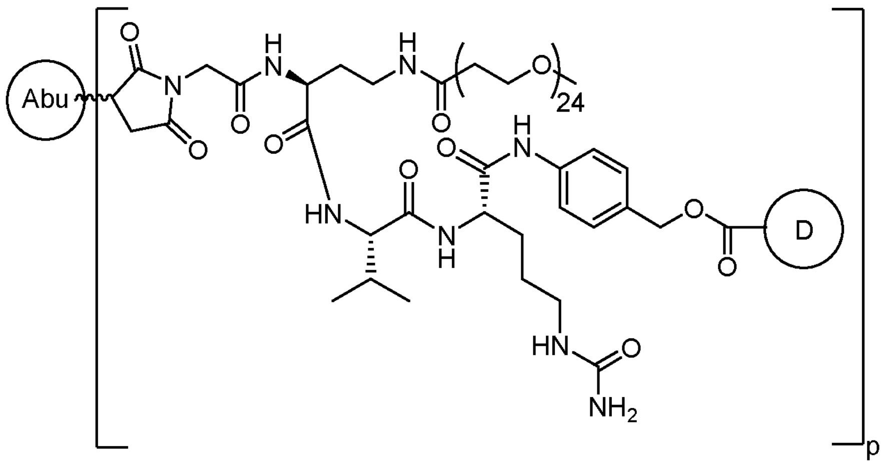

formula I-11

wherein

Abu is an antibody or an antigen-binding unit thereof that binds to HER2;

R is selected from: $-(CH_2)_r-$, $-(CHR^m)r-$, C3-C8 carbocyclyl, $-O-(CH_2)_r-$, arylene, $-(CH_2)_r$-arylene-, - arylene-$(CH_2)r-$, $-(CH_2)_r$-(C3-C8 carbocyclyl)-, -(C3-C8 carbocyclyl)-$(CH_2)_r-$, C3-C8 heterocyclyl, - $(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$ and-$(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10; or R is $-(CH_2)_r-$, or r is 1 or 5;

D is a drug;

n is an integer from 1-24; or n is 4-12;

p is 1-10, e.g., 1, 2, 3, 4, 5, 6, 7, 8, 9 or 10.

In one or more embodiments, the drug is an anti-cancer drug, a cytotoxic drug, a cell differentiation factor, a stem cell trophic factor, a steroid drug, a drug for treating autoimmune diseases, an antiinflammatory drug or a drug for treating infectious diseases.

In one or more embodiments, the drug is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF, or AF.

In one or more embodiments, the drug is a DNA damaging agent, e.g., a calicheamicin, a duocarmycin, the anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, or N-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan.

In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, the drug is

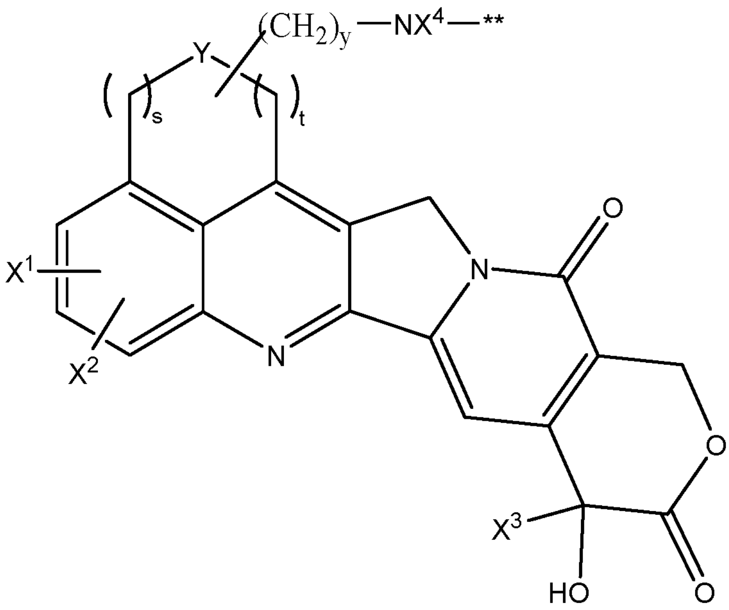
,

wherein
$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl,

carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-0-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

* * is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or p-methoxybenzyloxycarbonyl.

In one or more embodiments, the drug is

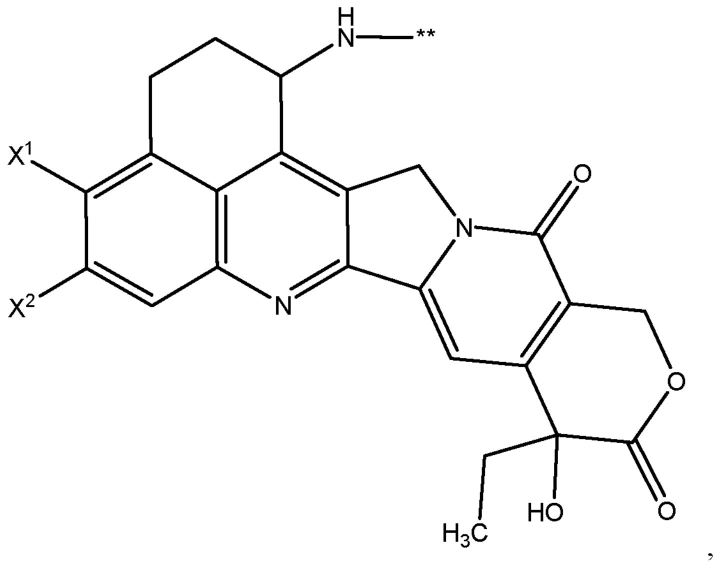

wherein $X^1$ and $X^2$ are each independently C1-C6 alkyl, halogen, or -OH; ** is point of connection.

In one or more embodiments, the drug is

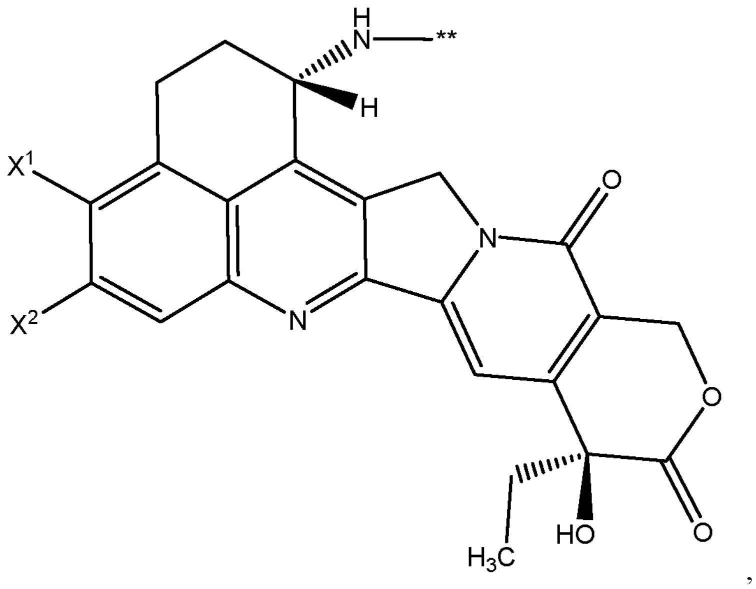

wherein $X^1$ and $X^2$ are each independently C1-C6 alkyl, halogen, or -OH; ** is point of connection.

In one or more embodiments, C1-C6 alkyl is $-CH_3$

In one or more embodiments, halogen is F.

In one or more embodiments, $X^1$ and $X^2$ are each independently $-CH_3$, F or -OH.

In one or more embodiments, $X^1$ and $X^2$ are each independently $-CH_3$

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently F or $-CH_3$

In one or more embodiments, $X^1$ is $-CH_3$ and $X^2$ is F.

In one or more embodiments, R is $-(CH_2)_r-$, or r is 1 or 5.

In one or more embodiments, R is $-(CH_2)-$.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, the antibody-drug conjugate has a structure shown as formula 1-12, I-13, I-14, I-15, I-16, I-17, I-18, I-19, I-20, I-21, I-22, I-23, I-24, or I-25, or a stereoisomer, or a pharmaceutically acceptable salt or solvate thereof:

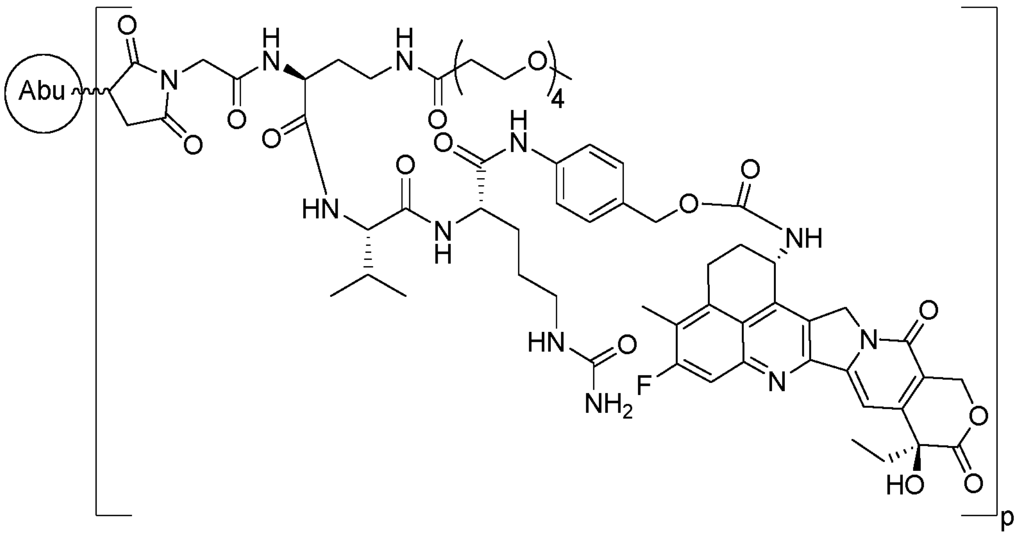

formula I-12

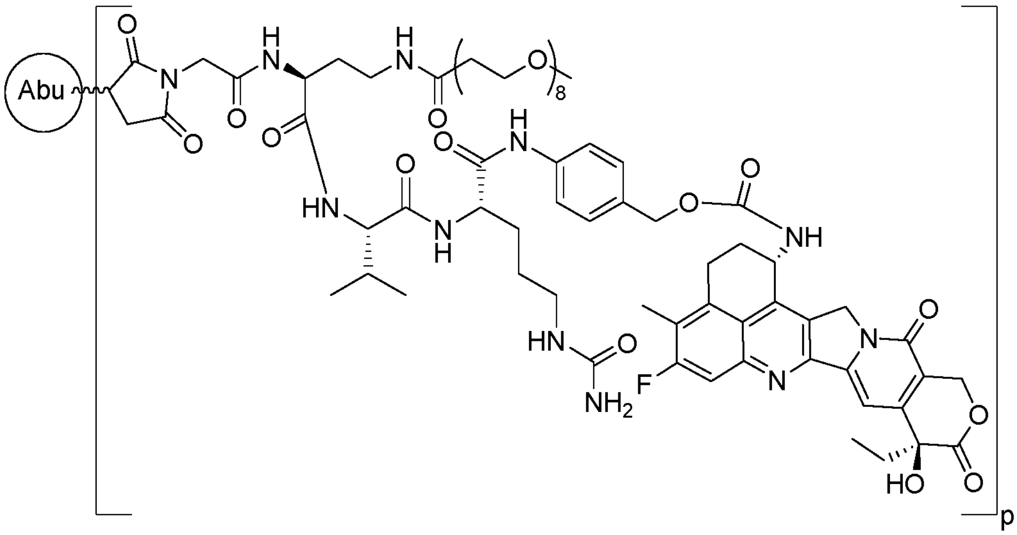

formula I-13

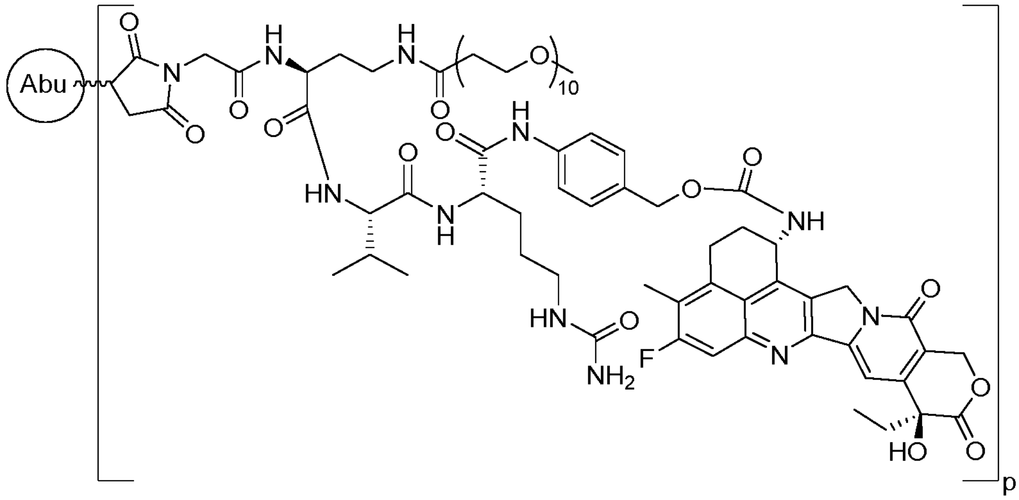

formula I-14

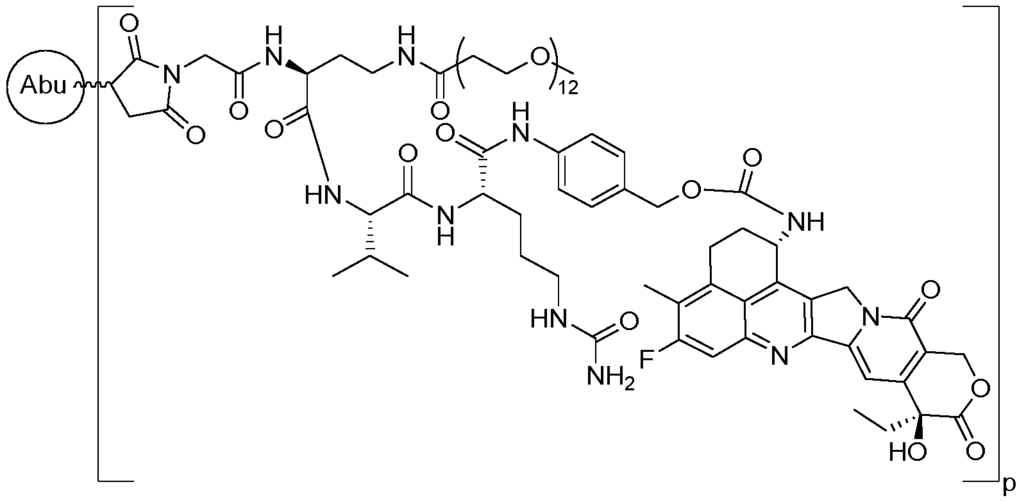

formula I-15

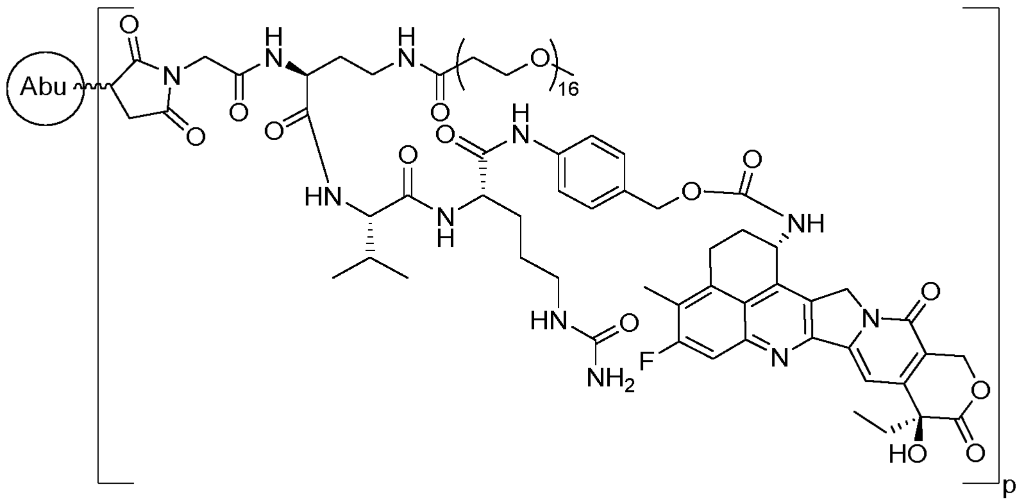

formula I-16

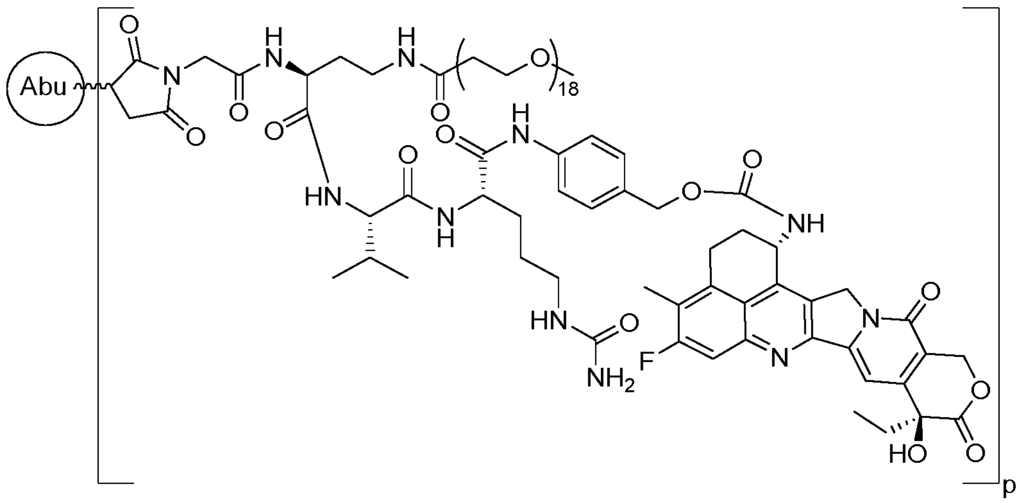

formula I-17

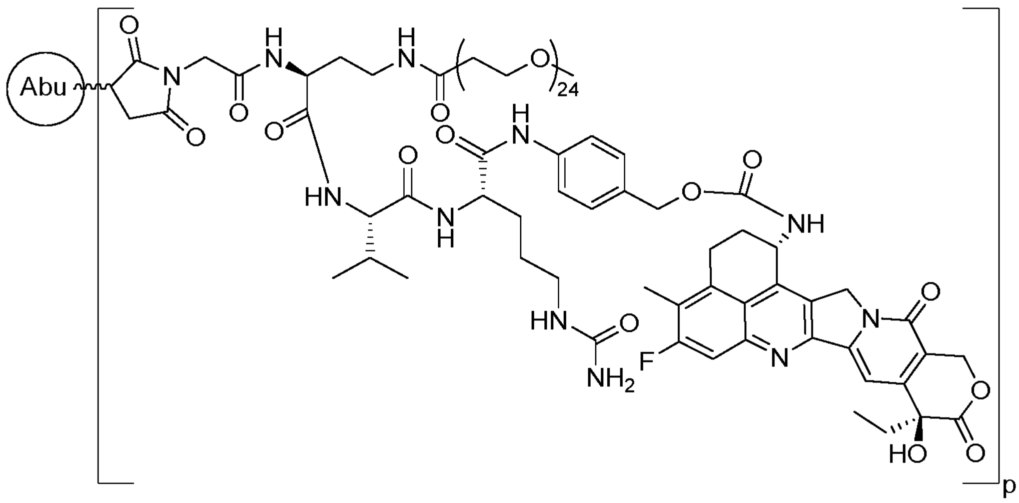

formula I-18

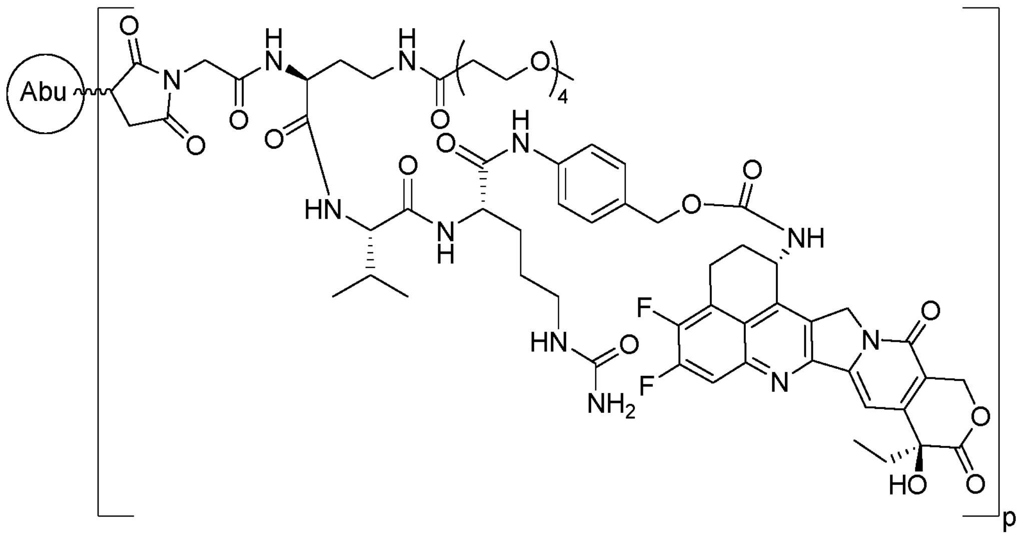

formula I-19

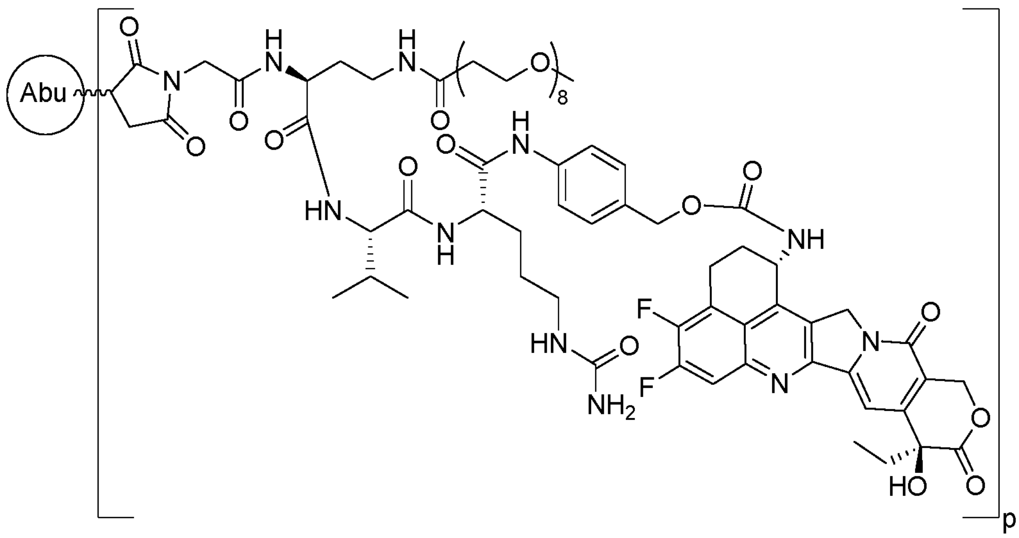

formula I-20

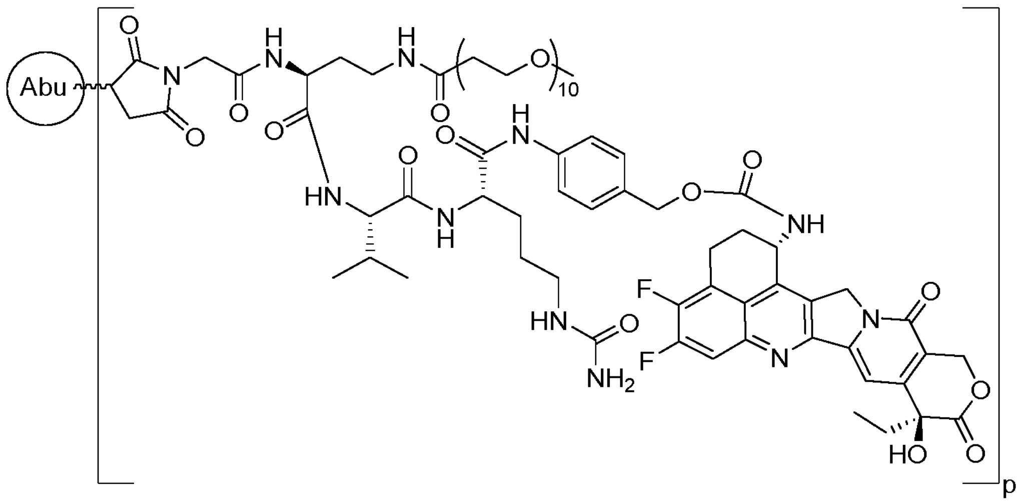

formula I-21

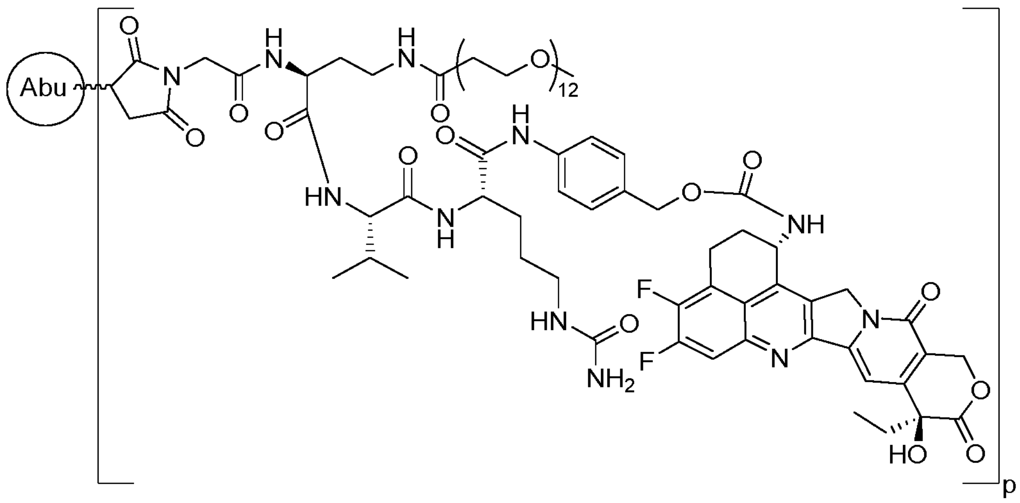

formula I-22

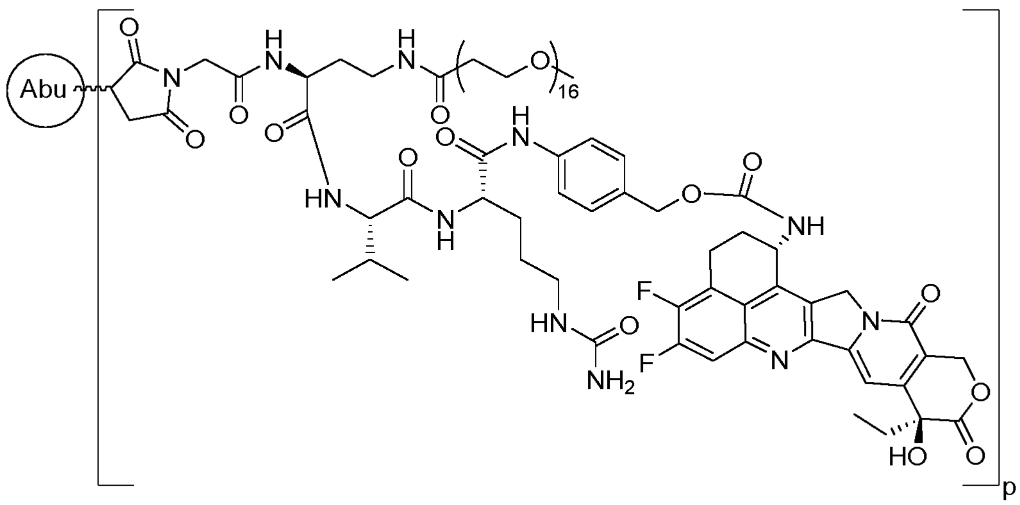

formula I-23

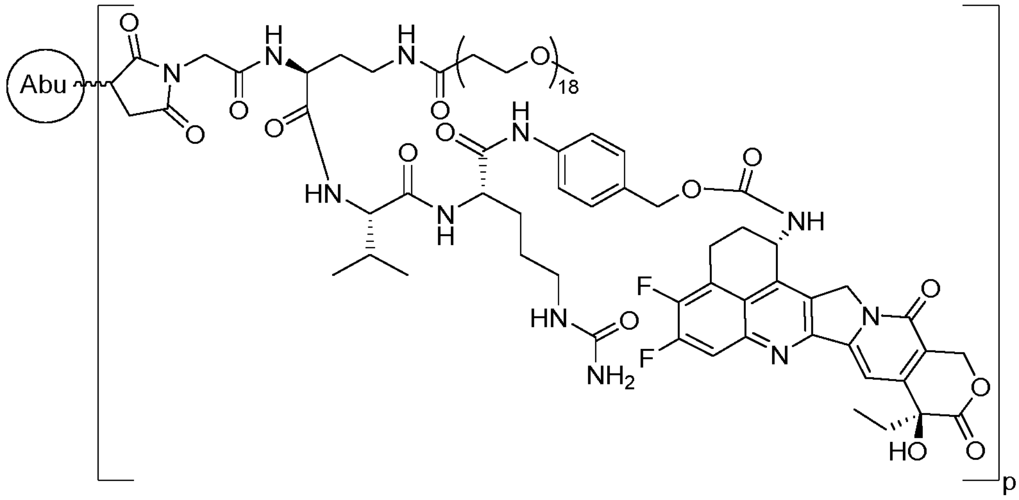

formula I-24

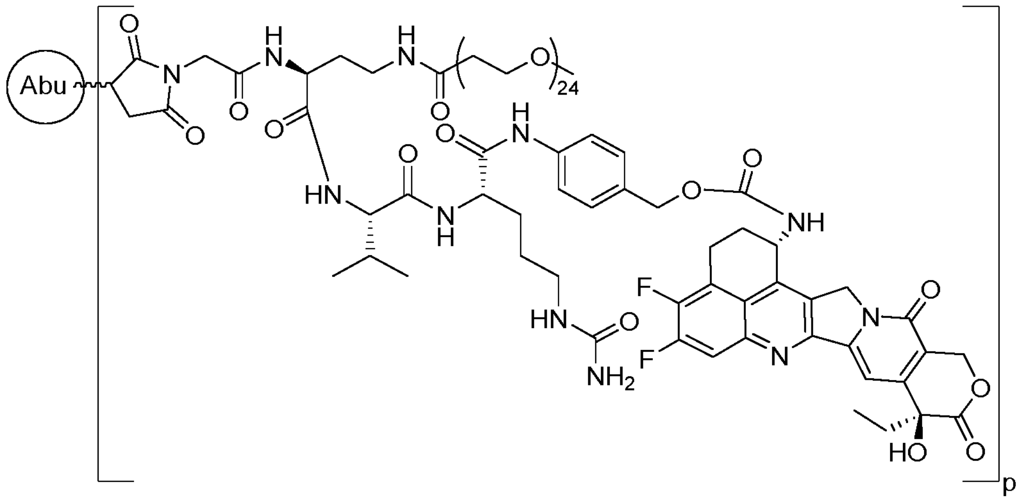

formula I-25

wherein

Abu is an antibody or an antigen-binding fragment that binds to HER2;

p is 1-10, e.g., 1, 2, 3, 4, 5, 6, 7, 8, 9 or 10.

In one or more embodiments, p is 2-8.
In one or more embodiments, p is 4-8.
In one or more embodiments, p is 6-8.
In one or more embodiments, p is 7-8.
In one or more embodiments, Abu is trastuzumab (e.g. Herceptin®, Herceptin Hylecta or biosimilar thereof, e.g. Zercepac®, Herzuma®, Kanjinti®, Ogivri®, Ontruzant®, Trazimera®).
The antibody-drug conjugate described herein is disclosed in WO2022/253284.
In one or more embodiments, the effective amount refers to the amount of an active compound or agent that results in a biological or drug response of tissues, systems, animals, individuals, and humans which is being sought by researchers, vets, doctors, or other clinical doctors, including the treatment of a disease, such as a HER2-positive solid tumor.
Examples of the HER2-positive solid tumor include, but are not limited to, ovarian cancer, uterine cancer, endometrial cancer, fallopian tube cancer, breast cancer, cervical cancer, gastric cancer, colorectal cancer, squamous cell

cancer (e.g., epithelial squamous cell cancer), lung cancer (including small cell lung cancer, non-small cell lung cancer, lung adenocarcinoma, and lung squamous cell carcinoma), peritoneal cancer, hepatocellular carcinoma, gastric cancer (including gastrointestinal cancer), pancreatic cancer, glioblastoma, cervical cancer, liver cancer, bladder cancer, urinary tract cancer, hepatoma, breast cancer, colon cancer, rectal cancer, salivary gland cancer, renal cancer, prostate cancer, vulval cancer, thyroid cancer, anal cancer, and penile cancer.

In one or more embodiments, the antibody-drug conjugate is administered at a dose of about 1-30 mg/kg each time. In one or more embodiments, the antibody-drug conjugate is administered at a dose of about 1-10 mg/kg each time. In one or more embodiments, the antibody-drug conjugate is administered at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, about 10 mg/kg, or a range between any two of these values (inclusive) or any value therein each time.

In one or more embodiments, the antibody-drug conjugate is administered at a dose of about 50-3000 mg each time. In one or more embodiments, the antibody-drug conjugate is administered at a dose of about 100-2000 mg, 200-1000 mg, 300-800 mg, or 400-700 mg each time. In one or more embodiments, the antibody-drug conjugate is administered at a dose of about 50 mg, about 100 mg, about 200 mg, about 250 mg, about 300 mg, about 400 mg, about 500 mg, about 600 mg, or about 700 mg, or a range between any two of these values (inclusive) or any value therein each time.

In one or more embodiments, one treatment cycle is at least 1 week, at least 2 weeks, at least 3 weeks, at least 4 weeks, at least 5 weeks, at least 6 weeks, or at least 7 weeks. In one or more embodiments, one treatment cycle is 1 week, 2 weeks, 3 weeks, 4 weeks, 5 weeks, 6 weeks, 7 weeks, or a range between any two of these values (inclusive) or any value therein. In one or more embodiments, the antibody-drug conjugate is administered about once a week, or about once every 2 weeks, every 3 weeks, every 4 weeks, every 5 weeks, every 6 weeks, or every 7 weeks.

In one or more embodiments, the antibody-drug conjugate is administered about once a week at a dose of about 1-30 mg/kg each time. In one or more embodiments, the antibody-drug conjugate is administered about once a week at a dose of about 1-10 mg/kg each time. In one or more embodiments, the antibody-drug conjugate is administered about once a week at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once a week at a dose of about 50-3000 mg each time. In one or more embodiments, the antibody-drug conjugate is administered about once a week at a dose of about 100-2000 mg, 200-1000 mg, 300-800 mg, or 400-700 mg each time. In one or more embodiments, the antibody-drug conjugate is administered about once a week at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 2 weeks at a dose of about 1-30 mg/kg each time. In one or more embodiments, the antibody-drug conjugate is administered about once every 2 weeks at a dose of about 1-10 mg/kg each time. In one or more embodiments, the antibody-drug conjugate is administered about once every 2 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 2 weeks at a dose of about 50-3000 mg each time. In one or more embodiments, the antibody-drug conjugate is administered about once every 2 weeks at a dose of about 100-2000 mg, 200-1000 mg, 300-800 mg, or 400-700 mg each time. In one or more embodiments, the antibody-drug conjugate is administered about once every 2 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 3 weeks at a dose of about 1-30 mg/kg each time. In one or more embodiments, the antibody-drug conjugate is administered about once every 3 weeks at a dose of about 1-10 mg/kg each time. In one or more embodiments, the antibody-drug conjugate is administered about once every 3 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 3 weeks at a dose of about 50-3000 mg each time. In one or more embodiments, the antibody-drug conjugate is administered about once every 3 weeks at a dose of about 100-2000 mg, 200-1000 mg, 300-800 mg, or 400-700 mg each time. In one or more embodiments, the antibody-drug conjugate is administered about once every 3 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 4 weeks at a dose of about 1-30 mg/kg each time. In one or more embodiments, the antibody-drug conjugate is administered about once every 4 weeks at a dose of about 1-10 mg/kg each time. In one or more embodiments, the antibody-drug conjugate is

administered about once every 4 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 4 weeks at a dose of about 50-3000 mg each time. In one or more embodiments, the antibody-drug conjugate is administered about once every 4 weeks at a dose of about 100-2000 mg, 200-1000 mg, 300-800 mg, or 400-700 mg each time. In one or more embodiments, the antibody-drug conjugate is administered about once every 4 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 5 weeks at a dose of about 1-30 mg/kg each time. In one or more embodiments, the antibody-drug conjugate is administered about once every 5 weeks at a dose of about 1-10 mg/kg each time. In one or more embodiments, the antibody-drug conjugate is administered about once every 5 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 5 weeks at a dose of about 50-3000 mg each time. In one or more embodiments, the antibody-drug conjugate is administered about once every 5 weeks at a dose of about 100-2000 mg, 200-1000 mg, 300-800 mg, or 400-700 mg each time. In one or more embodiments, the antibody-drug conjugate is administered about once every 5 weeks at a dose of about 50 mg, about 100 mg, about 200 mg, about 250 mg, about 300 mg, about 400 mg, about 500 mg, about 600 mg, or about 700 mg, or a range between any two of these values (inclusive) or any value therein each time.

In one or more embodiments, the antibody-drug conjugate is administered about once every 6 weeks at a dose of about 1-30 mg/kg each time. In one or more embodiments, the antibody-drug conjugate is administered about once every 6 weeks at a dose of about 1-10 mg/kg each time. In one or more embodiments, the antibody-drug conjugate is administered about once every 6 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 6 weeks at a dose of about 50-3000 mg each time. In one or more embodiments, the antibody-drug conjugate is administered about once every 6 weeks at a dose of about 100-2000 mg, 200-1000 mg, 300-800 mg, or 400-700 mg each time. In one or more embodiments, the antibody-drug conjugate is administered about once every 6 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the patient receives treatment for one treatment cycle. In one or more embodiments, the patient receives treatment for multiple (e.g., 2, 3, 4, 5, 6, 7, 8, 9, 10, 11, 12, 15, or 17, or a range between any two of these values (inclusive) or any value therein) treatment cycles. In one or more embodiments, the patient receives treatment until the condition is alleviated and no treatment is required.

In one or more embodiments, in the method for treating a HER2-positive solid tumor provided herein, the antibody-drug conjugate may be administered by any convenient route, for example, by infusion or bolus injection, or by absorption through epithelial or skin mucosa (e.g., oral mucosa, rectal and intestinal mucosa, etc.). Accordingly, the pharmaceutical composition may be administered intravenously, subcutaneously, orally, rectally, parenterally, intracerebrally, intravaginally, intraperitoneally, topically (e.g., by powder, ointment, drop, or transdermal patch), or buccally, or by oral or nasal spray. In one or more embodiments, the administration route is intravenous infusion or subcutaneous injection.

In one or more embodiments, the antibody-drug conjugate may be formulated into a pharmaceutical composition comprising the antibody-drug conjugate at the single dose as described above and administered to the patient in a form suitable for the selected administration route, e.g., parenteral, intravenous (i.v.), intramuscular, local, or subcutaneous (s.c.) administration. In one or more embodiments, the administration route is intravenous infusion or subcutaneous injection.

In one or more embodiments, in the method or use for treating a HER2-positive solid tumor provided herein, the antibody-drug conjugate is used in combination with one or more additional therapies. Suitable additional therapies include existing drugs (second drugs) and/or surgical therapies for specific applications (such as cancer). For example, the antibody-drug conjugate is used in combination with one or more additional chemotherapeutic or anti-tumor agents. Alternatively, the additional chemotherapeutic agent is radiotherapy. In one or more embodiments, the chemotherapeutic agent is an apoptosis inducer.

In one or more embodiments, the antibody-drug conjugate and the additional agent are prepared as a single therapeutic composition, and the antibody-drug conjugate and the additional agent are administered simultaneously. Alternatively, the antibody-drug conjugate and the additional agent are separated from each other, for example, are each

prepared as a separate therapeutic composition, and the antibody-drug conjugate and the additional agent are administered simultaneously, or the antibody-drug conjugate and the additional agent are administered at different time points during a treatment regimen. For example, the antibody-drug conjugate is administered prior to the administration of the additional agent, the antibody-drug conjugate is administered subsequent to the administration of the additional agent, or the antibody-drug conjugate and the additional agent are administered in an alternating manner. Herein, the antibody-drug conjugate and the additional agent are administered at a single dose or at multiple doses.

In one or more embodiments, in the method for treating a HER2-positive solid tumor provided herein, the antibody-drug conjugate and the additional agent may be administered by any convenient route, for example, by infusion or bolus injection, or by absorption through epithelial or skin mucosa (e.g., oral mucosa, rectal and intestinal mucosa, etc.), and may be administered in combination with other bioactive agents. Accordingly, the pharmaceutical composition may be administered intravenously, subcutaneously, orally, rectally, parenterally, intracerebrally, intravaginally, intraperitoneally, topically (e.g., by powder, ointment, drop, or transdermal patch), or buccally, or by oral or nasal spray. In one or more embodiments, the administration route is intravenous infusion or subcutaneous injection.

In one or more embodiments, the antibody-drug conjugate and/or other agents in combination with the antibody-drug conjugate may be formulated into a pharmaceutical composition and administered to the patient in a form suitable for the selected administration route, e.g., parenteral, intravenous (i.v.), intramuscular, local, or subcutaneous (s.c.) administration. In one or more embodiments, the administration route is intravenous infusion or subcutaneous injection.

In one or more embodiments, the antibody-drug conjugate is administered at a dose of about 1-30 mg/kg each time. In one or more embodiments, the antibody-drug conjugate is administered at a dose of about 1-10 mg/kg each time. In one or more embodiments, the antibody-drug conjugate is administered at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, about 10 mg/kg, or a range between any two of these values (inclusive) or any value therein each time.

In one or more embodiments, the antibody-drug conjugate is administered at a dose of about 50-3000 mg each time. In one or more embodiments, the antibody-drug conjugate is administered at a dose of about 100-2000 mg, 200-1000 mg, 300-800 mg, or 400-700 mg each time. In one or more embodiments, the antibody-drug conjugate is administered at a dose of about 50 mg, about 100 mg, about 200 mg, about 250 mg, about 300 mg, about 400 mg, about 500 mg, about 600 mg, or about 700 mg, or a range between any two of these values (inclusive) or any value therein each time.

In one or more embodiments, one treatment cycle is at least 1 week, at least 2 weeks, at least 3 weeks, at least 4 weeks, at least 5 weeks, at least 6 weeks, or at least 7 weeks. In one or more embodiments, one treatment cycle is 1 week, 2 weeks, 3 weeks, 4 weeks, 5 weeks, 6 weeks, 7 weeks, or a range between any two of these values (inclusive) or any value therein. In one or more embodiments, the antibody-drug conjugate is administered about once a week, or about once every 2 weeks, every 3 weeks, every 4 weeks, every 5 weeks, every 6 weeks, or every 7 weeks.

In one or more embodiments, the antibody-drug conjugate is administered about once a week at a dose of about 1-30 mg/kg each time. In one or more embodiments, the antibody-drug conjugate is administered about once a week at a dose of about 1-10 mg/kg each time. In one or more embodiments, the antibody-drug conjugate is administered about once a week at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once a week at a dose of about 50-3000 mg each time. In one or more embodiments, the antibody-drug conjugate is administered about once a week at a dose of about 100-2000 mg, 200-1000 mg, 300-800 mg, or 400-700 mg each time. In one or more embodiments, the antibody-drug conjugate is administered about once a week at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 2 weeks at a dose of about 1-30 mg/kg each time. In one or more embodiments, the antibody-drug conjugate is administered about once every 2 weeks at a dose of about 1-10 mg/kg each time. In one or more embodiments, the antibody-drug conjugate is administered about once every 2 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 2 weeks at a dose of about 50-3000 mg each time. In one or more embodiments, the antibody-drug conjugate is administered about once every 2 weeks at a dose of about 100-2000 mg, 200-1000 mg, 300-800 mg, or 400-700 mg each time. In one or more embodiments, the antibody-drug conjugate is administered about once every 2 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 3 weeks at a dose of about 1-30 mg/kg each time. In one or more embodiments, the antibody-drug conjugate is administered about once every 3 weeks at a dose of about 1-10 mg/kg each time. In one or more embodiments, the antibody-drug conjugate is

administered about once every 3 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 3 weeks at a dose of about 50-3000 mg each time. In one or more embodiments, the antibody-drug conjugate is administered about once every 3 weeks at a dose of about 100-2000 mg, 200-1000 mg, 300-800 mg, or 400-700 mg each time. In one or more embodiments, the antibody-drug conjugate is administered about once every 3 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 4 weeks at a dose of about 1-30 mg/kg each time. In one or more embodiments, the antibody-drug conjugate is administered about once every 4 weeks at a dose of about 1-10 mg/kg each time. In one or more embodiments, the antibody-drug conjugate is administered about once every 4 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 4 weeks at a dose of about 50-3000 mg each time. In one or more embodiments, the antibody-drug conjugate is administered about once every 4 weeks at a dose of about 100-2000 mg, 200-1000 mg, 300-800 mg, or 400-700 mg each time. In one or more embodiments, the antibody-drug conjugate is administered about once every 4 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the antibody-drug conjugate is administered about once every 5 weeks at a dose of about 1-30 mg/kg each time. In one or more embodiments, the antibody-drug conjugate is administered about once every 5 weeks at a dose of about 1-10 mg/kg each time. In one or more embodiments, the antibody-drug conjugate is administered about once every 5 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 5 weeks at a dose of about 50-3000 mg each time. In one or more embodiments, the antibody-drug conjugate is administered about once every 5 weeks at a dose of about 100-2000 mg, 200-1000 mg, 300-800 mg, or 400-700 mg each time. In one or more embodiments, the antibody-drug conjugate is administered about once every 5 weeks at a dose of about 50 mg, about 100 mg, about 200 mg, about 250 mg, about 300 mg, about 400 mg, about 500 mg, about 600 mg, or about 700 mg, or a range between any two of these values (inclusive) or any value therein each time.

In one or more embodiments, the antibody-drug conjugate is administered about once every 6 weeks at a dose of about 1-30 mg/kg each time. In one or more embodiments, the antibody-drug conjugate is administered about once every 6 weeks at a dose of about 1-10 mg/kg each time. In one or more embodiments, the antibody-drug conjugate is administered about once every 6 weeks at a dose of about 1 mg/kg, about 1.2 mg/kg, about 2.4 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein each time. In one or more embodiments, the antibody-drug conjugate is administered about once every 6 weeks at a dose of about 50-3000 mg each time. In one or more embodiments, the antibody-drug conjugate is administered about once every 6 weeks at a dose of about 100-2000 mg, 200-1000 mg, 300-800 mg, or 400-700 mg each time. In one or more embodiments, the antibody-drug conjugate is administered about once every 6 weeks at a dose of about 50 mg, about 100 mg, about 200 mg, about 250 mg, about 300 mg, about 400 mg, about 500 mg, about 600 mg, about 700 mg, or a range between any two of these values (inclusive) or any value therein each time.

In one or more embodiments, the patient receives treatment for one treatment cycle. In one or more embodiments, the patient receives treatment for multiple (e.g., 2, 3, 4, 5, 6, 7, 8, 9, 10, 11, 12, 15, or 17, or a range between any two of these values (inclusive) or any value therein) treatment cycles. In one or more embodiments, the patient receives treatment until the condition is alleviated and no treatment is required.

In one or more embodiments, the antibody-drug conjugate, the pharmaceutical composition comprising the antibody-drug conjugate, or the second drug is administered by injection. In one or more embodiments, the antibody-drug conjugate, the pharmaceutical composition comprising the antibody-drug conjugate, or the second drug is administered by subcutaneous (s.c.) injection, intraperitoneal (i.p.) injection, parenteral injection, intra-arterial injection, intravenous (i.v.) injection, or the like. In one or more embodiments, the antibody-drug conjugate, the pharmaceutical composition comprising the antibody-drug conjugate, or the second drug is administered by infusion. In one or more embodiments, the antibody-drug conjugate, the pharmaceutical composition comprising the antibody-drug conjugate, or the second drug is administered by bolus injection. In one or more embodiments, the antibody-drug conjugate, the pharmaceutical composition comprising the antibody-drug conjugate, or the second drug is administered by intravenous injection. In

one or more embodiments, the antibody-drug conjugate, the pharmaceutical composition comprising the antibody-drug conjugate, or the second drug is administered by intravenous infusion. In one or more embodiments, the antibody-drug conjugate, the pharmaceutical composition comprising the antibody-drug conjugate, or the second drug is administered by subcutaneous injection. The dose of the antibody-drug conjugate will depend on the properties of the drug, the extent to which the internalization, transport, and release of the drug are triggered at the cell surface, and the disease being treated and the conditions of the patient (e.g., age, gender, body weight, etc.).

In one or more embodiments, the antibody-drug conjugate, the pharmaceutical composition comprising the antibody-drug conjugate, or the second drug is administered by intravenous (i.v.) infusion (i.e., intravenous infusion). In one or more embodiments, the duration of the intravenous infusion of the antibody-drug conjugate or the pharmaceutical composition comprising the antibody-drug conjugate is about 30 minutes, about 35 minutes, about 40 minutes, about 45 minutes, about 50 minutes, about 55 minutes, about 60 minutes, about 65 minutes, about 70 minutes, about 75 minutes, about 80 minutes, about 85 minutes, about 90 minutes, about 95 minutes, about 100 minutes, about 105 minutes, about 110 minutes, about 105 minutes, about 120 minutes, or a range between any two of these values (inclusive), or any value therein. In one or more embodiments, the duration of the first intravenous infusion of the antibody-drug conjugate or the pharmaceutical composition comprising the antibody-drug conjugate is 90 (± 5) minutes.

In one or more embodiments, the second drug is an antibody. In one or more embodiments, the second drug is a HER2-targeted antibody. In one or more embodiments, the HER2-targeted antibody is an antibody that binds to the extracellular domain-II region of a HER-2 receptor, and blocks the heterodimerization of HER-2 with other EGFR family receptors. In one or more embodiments, a heavy chain variable region of the HER2-targeted antibody comprises an HCDR1 set forth in SEQ ID NO: 1, an HCDR2 set forth in SEQ ID NO: 2, and an HCDR3 set forth in SEQ ID NO: 3; a light chain variable region of the HER2-targeted antibody comprises an LCDR1 set forth in SEQ ID NO: 4, an LCDR2 set forth in SEQ ID NO: 5, and an LCDR3 set forth in SEQ ID NO: 6.

In one or more embodiments, a heavy chain variable region of the HER2-targeted antibody comprises a sequence set forth in SEQ ID NO: 7, a sequence having at least 80% identity to the sequence set forth in SEQ ID NO: 7, or a sequence having one or more conservative amino acid substitutions as compared to the sequence set forth in SEQ ID NO: 7; and/or a light chain variable region of the antibody comprises a sequence set forth in SEQ ID NO: 8, a sequence having at least 80% identity to the sequence set forth in SEQ ID NO: 8, or a sequence having one or more conservative amino acid substitutions as compared to the sequence set forth in SEQ ID NO: 8.

In one or more embodiments, a heavy chain of the HER2-targeted antibody comprises a sequence set forth in SEQ ID NO: 9, a sequence having at least 80% identity to the sequence set forth in SEQ ID NO: 9, or a sequence having one or more conservative amino acid substitutions as compared to the sequence set forth in SEQ ID NO: 9; and/or a light chain of the antibody comprises a sequence set forth in SEQ ID NO: 10, a sequence having at least 80% identity to the sequence set forth in SEQ ID NO: 10, or a sequence having one or more conservative amino acid substitutions as compared to the sequence set forth in SEQ ID NO: 10.

In one or more embodiments, a heavy chain of the HER2-targeted antibody comprises a sequence set forth in SEQ ID NO: 9, and a light chain of the antibody comprises a sequence set forth in SEQ ID NO: 10.

In one or more embodiments, the HER2-targeted antibody has a fucosylation level of 0%-5%, and the antibody comprises no less than 60% of G0.

In one or more embodiments, the HER2-targeted antibody has a content of G0-GN glycoform of 3%-7%, a content of G1 glycoform of 6%-12%, a content of G1' glycoform of 6%-12%, and a content of G2 glycoform of not more than 3%. In one or more embodiments, the second drug is BAT0303F. In one or more embodiments, the second drug is pertuzumab (e.g., Perjeta® or a biosimilar thereof, or a corresponding monoclonal antibody with an enhanced ADCC effect thereof, or a partially or totally defucosylated monoclonal antibody).

In one or more embodiments, the HER2-targeted antibody is an antibody with enhanced ADCC (antibody-dependent cell-mediated cytotoxic effect).

In one or more embodiments, the antibody-drug conjugate and the second drug are administered to the patient sequentially in any order, or simultaneously.

In one or more embodiments, the second drug is administered at a dose of about 1-15 mg/kg each time. In one or more embodiments, the second drug is administered at a dose of about 3-15 mg/kg each time. In one or more embodiments, the second drug is administered at a dose of about 1 mg/kg, about 3 mg/kg, about 6 mg/kg, about 10 mg/kg, or about 15 mg/kg, or a range between any two of these values (inclusive) or any value therein each time. In one or more embodiments, the second drug is administered at a dose of about 50 mg, about 100 mg, about 200 mg, about 300 mg, about 400 mg, about 500 mg, about 600 mg, about 700 mg, about 800 mg, about 900 mg, or about 1000 mg, or a range between any two of these values (inclusive) or any value therein each time. In one or more embodiments, the second drug is administered about once a week. In one or more embodiments, the second drug is administered about once every 2 weeks. In one or more embodiments, the second drug is administered about once every 3 weeks. In one or more embodiments, the second drug is administered about once every 4 weeks. In one or more embodiments, the second drug is administered about once every 5 weeks. In one or more embodiments, the second drug is administered about once

every 6 weeks. In one or more embodiments, the second drug is administered at a dose of about 50-1000 mg each time. In one or more embodiments, the second drug is administered about once every 3 weeks at a first dose of about 840 mg followed by a dose of about 420 mg each time.

In one or more embodiments, the duration of the intravenous infusion of the second drug is about 30 minutes, about 35 minutes, about 40 minutes, about 45 minutes, about 50 minutes, about 55 minutes, about 60 minutes, or a range between any two of these values (inclusive) or any value therein. In one or more embodiments, the duration of the first intravenous infusion of the second drug is 60 minutes.

## BRIEF DESCRIPTION OF THE DRAWINGS

FIG. 1 shows the bystander effect of ADC1 to ADC3;
FIG. 2 shows the effect of ADC1 to ADC3 in inhibiting tumor growth.

## DETAILED DESCRIPTION

"About" refers to a general error range for corresponding values as readily understood by those skilled in the relevant art. In some embodiments, "about" mentioned herein refers to the values described and ranges thereof of ± 10%, ± 5%, or ± 1%.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of the extent of disease, stabilized (i.e., not worsening) state of disease, delay or slowing of progressive disease, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from the administration of the antibody or the pharmaceutical composition disclosed herein for detection, diagnostic procedures, and/or treatment. The term "HER2-positive solid tumor" encompasses cancer cells having a higher-than-normal level of HER2. Examples of the HER2- positive solid tumor include, but are not limited to, HER2-positive breast cancer and HER2-positive gastric cancer. Optionally, the HER2-positive solid tumor has an immunohistochemistry (IHC) score of 2+ or 3+ and/or an *in situ* hybridization (ISH) amplification ratio of ≥ 2.0.

As used herein, the term "effective amount" or "therapeutically effective amount" refers to the amount of a drug, e.g., an antibody or ADC, that is sufficient to reduce or ameliorate the severity and/or duration of a condition (e.g., cancer) or one or more symptoms thereof, prevent the progression of a condition, cause regression of a condition, prevent the recurrence, development, onset or progression of one or more symptoms associated with a condition, detect a condition, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with cancer. For example, the effective amount may improve progression-free survival (PFS), improve overall survival (OS), or decrease the likelihood of recurrence.

The terms "patient" and "subject" are used interchangeably and refer to any mammal in need of diagnosis, prognosis, or treatment, including, but not limited to, humans, dogs, cats, guinea pigs, rabbits, rats, mice, horses, cattle, etc., particularly animals with one or more conditions of a HER2-positive solid tumor. In one or more embodiments, the patient is a human.

"Patient population" refers to a group of cancer patients. Such populations may be used to demonstrate the statistically significant efficacy and/or safety of drugs, such as the antibody-drug conjugate of the present invention and the combination thereof with a second drug.

"Survival" means that the patients remain alive, and includes disease-free survival (DFS), progression-free survival (PFS), and overall survival (OS). Survival can be evaluated by the Kaplan-Meier method, and any difference in the survival can be calculated using a hierarchical log-rank test.

"Disease-free survival" refers to the time a patient survives from the initiation of therapy or the initial diagnosis without recurrence of the cancer. Events used in DFS analysis may include local, regional and distant cancer recurrence, secondary cancer occurrence, and death from any cause in patients without prior events.

"Extended survival" means increased PFS, DFS, and/or OS in a treated patient relative to an untreated patient or relative to a control treatment regimen.

"Chemotherapy" is the use of chemotherapeutic agents that can be used to treat cancer.

"$IC_{50}$" represents 50% inhibitory concentration, i.e., a concentration of drug or inhibitor required to inhibit half of a given biological process.

The term "administration" as used herein refers to administering a substance for therapeutic purposes (e.g., treating a tumor).

The term "drug for treating a tumor" as used herein refers to an agent having the functional property of inhibiting in a human the development or progression of a tumor, particularly a malignant (cancerous) lesion, such as cancer, sarcoma, lymphoma, or leukemia. Inhibition of metastasis is a property of anti-tumor drugs in many cases.

MTD definition: MTD (maximum tolerated dose) is defined as the highest dose level at which ≤ 1/6 subjects in a dose group were observed to experience DLT during the DLT evaluation period.

PK (pharmacokinetic) parameters used herein are defined as follows:

PK parameters of single administration: Cmax, Tmax, T1/2, CL, Vd, Ke, MRT, AUC(0-τ), and AUC (0-∞):

| | |
|---|---|
| Cmax | Peak concentration. Directly obtained from the measured plasma concentration-time data. |
| Tmax | Time to peak. Directly obtained from the measured plasma concentration-time data. |
| t1/2 | Terminal elimination half-life. t1/2=ln2/λz |
| CL | Apparent clearance. CL= Dpo / AUC0-∞ |
| Vd | Apparent volume of distribution. Vd=Dpo/ AUC0-∞/λz |
| Ke (λz) | Elimination rate constant, a slope of the terminal segment of a semi-logarithmic log of drug concentration-time curve calculated by using the linear regression method. |
| MRT0-t | Mean residence time, calculated by the formula: AUMC0-t/AUC0-t, where the calculation formula for AUMC0-t is: AUMC(i, i+1) = $(t_{i+1} - t_j)(t_{i+1} * C_{i+1} - t_i * C_i)/2$, and AUMC is the sum of all AUMC(i, i+1) |
| MRT0-∞ | Calculation formula: AUMC0-∞/AUC0-∞, where AUMC0-∞= AUMC0-t+ Tlast *\|Clast \|/λz+\|Clast \|/(λz*λz) |
| AUC0-t | Area under the curve from zero to the time at which the lowest plasma concentration can be detected. Calculated by the linear trapezoidal rule: AUC(i, i+1) = (Ti+1 - Ti)(Ci + Ci+1)/2, where AUC0-t is the sum of all AUC(i, i+1). |
| AUC0-∞ | Area under the curve of time extrapolated from zero to infinity. AUC0-∞ = AUC0-t + Ct/λz (Ct is the last measurable plasma concentration). |
| AUC_%Extrap | The proportion of AUC theoretically extrapolated from the last point to infinity relative to AUC0-∞ |

PK parameters of multiple doses: Cmax,ss, Cavg,ss, Cmin,ss, AUC(0-τ)ss, AUC(0-∞)ss, Tmax,ss, T1/2,ss, CL, Vss, Ke, MRT, accumulation index (Rac), and fluctuation index DF:

| | |
|---|---|
| Cmax, ss | Peak concentration at steady state. Directly obtained from the measured plasma concentration-time data at steady state within dosing intervals. |
| Cmin, ss | Minimum concentration at steady state. Directly obtained from the measured plasma concentration-time data at steady state within dosing intervals. |
| Cavg,ss | Mean plasma concentration at steady state within dosing intervals. Cav = AUC0-τ/τ, where τ is the dosing interval |
| AUC(0-t)ss | Area under the curve at steady state from zero to the time at which the lowest plasma concentration can be detected. Calculated by the linear trapezoidal rule: AUC(i, i+1),ss |
| | = (Ti+1 - Ti)(Ci + Ci+1)/2, AUC0-t, where ss is the sum of all AUC(i, i+1),ss. |
| AUC(0-∞)ss | Area under the curve of time at steady state extrapolated from zero to infinity.<br>AUC0-∞,ss = AUC0-t,ss + Ct/λz (Ct is the last measurable plasma concentration at steady state). |
| Tmax,ss | Time to peak. Directly obtained from the measured plasma concentration-time data. |
| t1/2,ss | Terminal elimination half-life. t1/2,ss = ln2/λz |

(continued)

| | |
|---|---|
| CLss | Apparent clearance at steady state. CLss= Dpo /AUCss。 |
| Vd | Volume of distribution at steady state, Vd =Dpo/ AUC0-∞,ss/λz |
| Ke (λz) | Elimination rate constant, a slope of the terminal segment of a semi-logarithmic log of drug concentration-time curve calculated by using the linear regression method. |
| MRTO-∞, ss | Calculation formula: AUMC0-∞,ss/AUC0-∞,ss, where AUMC0-∞, ss = AUMC0-t,ss + Tlast × \|Clast\|/λz + \|Clast\|/(λz × λz) |
| Rac | Accumulation ratio. $Rac=\frac{1}{1-e^{-Ke\tau}}$ |
| DF | The degree of fluctuation. DF=( Cmax,ss- Cmin,ss)/ Cavg,ss |
| AUC_%Extrap | The proportion of AUC theoretically extrapolated from the last point to infinity relative to AUC0-∞ |

ORR (objective response rate): the proportion of patients whose tumors have shrunk by a certain amount and have been maintained for a certain period of time, including cases of CR (complete response) and PR (partial response).

Duration of response (DOR): DOR is defined as the time from the first evaluation of objective response to the first evaluation of PD (progressive disease) or any-cause death before PD, reflecting the duration of ORR.

Disease control rate (DCR): the proportion of patients whose tumors have shrunk or stabilized and have been maintained for a certain period of time, including cases of CR, PR, and SD (stable disease).

Progression-free survival (PFS): the time from the first dose to the occurrence of objective progression or all-cause death of the tumor (whichever occurs first).

Overall survival (OS): the time from the date of the first dose to the occurrence of death from any cause.

The term "antibody-drug conjugate" and "ADC", are used interchangeably and refer to a binding protein (e.g., an antibody or an antigen-binding unit thereof) that is linked to one or more chemical drugs, which may optionally be a therapeutic agent or a cytotoxic agent. In a preferred embodiment, the ADC comprises an antibody, a drug (e.g., a cytotoxic drug), and a linker capable of attaching or conjugating the drug to the antibody. Non-limiting examples of drugs that can be included in the ADC include a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor), and a radiosensitizer.

The term "drug to antibody ratio" or "DAR" refers to the number of drugs (e.g., Exatecan) that are attached to one antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino, and aryl) is indicated by the prefix "Cx-Cy" or "Cx-y", wherein x is the minimum number of carbon atoms and y is the maximum number of carbon atoms. Thus, for example, "C1-C6 alkyl" refers to an alkyl containing 1 to 6 carbon atoms. If a substituent is described as "substituted with ... ", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. For illustration, monofluoroalkyl is an alkyl substituted with one fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with ... ", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, thiol, alkylthio, amino, C1-C6 aminoalkyl, C1-C6 aminoalkylamino, C1-C6 alkyl connected to a heterocyclic

ring, C1-C6 alkylamino connected to a heterocyclic ring, heterocyclyl, amino-substituted heterocyclyl, heterocyclylamino, carbamoyl, morpholin-1-yl, piperidin-1-yl, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$, or $-(CH_2CH_2O)_qC(O)NR^n(CH_2)_q-CH_3$; wherein each $R^n$ is independently H, C1-C6 alkyl, C3-C8 carbocyclyl, phenyl, or benzyl, and each q is independently 1, 2, 3, 4, 5, 6, 7, 8, 9, or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, n-propyl, isopropyl, n-butyl, isobutyl, sec-butyl, tert-butyl, n-pentyl, neopentyl, n-hexyl, etc. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphono, etc.

The term "alkenyl", by itself or as part of another substituent, refers to an unsaturated branched, linear, or cyclic alkyl having at least one carbon-carbon double bond derived by the removal of one hydrogen atom from a single carbon atom of a parent olefin. Typical alkenyl includes, but is not limited to, ethenyl; propenyl (e.g., prop-1-en-1-yl, prop-1-en-2-yl, prop-2-en-1-yl, prop-2-en-2-yl, or cycloprop-1-en-1-yl); cycloprop-2-en-1-yl; butenyl (e.g., but-1-en-1-yl, but-1-en-2-yl, 2-methyl-prop-1-en-1-yl, but-2-en-1-yl, but-2-en-2-yl, but-1,3-dien-1-yl, but-1,3-dien-2-yl, cyclobut-1-en-1-yl, cyclobut-1-en-3-yl, cyclobut-1,3-dien-1-yl, etc.), and the like.

The term "alkynyl", by itself or as part of another substituent, refers to an unsaturated branched, linear, or cyclic alkyl having at least one carbon-carbon triple bond derived by the removal of one hydrogen atom from a single carbon atom of a parent alkyne. Typical alkynyl includes, but is not limited to, ethynyl; propynyl (e.g., prop-1-yn-1-yl, prop-2-yn-1-yl, etc.); butynyl (e.g., but-1-yn-1-yl, but-1-yn-3-yl, but-3-yn-1-yl, etc.), and the like.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically indicated, the carbocyclyl may be optionally substituted with one or more substituents independently selected from: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated $\pi$-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphono, etc. Examples of unsubstituted aryl include, but are not limited to, phenyl, naphthyl, and anthracenyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4H)-ylidene, tetrahydrofuranyl, trioxacyclohexyl, trithianyl, triazinanyl, tetrahydropyranyl, thiomorpholinyl, thiomorpholinyl, 1-oxo-thiomorpholinyl, and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, n-propoxy, 1-methylethoxy (isopropoxy), n-butoxy, isobutoxy, sec-butoxy, and tert-butoxy. Alkoxy may be substituted or unsubstituted.

The "halogen" refers to fluoro (F), chloro (Cl), bromo (Br), or iodo (I).

The "amino" refers to-$NH_2$

The "cyano" refers to -CN.

The "nitro" refers to -$NO_2$

The "hydroxy" refers to -OH.

The "carboxyl" refers to -COOH.

The "thiol" refers to -SH.

The "carbonyl" refers to C=O.

"Stereoisomers" refer to isomeric compounds having the same linking order of atoms but different spatial arrangements of the atoms. Stereoisomers may have one or more stereocenters and each center may exist as R or S, and stereoisomers may also be cis-trans isomers. Stereoisomers of the compounds provided herein include any one of all diastereomeric, enantiomeric and cis-trans isomeric forms thereof, or suitable mixtures thereof.

The pharmaceutically acceptable salt includes those derived from the compound with a variety of organic and inorganic counterions well known in the art, and salts as examples only include organic or inorganic salts such as lithium salt, sodium salt, potassium salt, calcium salt, magnesium salt, ammonium salt, isopropylamine, trimethylamine, diethylamino, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethyl, piperidine, polyamine resin, and tetraalkylammonium salt when the molecule contains an acidic functional group; and organic or inorganic acid salts such as hydrochloride salt, hydrobromide salt, tartrate salt, mesylate salt, acetate salt, maleate salt, and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. These salts can generally be prepared by conventional methods by reacting, for example, an appropriate acid or base with the compound. The solvate includes hydrates.

Other chemical terms herein are used according to conventional usage in the art, such as the McGraw-Hill Dictionary of Chemical Terms (Ed. Parker, S., McGraw-Hill, San Francisco (1985)).

All the publications, and patents cited herein are incorporated by reference in their entirety for all purposes.

The antibody-drug conjugate of the present invention is a HER2 antibody-drug conjugate (HER2-ADC) formed by conjugating an antibody or an antigen-binding unit thereof that binds to HER2 to a drug. Antibody-drug conjugates (ADCs) can increase the therapeutic efficacy of antibodies in treating diseases (e.g., cancer) due to their ability to selectively deliver one or more drugs to a target tissue (e.g., a tumor expressing HER2). In one or more embodiments, the HER2-ADC of the present invention has properties including, but not limited to, binding to HER2 (e.g., human HER2) *in vitro,* binding to cells expressing HER2, high affinity, strong internalization capability, and reducing or inhibiting the growth of cancer cells or tumors.

In one or more embodiments, the HER2-ADC has a structure shown as formula I, or a stereoisomer, or a pharmaceutically acceptable salt or solvate thereof.

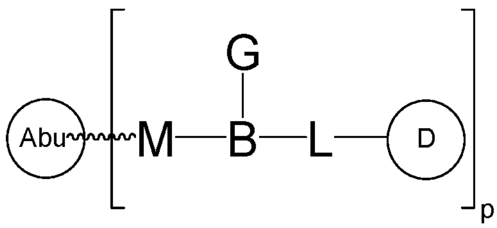

formula I

wherein Abu, M, B, G, L, D, and p are as defined herein.

In one or more embodiments, Abu is trastuzumab.

In one or more embodiments, the antibody-drug conjugate described herein has a significant bystander effect.

The present invention further provides a preparation method for intermediates and antibody-drug conjugates. The intermediates and the antibody-drug conjugates of the present invention can be prepared using known formulations and methods. In one or more embodiments, the preparation method is as follows.

Preparation method for compound as shown in formula II

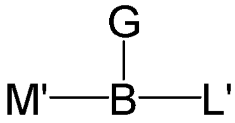

formula II

wherein

M' is

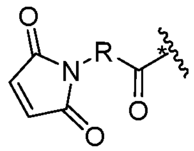

,

wherein * links to B, R is selected from: $-(CH_2)_r-$, $-(CHR^m)_r-$, C3-C8 carbocycl yl, $-O-(CH_2)_r-$, arylene, $-(CH_2)_r$-arylene-, -arylene-$(CH_2)_r-$, $-(CH_2)_r$-(C3-C8 carbocyclyl)-, -(C3-C8 carb ocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, - $(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC$ $(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 car bocyclyl, phenyl or benzyl; and each r is independently 1, 2, 3, 4, 5, 6, 7, 8, 9 or 10;

B is

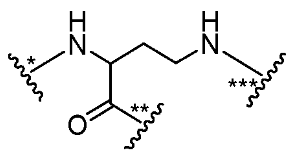

;

wherein * links to M', ** links to L', *** links to G;

L' is $-(AA)_i-(FF')_f$, wherein, AA, i, f are described as in formula I; FF' is

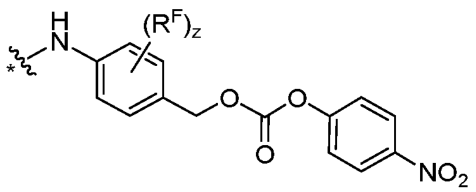

,

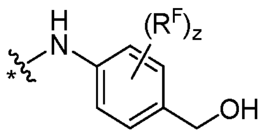

,

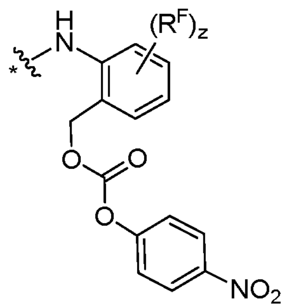

,

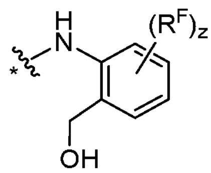

,

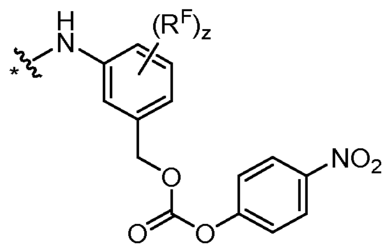

or

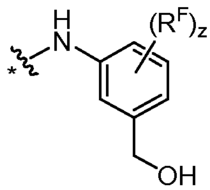

,

wherein $R^F$ is C1-C6 alkyl, C1-C6 alkoxy, $-NO_2$ or halogen; z is 0, 1, 2, 3 or 4; wherein * links to AA;

G is described as in formula I.

In one or more embodiments, FF' is

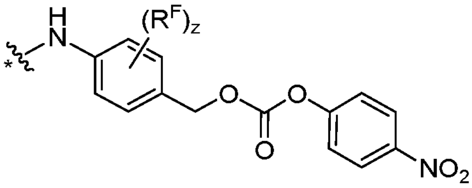, 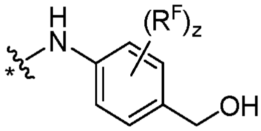,

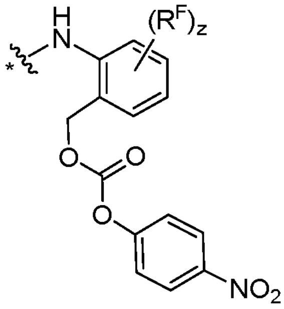, 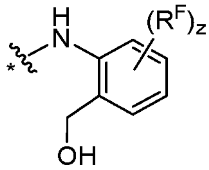, 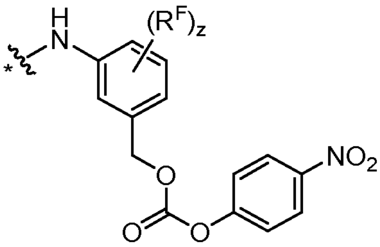 or 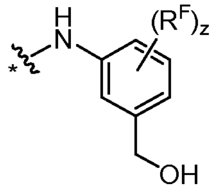,

wherein $R^F$ is C1-C6 alkyl, C1-C6 alkoxy, $-NO_2$ or halogen; wherein * links to AA.

In one or more embodiments, $R^F$ is F.

In one or more embodiments, z is 0.

In one or more embodiments, z is 1 or 2.

In one or more embodiments, B is links to G.

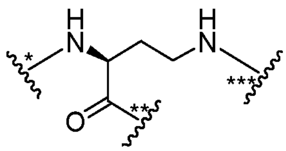;

wherein * links to M', ** links to L', ***

In one or more embodiments, FF' is

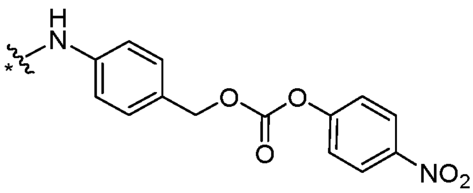, 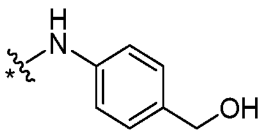,

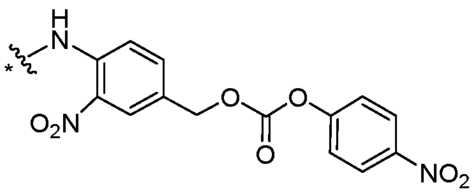, 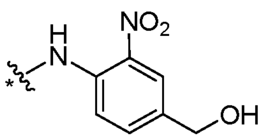,

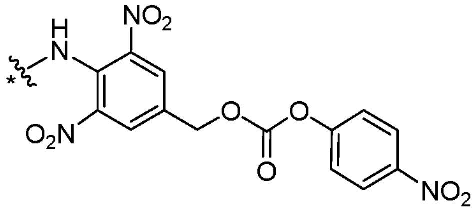 , 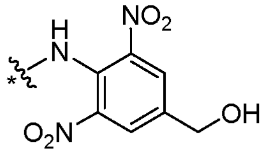 ,

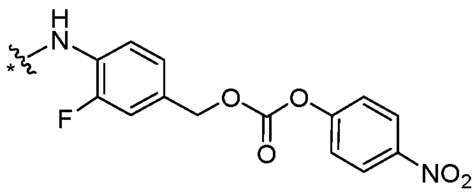 , 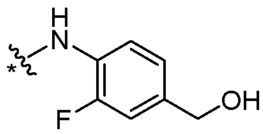 ,

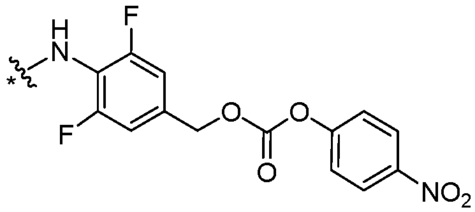 , 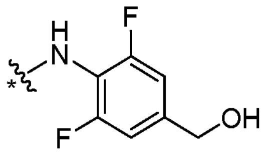 ,

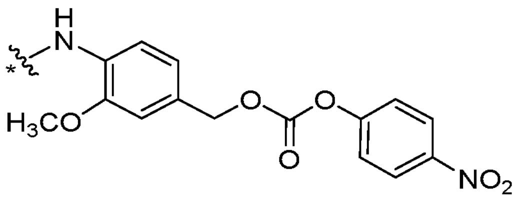 , 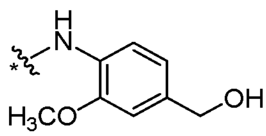 ,

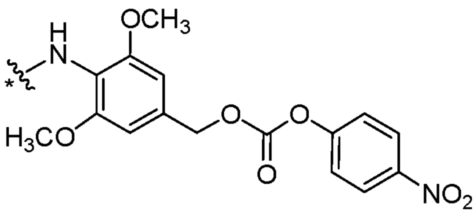 , 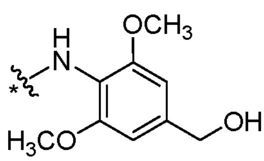 , 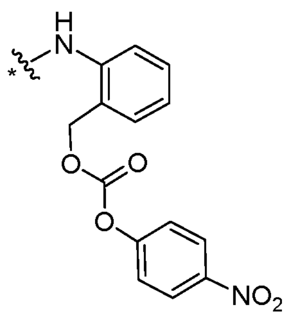 ,

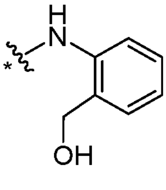 , 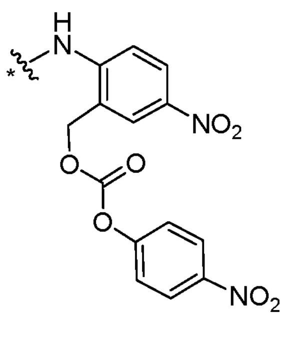 , 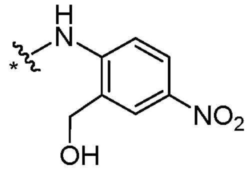 , 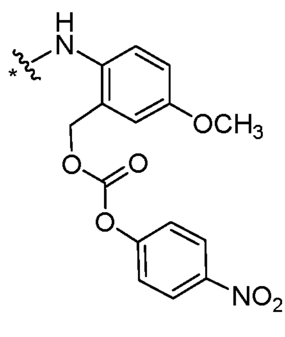 ,

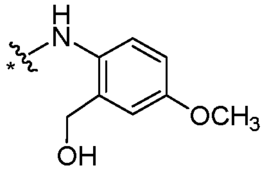 , 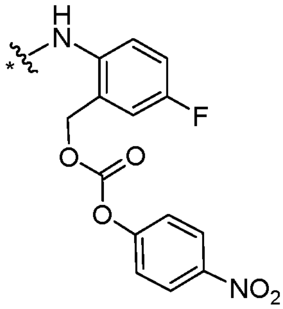 , 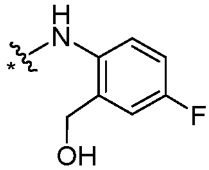 , 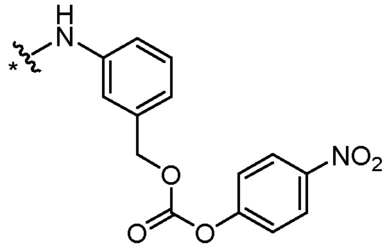 ,

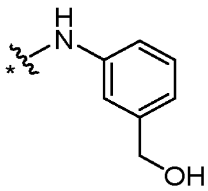 , 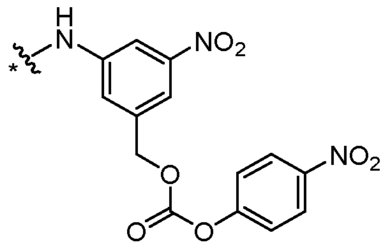 , 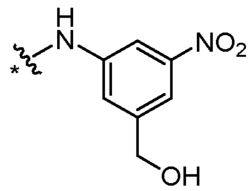 ,

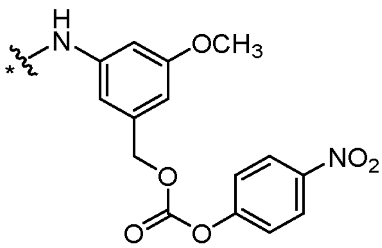 , 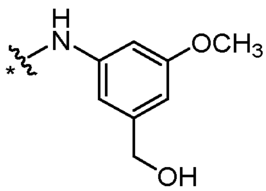 , 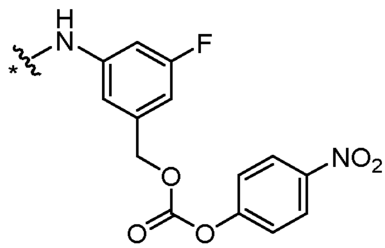 ,

or

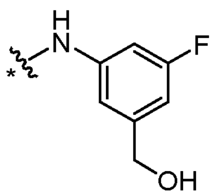 ;

wherein * links to AA.

In one or more embodiments, FF' is

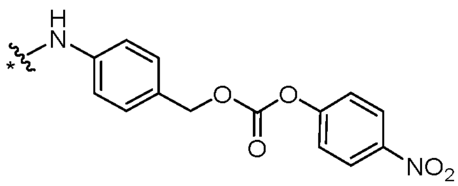 or 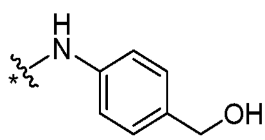 ,

f is 1; wherein * links to AA.

In one or more embodiments, L' is

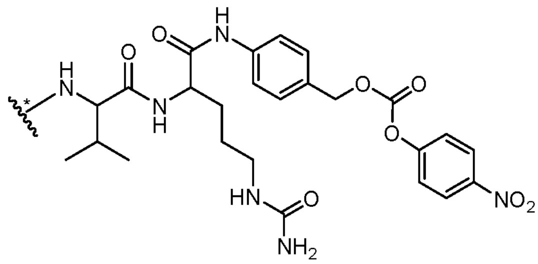

or

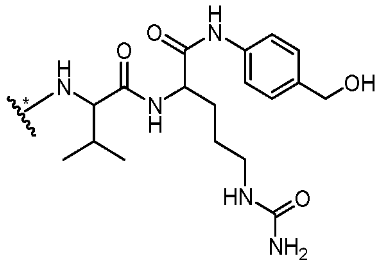

,

wherein * links to B.

In one or more embodiments, L' is

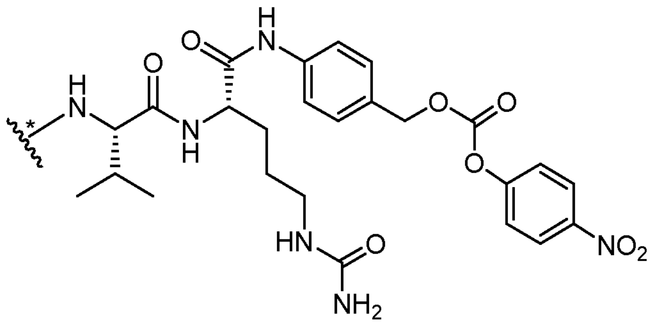

or

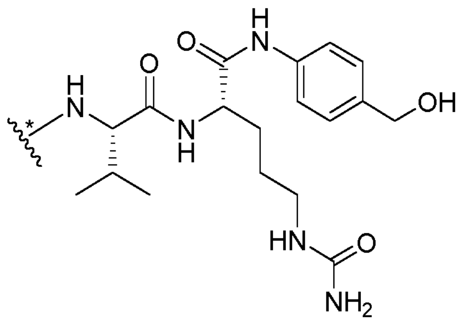

,

wherein * links to B.

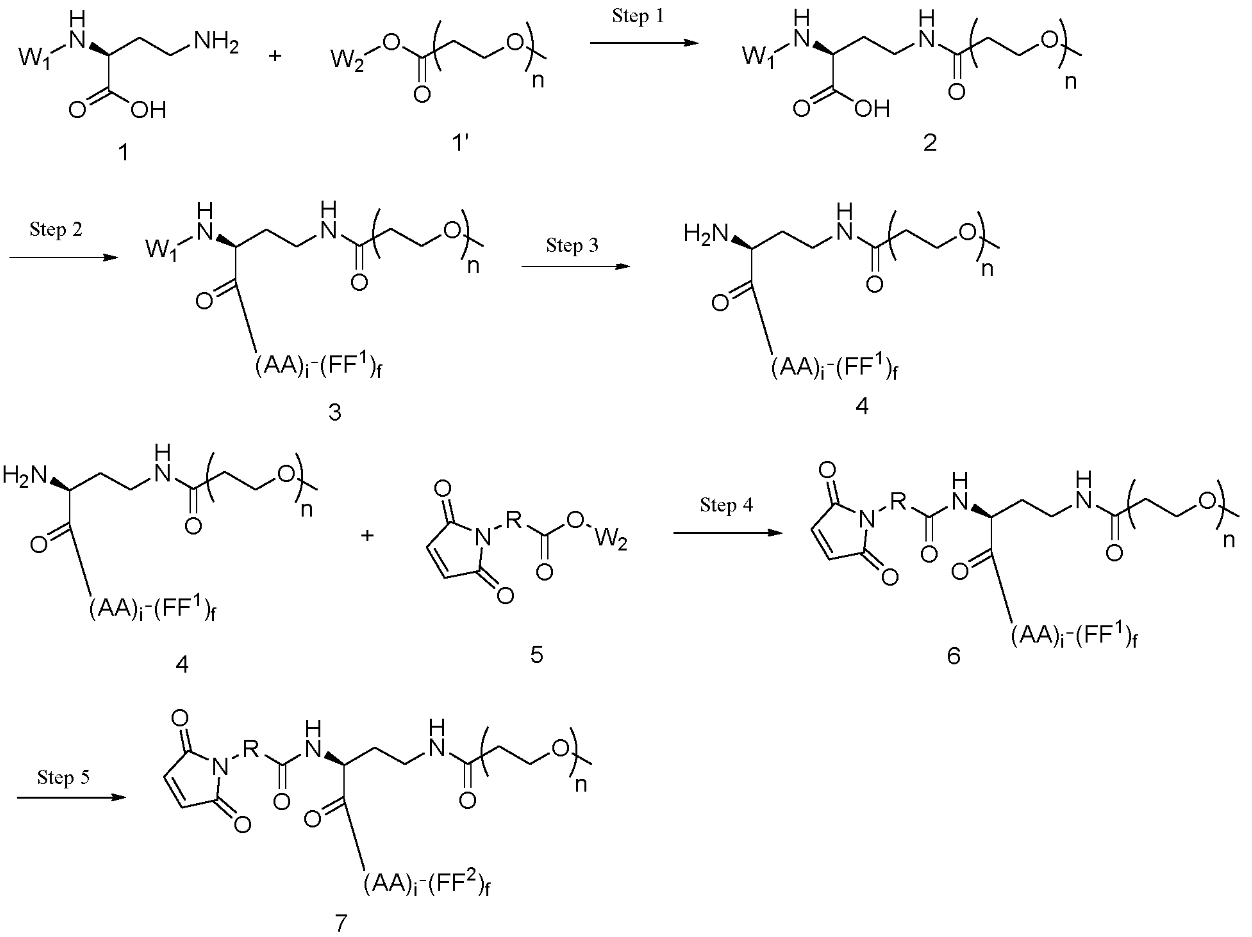


1 1' 2 3 4 4 5 6 7

Step 1: reacting a compound of general formula 1 with a compound of general formula 1' under a basic condition to obtain a compound of general formula 2;

Step 2: reacting the compound of general formula 2 with general formula $(AA)_i$-$(FF^1)_f$ in the presence of a condensing agent under a basic condition to obtain a compound of general formula 3;

Step 3: removing the amino-protecting group $W_1$ of the compound of general formula 3 to obtain a compound of general formula 4;

Step 4: reacting the compound of general formula 4 with a compound of general formula 5 under a basic condition to obtain a compound of general formula 6; and

Step 5: reacting the compound of general formula 6 with bis(p-nitrophenyl)carbonate under a basic condition to obtain a compound of general formula 7.

wherein

$W_1$ is an amino-protecting group, e.g., 9-fluorenylmethyloxycarbonyl; $W_2$ is a carboxylic acid active ester, for example, a succinimidyl ester.

wherein n, AA, R, i, f are described as in formula II herein, FF' is

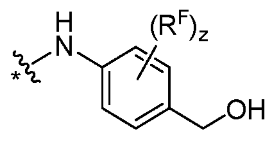

,

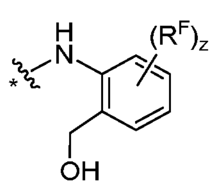 or 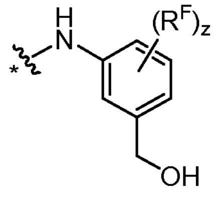 ,

wherein $R^F$ is C1-C6 alkyl, C1-C6 alkoxy, $-NO_2$ or halogen; z is 0, 1, 2, 3 or 4; wherein * links to AA; $FF^2$ is

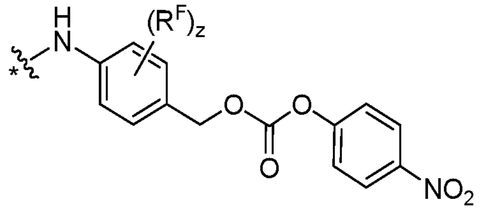 ,

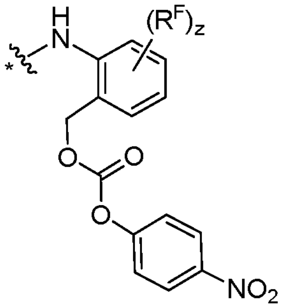 or 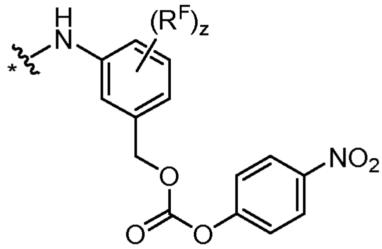 ,

wherein $R^F$ is C1-C6 alkyl, C1-C6 alkoxy, $-NO_2$ or halogen;z is 0, 1, 2, 3 or 4;wherein * links to AA.

In one or more embodiments, $R^F$ is F.

In one or more embodiments, z is 0.

In one or more embodiments, z is 1 or 2.

The basic conditions described above can be provided using reagents including organic bases and inorganic bases, wherein the organic bases include, but are not limited to, triethylamine, diethylamine, A-methylmorpholine, pyridine, piperidine, *N*,*N*-diisopropylethylamine, n-butyllithium, lithium diisopropylamide, potassium acetate, sodium tert-butoxide, or potassium tert-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N',N'-tetramethyl-O-(7-azabenzotriazol-1-yl) urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azobenzotriazol, O-benzotriazol-N,N,N',N'-tetramethyluronium hexafluorophosphate, 2-(7-azobenzotriazol)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method for intermediate as shown in formula III

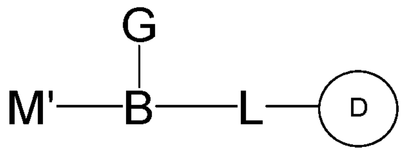

formula III

wherein, G, L, D are described as in formula I, M' is described as in formula II, B is

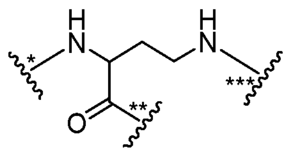

,

wherein * links to M' ,** links to L, *** links to G.

In one or more embodiments, B is

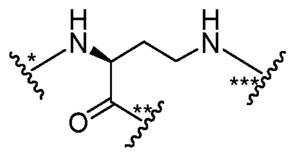

,

wherein * links to M', ** links to L, *** links to G.

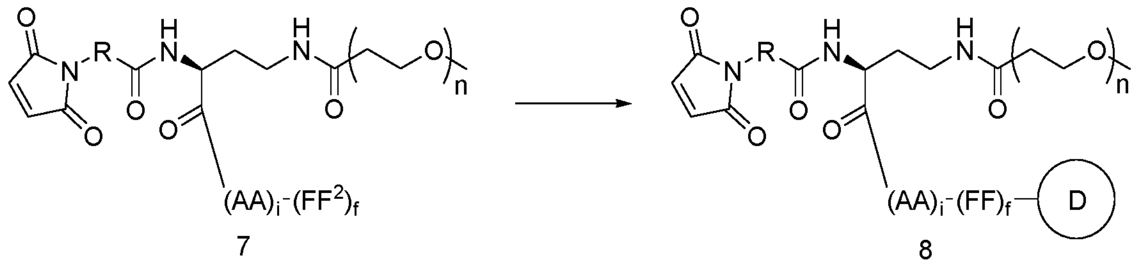

7

8

wherein n, AA, R, i, f, $FF^2$, FF, D are described herein.

The compound of general formula 7 and D are reacted in the presence of a condensing agent under a basic condition to obtain a compound of general formula 8.

The basic conditions described above can be provided using reagents including organic bases and inorganic bases, wherein the organic bases include, but are not limited to, triethylamine, diethylamine, A-methylmorpholine, pyridine, piperidine, *N*,*N*-diisopropylethylamine, n-butyllithium, lithium diisopropylamide, potassium acetate, sodium tert-butoxide, or potassium tert-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N',N'-tetramethyl-O-(7-azabenzotriazol-1-yl) urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azobenzotriazol, O-benzotriazol-N,N,N',N'-tetramethyluronium hexafluorophosphate, 2-(7-azobenzotriazol)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method for

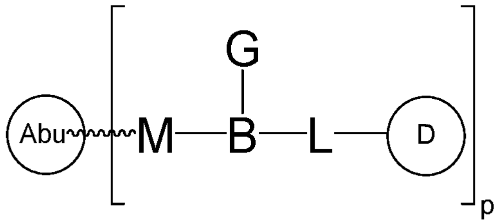

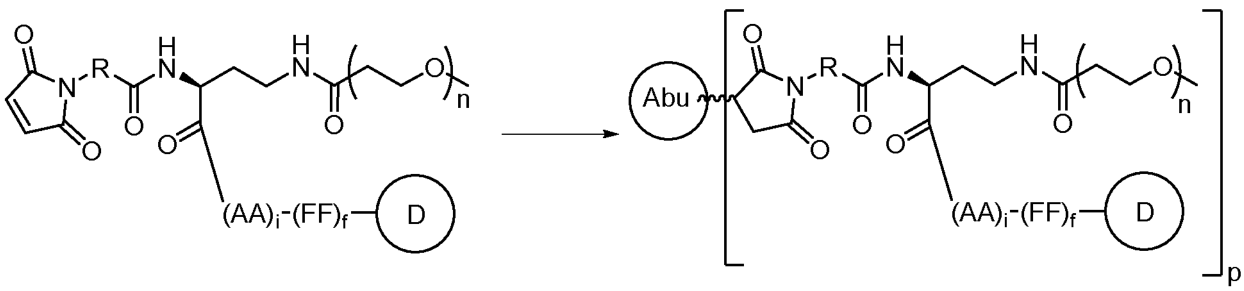

8 9

wherein n, AA, R, i, f, FF, D and Abu are described herein.

The compound of general formula 8 is conjugated to Abu under a weakly acidic condition to obtain a compound of formula 9.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, acetic acid, hypochlorous acid, hydrofluoric acid, sulfurous acid, hydrosulohuric acid, silicic acid, metasilicic acid, phosphoric acid, metaphosphoric acid, sodium bicarbonate, and sodium bisulfite.

In one or more embodiments, the ADC of the present invention may be used in combination with other therapeutic or prophylactic regimens, including use of one or more ADCs of the present invention together with or in combination with one or more other therapeutic agents or methods. For combination therapy, the ADC may be administered simultaneously or separately with other therapeutic agents. When administered separately, the ADC of the present invention may be administered before or after the administration of another additional therapeutic agent.

In one or more embodiments, when administering the ADC of the present invention to a patient, a HER2-targeted antibody, such as pertuzumab (e.g., Perjeta® or a biosimilar thereof, or a corresponding monoclonal antibody with an enhanced ADCC effect thereof, or a partially or totally defucosylated monoclonal antibody) may also be administered in combination with the ADC of the present invention to the patient.

## Examples

The materials, reagents, etc., used in the following examples can be commercially available or obtained using known methods unless otherwise stated.

### Example 1. Preparation of Antibodies

The following antibodies were prepared using conventional methods. After vector construction, eukaryotic cells such as HEK293 cells (Life Technologies Cat. No. 11625019) were transiently transfected, or CHO cells were stably transfected, and stable cell lines were selected and purified for expression. Preparation and purification of trastuzumab antibody: Referring to the method of Wood et al., J Immunol., 145: 3011 (1990), etc., anti-HER2 antibody, a monoclonal antibody that specifically binds to the extracellular domain of HER2, was produced in CHO cells. Expression vectors OptiCHO™ Antibody Express System (invitrogen) containing antibody genes were constructed using conventional molecular biological methods, with CHO cells as host cells.

**Example 2. Synthesis Procedures for Compound (1S,9S)-1-amino-9-ethyl-4,5-difluoro-9-hydroxy-1,2,3,9,12,15-hexahydro-10H,13H-benzo [de]pyrano [3',4':6,7]indolizino [1,2-b] quinolin-10,13-dione hydrochloride (D-1)**

1. Synthesis of*N*,*N*'-(3,4-difluoro-8-oxo-5,6,7,8-tetrahydronaphthalene-1,7-diyl)diacetamide

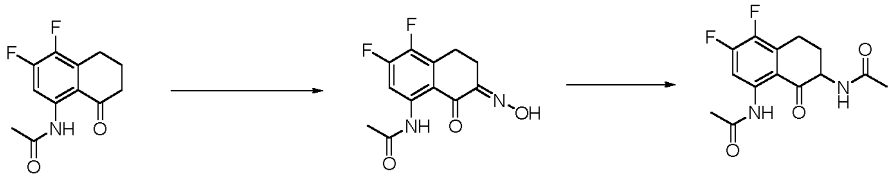

A solution of potassium tert-butoxide in tetrahydrofuran (42 mL, 1 M) was added to a dry reaction flask under a nitrogen atmosphere, stirred, and cooled to 0-5 °C. N-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by tert-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). With temperature maintained at 5-10 °C, zinc powder (8 eq) was added according to the amount of N-(3,4-difluoro-8-oxo-5,6,7,8-tetrahydronaphthalen-1-yl)acetamide. The mixture was stirred at 20-25 °C for 1 h. The mixture was filtered, and the solid was rinsed with ethyl acetate (50 mL). The temperature was reduced to 0-5 °C, and 15% aqueous solution of $NaCO_3$ (50 mL) was added dropwise for three washings. Ethyl acetate (25 mL) was added for extraction. The organic phases were pooled and washed with a saturated aqueous solution of NaCl. Ethyl acetate (10 mL) was added, and the mixture was stirred at 40 °C for 30 min, slowly cooled to 0-5 °C, and stirred for 2 h. The mixture was filtered, and the solid was washed with ethyl acetate/petroleum ether (1/2, 10 mL). Drying was performed in vacuo to obtain a gray powder (2.1 g, 33.7%). LC-MS: $[M+H]^+$=297。

2. Synthesis of N-(8-amino-5,6-difluoro-1-oxo-1,2,3,4-tetrahydronaphthalen-2-yl)acetamide

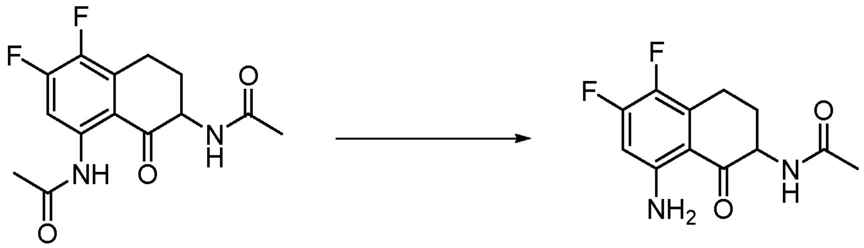

*N*,*N'*-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The resulting mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: $[M+H]^+$=255。

3. Synthesis of *N*-((9*S*)-9-ethyl-4,5-difluoro-9-hydroxy-10,13-dioxo-2,3,9,10,13,15-hexahydro-1*H*,12*H*-benzo[*de*]pyrano[3';4':6,7]indolizino[1,2-*b*]quinolin-1-yl)acetamide

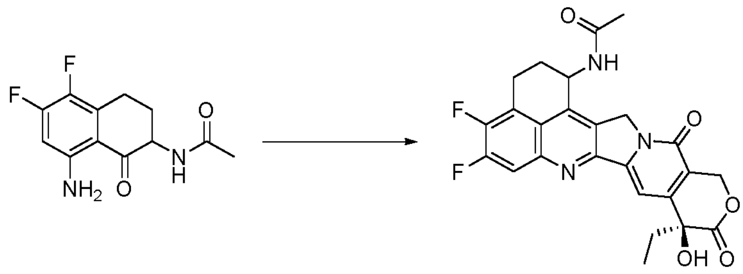

N (8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-*f*]indolizine-3,6,10(4*H*)-trione (1.15 g, 1 eq) and toluene (50 ml) were added to a reaction flask under a nitrogen atmosphere. The mixture was warmed to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: $[M+H]^+$=482。

4. Synthesis of (1*S*,9*S*)-1-amino-9-ethyl-4,5-difluoro-9-hydroxy-1,2,3,9,12,15-hexahydro-10*H*,13*H*-benzo [de]pyrano [3 ',4': 6,7]indolizino [1,2-*b*]quinolin-10,13-dione hydrochloride

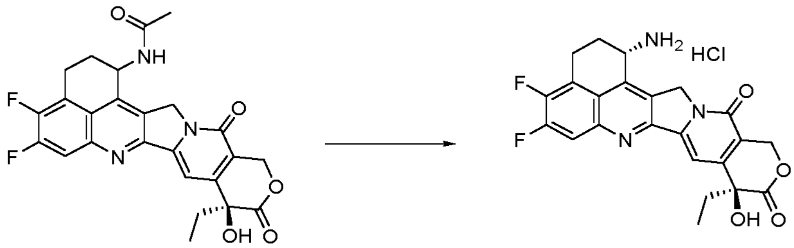

N-((9S)-9-ethyl-4,5-difluoro-9-hydroxy-10,13-dioxo-2,3,9,10,13,15-hexahydro-1H,12H-benzo[de]pyrano [3',4':6,7]indolizino[1,2-b]quinolin-1-yl)acetamide (1.1 g, 2.28 mmol) and a 6 M aqueous solution of hydrochloric acid (44 mL) were added to a reaction flask. The mixture was refluxed with stirring under a nitrogen atmosphere for 4 h. The mixture was concentrated to remove the solvent, and the residue was purified by HPLC to obtain a white powder (200 mg, 18%). LC-MS: $[M+H]^+$=440。

**Example 3. Synthesis Procedures for 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amino)benzyl (4-nitrophenyl) carbonate (CB07)**

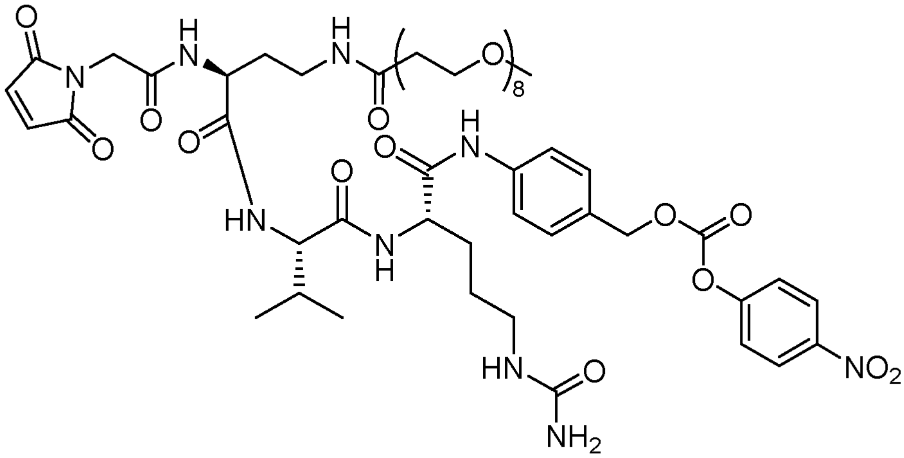

1) Synthesis of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01).

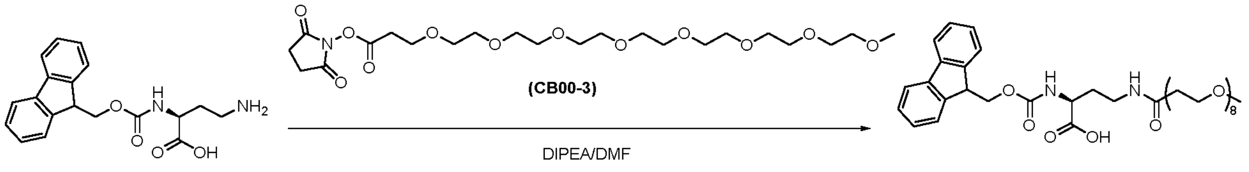


(CB00-2) (CB00-3) (CB01)

Under a nitrogen atmosphere at 0-5 °C, 8.14 g of N2-fluorenylmethoxycarbonyl-L-2,4-diaminobutyric acid (CB00-2) was dissolved in 40 mL of dimethylformamide (DMF), and 10 g of A-succinimidyl 4,7,10,13,16,19,22,25-octaoxahexacosanoate (CB00-3) and 10 mL of DMF were added. 6.5 mL of DIPEA was dropwise added with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol volume ratio 20:1 as elution solvents) to obtain CB01 in the form of a pale yellow oil (14.2 g).

2) Synthesis of (9*H*-fluoren-9-yl)methyl [(*S*)-1-[[(*S*)-1-[[4-(hydroxymethyl)phenyl] amino]-1-oxo-5 -ureidopentan-2-yl] amino] -3-methyl-1-oxobutan-2-yl]carbamate (CB02).

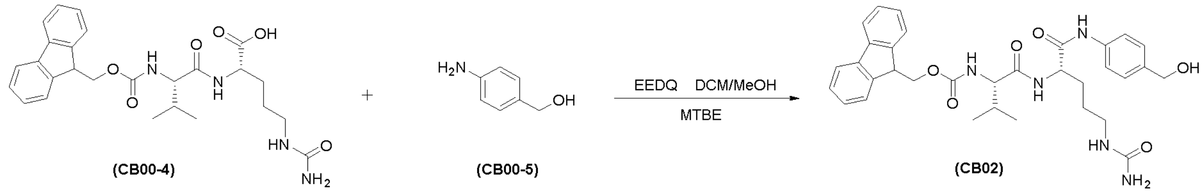


(CB00-4) (CB00-5) (CB02)

11 g of (*S*)-2-((*S*)-2-((((9*H*-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol at room temperature under a nitrogen atmosphere, and 17 g of 2-ethoxy-1-ethoxycarbonyl-1,2-dihydroquinoline (EEDQ) were added in portions with mechanical stirring. The resulting mixture was allowed to react for 15 h in the absence of light. After the completion of the reaction, the solvent was removed under reduced pressure to obtain a paste solid, which was subjected to silica gel column chromatography (with dichloromethane and methanol volume ratio 20:1 as elution solvents) to obtain an off-white solid (11.9 g).

3) Synthesis of (*S*)-2-((*S*)-2-amino-3-methylbutanamido)-*N*-(4-(hydroxymethyl)phenyl)-5-ureidopentanamide (CB03)

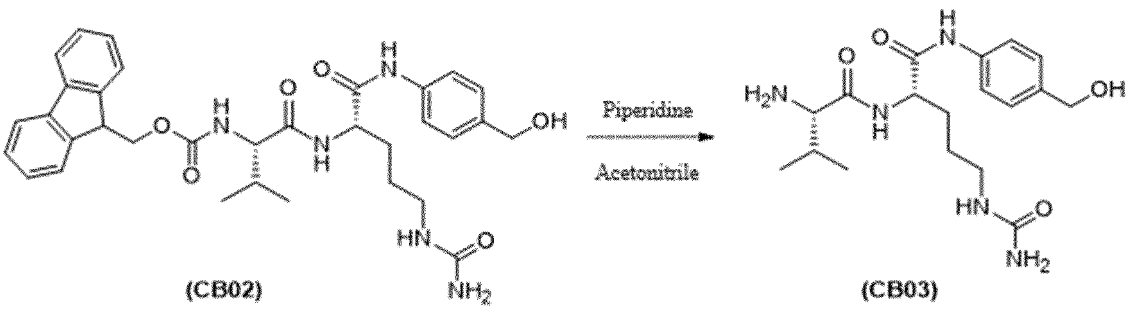


(CB02) (CB03)

Under a nitrogen atmosphere at room temperature, 300 mL of acetonitrile was added to 11.9 g of (9H-fluoren-9-yl)methyl [(S)-1-[[(S)-1-[[4-(hydroxymethyl)phenyl]amino]-1-oxo-5 -ureidopentan-2-yl]amino]-3-methyl-1-oxobutan-2-yl]carbamate (CB02), and 18 mL of piperidine was added dropwise with stirring. After the dropwise addition, the mixture was allowed to react at room temperature for 2 h. After the completion of the reaction, the mixture was distilled under reduced pressure to remove the solvent and piperidine, and the residue was subjected to silica gel column chromatography (with dichloromethane and methanol volume ratio 20:1 as elution solvents) to obtain CB03 in the form of a white solid (7.5 g).

4) Synthesis of (9*H*-fluoren-9-yl)methyl ((30S,33S, 36S)-41-amino-36-((4-(hydroxymethyl)phenyl)carbamoyl)-33-isopropyl-26,31,34,41-tetraoxo-2,5,8, 11, 14, 17,20,23-octaoxa-27,32,35,40-tetraaza-30-yl)carbamate (CB04).

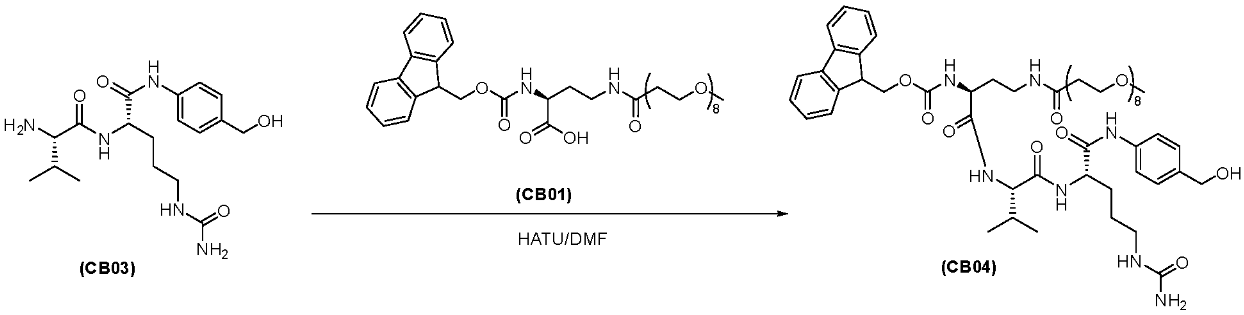

(CB03) (CB04)

At 0 °C under a nitrogen atmosphere, 14.2 g of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01) was dissolved in 100 mL of DMF, and 11 g of *N*,*N*,*N'*,*N'*-tetramethyl-*O*-(7-azabenzotriazol-1-yl)urea hexafluorophosphate (HATU) was added in portions. After 30 min of stirring, 7.5 g of (*S*)-2-((*S*)-2-amino-3-methylbutanamido)-*N*-(4-(hydroxymethyl)phenyl)-5-ureidopentanamide (CB03) was added, and the mixture was allowed to react for 2.5 h with 0 °C maintained. After the completion of the reaction, the mixture was distilled under reduced pressure to remove the solvent, and the residue was subjected to silica gel column chromatography (with dichloromethane and methanol volume ratio 10: 1 as elution solvents) to obtain CB04 in the form of a solid (9.66 g).

5) Synthesis of *N*-((*S*)-3-amino-4-(((*S*)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5, 8,11,14,17,20,23-octaoxahexacosan-26-amide (CB05).

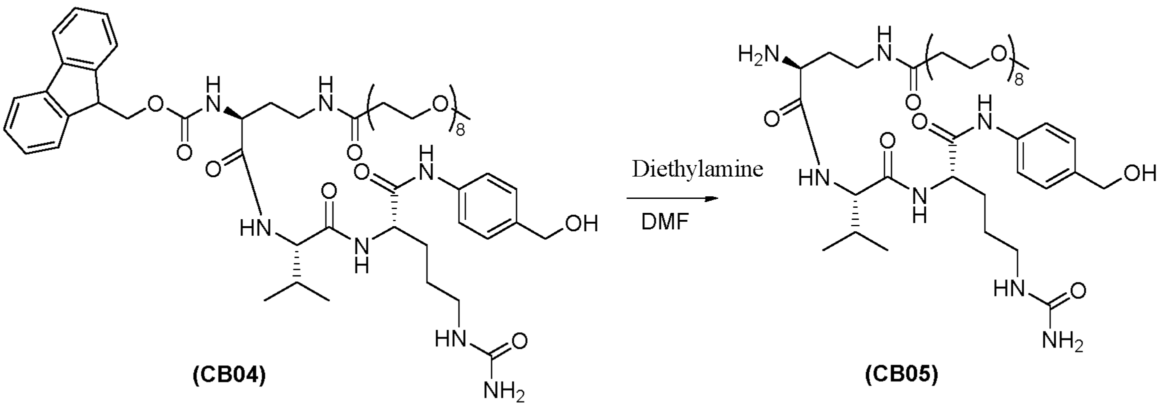

(CB04) (CB05)

9.66 g of (9*H*-fluoren-9-yl)methyl ((30S,33S, 36S)-41-amino-36-((4-(hydroxymethyl)phenyl)carbamoyl)-33-isopropyl-26,31,34,41-tetraoxo-2,5,8, 11, 14, 17,20,23-octaoxa-27,32,35,40-tetraaza-30-yl)carbamate (CB04) was dissolved in 50 mL of DMF at room temperature under a nitrogen atmosphere, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol volume ratio 7.5:1 as elution solvents) to obtain CB05 in the form of a pale yellow solid (7.7 g).

6) Synthesis of N-((*S*)-3 -(2-(2,5-dioxo-2,5-dihydro-1*H*-pyrrol-1-yl)acetamido)-4-(((*S*)-1-(((*S*)-1-((4-(hydroxymethyl) phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

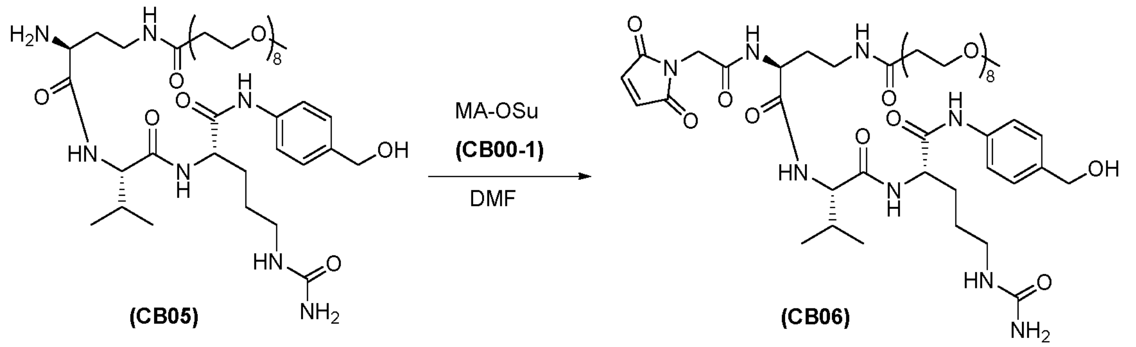


(CB05) (CB06)

7.7 g of N-((*S*)-3-amino-4-(((*S*)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5°C. The mixture was allowed to react at 0-5°C for 4 h. After the completion of the reaction, the mixture was distilled under reduced pressure to remove the solvent, and the residue was subjected to silica gel column chromatography (with dichloromethane and methanol as volume ratio 10:1 elution solvents) to obtain CB06 in the form of a pale yellow solid (9.5 g).

7) Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amino)benzyl (4-nitrophenyl) carbonate (CB07).

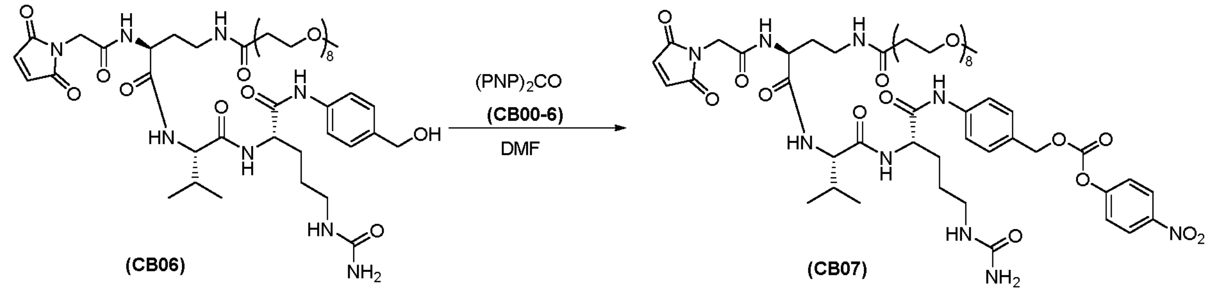


(CB06) (CB07)

9.5 g of *N*-((*S*)-3-(2-(2,5-dioxo-2,5-dihydro-1*H*-pyrrol-1-yl)acetamido)-4-(((*S*)-1-(((*S*)-1-((4-(hydroxymethyl) phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(p-nitrophenyl)carbonate ($(PNP)_2CO$) was added under a nitrogen atmosphere at 0 °C, followed by 8.2 mL of *N*,*N*-diisopropylethylamine (DIPEA) after dissolution. The mixture was allowed to react for 4 h with the 0 °C maintained. After the completion of the reaction, the mixture was distilled under reduced pressure to remove the solvent, and the residue was subjected to silica gel column chromatography (with dichloromethane and methanol volume ratio 8:1 as elution solvents) to obtain CB07 in the form of a white solid (2.6 g).

**Example 4. Synthesis of 4-((18S,21S,24S)-18-(2-(2,5-dioxo-2,5-dihydro-1Hpyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (CB14).**

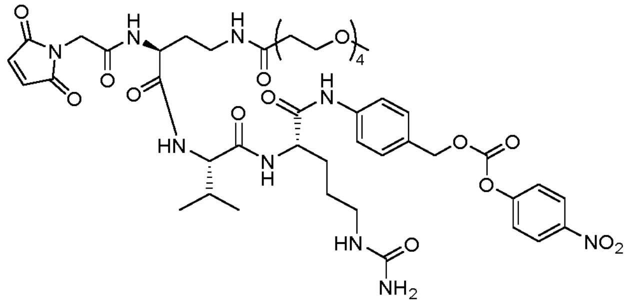

(CB14)

CB14 was prepared by referring to the synthesis of CB07 in Example 3, with A-succinimidyl 4,7,10,13,16,19,22,25-octaoxahexacosanoate replaced by A-succinimidyl 4,7,10,13-tetraoxatetradecanoate. CB 14 was eventually obtained in the form of a white solid.

**Example 5. Synthesis of Intermediate (CB07-Exatecan)**

Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)carbamate.

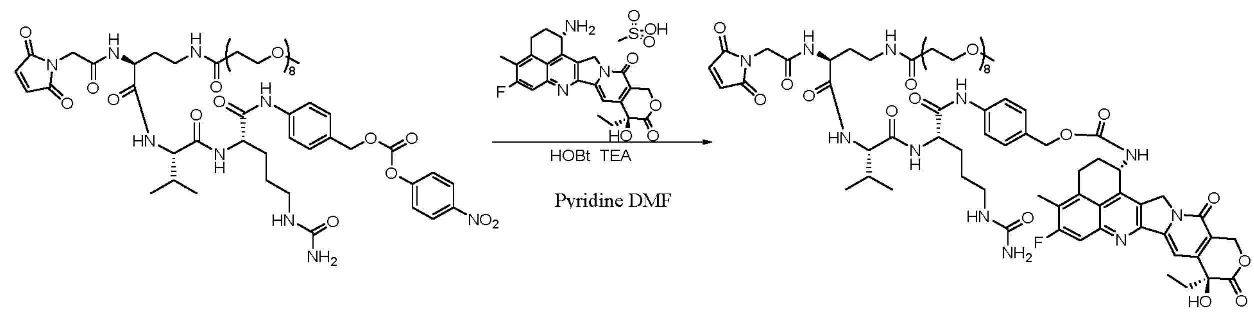


4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (2.6 g, 2.21 mmol) and N,N-dimethylformamide (23 mL) were added to a reaction flask R1, stirred under a nitrogen atmosphere and cooled to 0-5 °C. At the same time, (1S,9S)-1-amino-9-ethyl-5-fluoro-9-hydroxy-4-methyl-1,2,3,9,12,15-hexahydro-10H,13H-benzo[3',4':6,7]indolizino[1,2-b]quinolin-10,13-dione methanesulfonate (Exatecan, 0.98 g, 1.84 mmol, Advanced ChemBlocks) and N,N-dimethylformamide (5 mL) were taken and added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with N,N-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5°C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder (1.6 g, 59%). LC-MS: $[1/2M+H]^+$=737。

**Example 6. Synthesis of Intermediate (CB07-D-1)**

Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5, 8,11,14,17,20,23-octaoxa-27,32,35-triazaheptatriacontan-37-amido)benzyl ((1S,9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)carbamat.

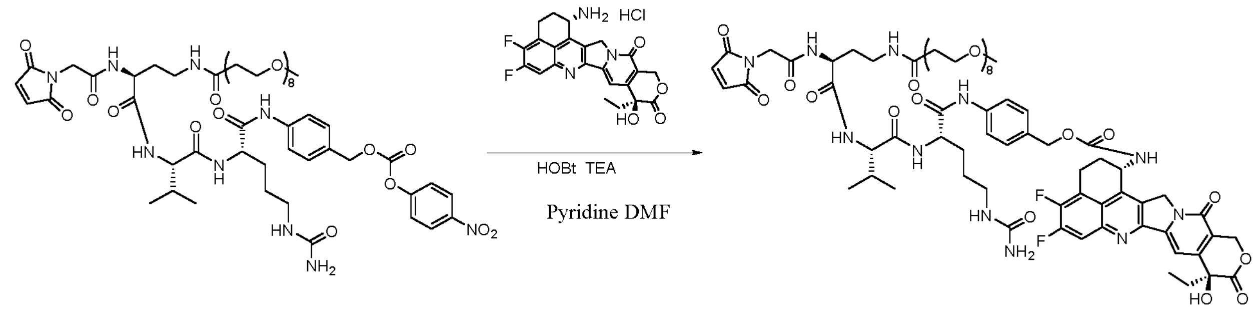


4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37 - amido)benzyl(4-nitrocarbonate) (220 mg, 0.189 mmol) and *N*,*N*-dimethylformamide (5 mL) were added to a reaction flask R1, stirred under a nitrogen atmosphere and cooled to 0-5 °C. At the same time, (1*S*,9*S*)-1-amino-9-ethyl-4,5-difluoro-9-hydroxy-1,2,3,9,12,15-hexahydro-10*H*,13*H*-benzo[de]pyrano[3',4':6,7]indolizino[1,2-b]quinoline-10,13-dione hydrochloride (D-1) (90 mg, 0.189 mmol) and *N*,*N*-dimethylformamide (5 mL) were taken and added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5°C for 10 min, warmed to room temperature, and stirred for 3 h. After the reaction was completed, the mixture was concentrated under reduced pressure to remove the solvent. The residue was purified by preparative high performance liquid chromatography (prep-HPLC) and lyophilized to obtain a white powder (55 mg, 20%). LC-MS: $[1/2M+H]^+$=739。

**Example 7. Synthesis of Intermediate (CB14-Exatecan)**

Synthesis of 4-((18*S*,21*S*,24*S*)-18-(2-(2,5-dioxane-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)-(1*S*,9*S*)-9-ethyl-5-fluoro-9-hydroxy-4-methyl-10,13-dioxo-2,3,9,10,13,15-hexahydro-1*H*,12*H*-benzo[*de*]pyran[3',4':6,7]indolizino[1,2-*b*]quinolin-1-carbamate.

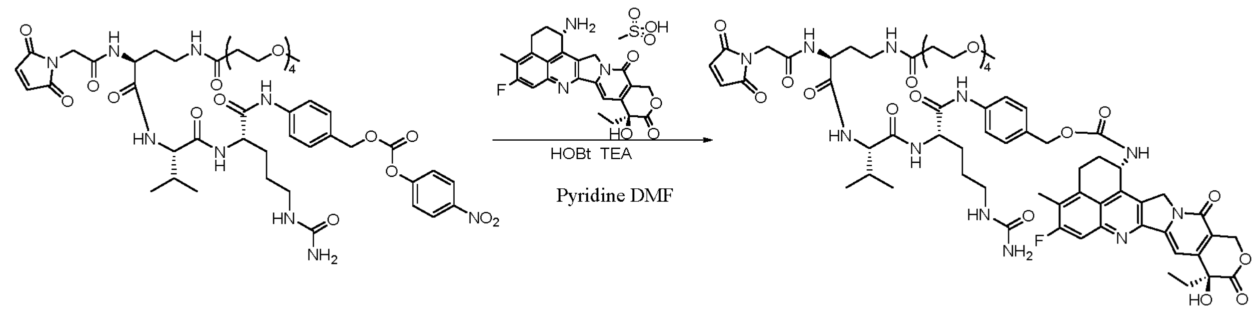


Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (190 mg, 0.19 mmol)and *N*,*N*-dimethylformamide (23 mL) were added to a reaction flask R1, stirred under a nitrogen atmosphere, and cooled to 0-5 °C. At the same time, (1*S*,9*S*)-1-amino-9-ethyl-5-fluoro-9-hydroxy-4-methyl-1,2,3,9,12,15-hexahydro-10*H*,13*H*-benzo[3',4':6,7]indolizino[1,2-*b*]quinoline-10,13-dione methanesulfonate (Exatecan methanesulfonate; 101 mg, 0.19 mmol; Advanced ChemBlocks) and *N*,*N*-dimethylformamide (5 mL) were taken and added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N*,*N*-dimethylformamide (1 mL), and the washing solution was added to reaction flask R1. 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder.

**Example 8: Synthesis of ADC1**

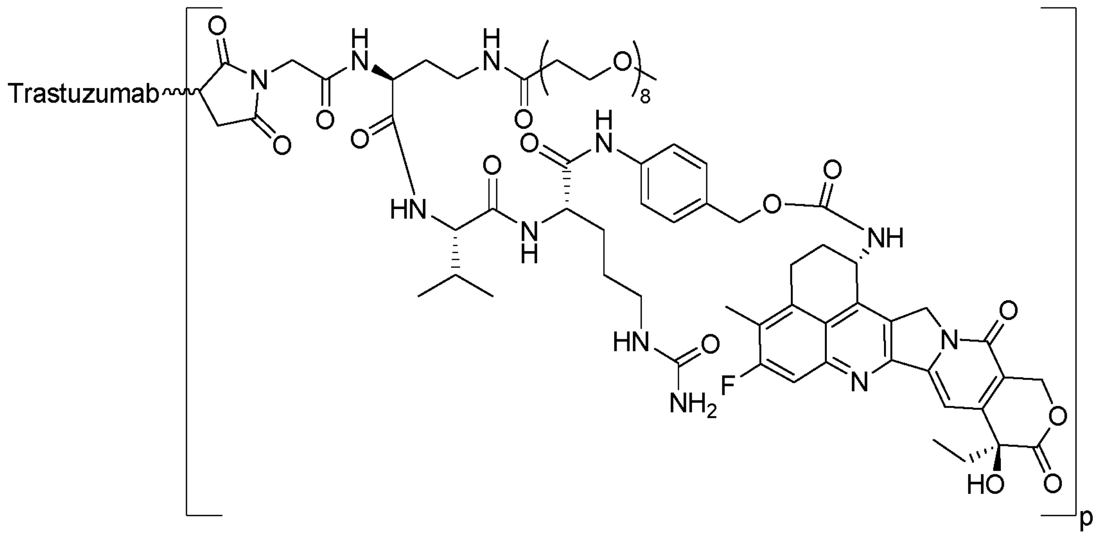

4.5 molar equivalents of TCEP (tris(2-carboxyethyl)phosphine) was added to Trastuzumab, and then the system was adjusted to pH 8 with 1 M Tris base. The system was incubated at 25 °C for 1.5 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of CB07-Exatecan was added according to the amount of the antibody substance, and after stirring at 25 °C for 1 h, 0.1 M N-acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The final concentration of ADC 1 obtained was 7.5 mg/mL, and the DAR, i.e., p, was 8 as measured by reversed-phase chromatography.

**Example 9: Synthesis of ADC2**

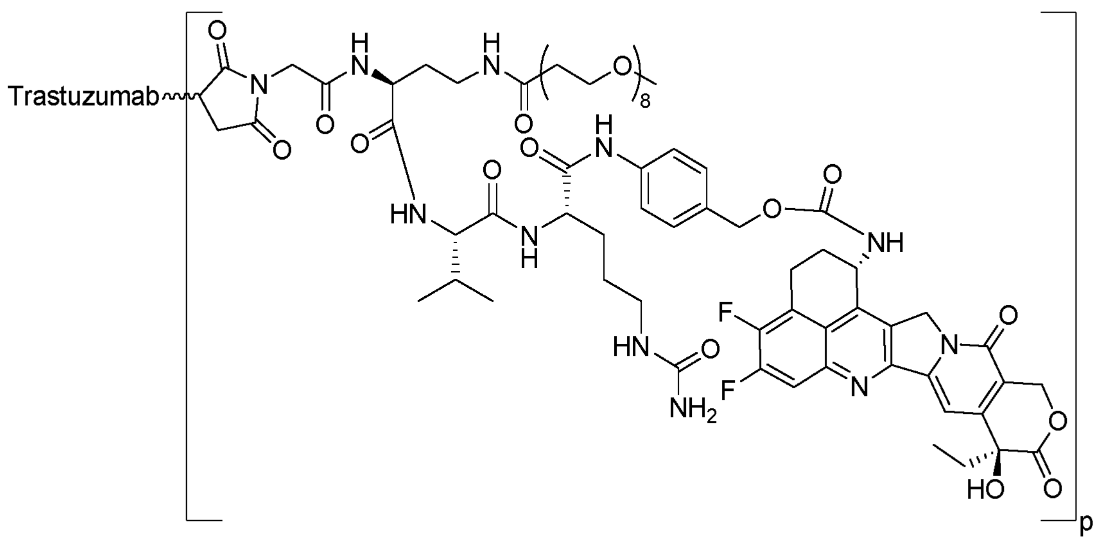

To Trastuzumab was added 4.5 molar equivalents of TCEP, and then the system was adjusted to pH 8 with 1 M Tris base. The system was incubated at 25 °C for 1.5 h for reduction. TCEP was removed by ultrafiltration for buffer

exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of CB07-D-1 was added, and after stirring at 25°C for 2 h, 0.1 M acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The concentration of ADC2 was 3.61 mg/mL, and the DAR, i.e., p, was 7.4 as measured by reversed-phase chromatography.

**Example 10: Synthesis of ADC3**

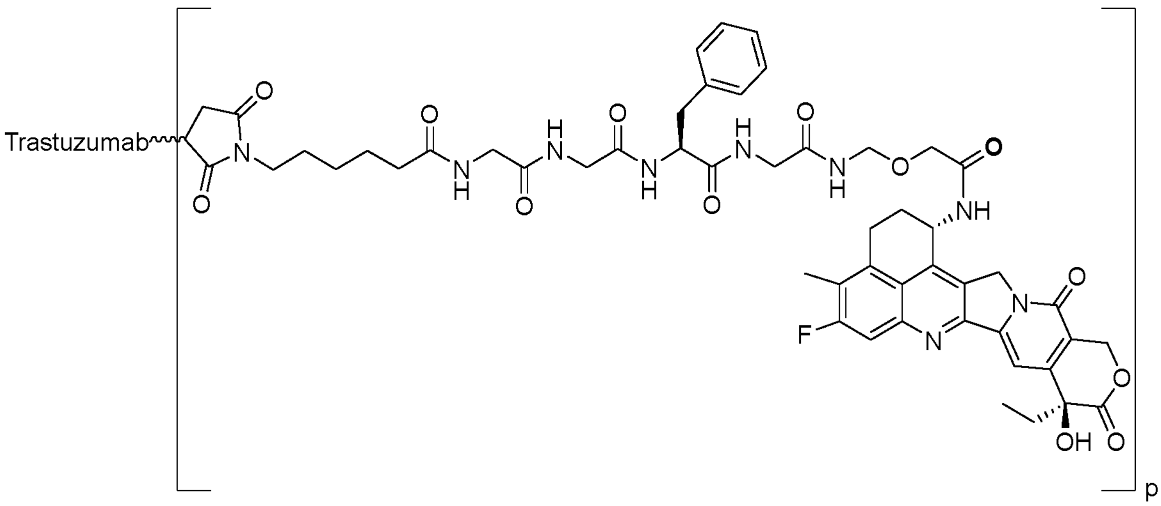

To Trastuzumab was added 4.5 molar equivalents of TCEP, and then the system was adjusted to pH 8 with 1M Tris base. The system was incubated at 25°C for 1.5h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of Deruxtecan (MCE, Cat.#HY-13631E) was added, and after stirring at 25 °C for 2 h, 0.1 M acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

The concentration of ADC3 was 4.19 g/L, and the DAR, i.e., p, was 7.6 as measured by RP-HPLC.

**Example 11: *In Vitro* Bioactivity of ADC1**

The inhibition of the growth of tumor cells by ADC1 antibody was assessed using HER2 positive breast tumor cell lines NCI-N87, MDA-MB-453, SK-BR-3 and BT474, as well as HER2 negative cell line MDA-MB-468 (purchased from Cell Bank of Chinese Academy of Sciences, Shanghai Institutes for Biological Sciences). Briefly, the cells were dissociated by digestion with trypsin after growing in the logarithmic phase and then suspended in 100 μL of complete medium. 4000-8000 cells were seeded in a 96-well plate for culture at 37 °C for 3-5 h or overnight, adhering to the walls. Then 100 μL of media with different concentrations of ADC 1 were added. After 120 h, the media in the Petri dish was removed, and relative cell proliferation analysis was performed using cell counting kit-8 (CCK-8, Dojindo, Japan) reagent. The results show that ADC1 has a growth inhibition effect on all of the HER2 positive cells, and the growth inhibition effect $EC_{50}$ on the negative cells MDA-MB-468 > 10000 ng/mL.

Table 1

| Cell line | $EC_{50}$ (ng/mL) ADC1 |
|---|---|
| NCI-N87 | 82 |

(continued)

| Cell line | $EC_{50}$ (ng/mL) ADC1 |
|---|---|
| MDA-MB-453 | 36 |
| SK-BR-3 | 43 |
| BT474 | 62 |
| MDA-MB-468 | > 10000 |

**Example 12: Bystander Effects of ADC1 and ADC2**

The HER2 positive cells SK-BR-3 and HER2 negative cells MDA-MB-468 were seeded into a 6-well plate in a density ratio of 75 thousand cells/well : 200 thousand cells/well. After 3-5 h of culture, ADC 1 and the control drug ADC3 were added at final concentrations of 0.1 nM, 0.5 nM and 5 nM, and 0.3 nM, 2 nM and 20 nM ADC2 was added. After 120 h of culture at 37 °C, the cells were digested with pancreatin and washed once with PBS after termination. The cells were counted, and then labeled with an FITC-labeled anti-HER2 antibody (different from Trastuzumab binding epitope) at 4 °C for 30 min to 1 h. The proportions of different cells were measured using a flow cytometer, and the cell proportions in the Petri dish after different treatments were calculated. As can be seen from FIG. 1, bystander effects were seen with ADC 1, ADC2 and ADC3, among which ADC1 showed stronger bystander effects than ADC2 and the control drug ADC3.

**Example 13: Pharmacodynamic Study of Drugs ADC1, ADC2 and ADC3 in Subcutaneous Xenograft Female BALB/c Nude Mouse Animal Model of Human Ovarian Cancer SK-OV-3 Cell Line**

Test drugs: ADC1 (5mg/kg)

ADC2 (5mg/kg)

ADC3 (5mg/kg)

Preparation method: all were diluted with PBS

Test animals: BALB/c nude, 6 per group (Jiangsu GemPharmatech Co., Ltd.)

Test method: The SK-OV-3 cells were cultured in McCoy's 5a medium containing 10% fetal bovine serum. SK-OV-3 cells in the exponential phase were collected and resuspended in PBS to an appropriate concentration for subcutaneous tumor grafting in mice. The test mice were inoculated subcutaneously at the right anterior scapula on the right dorsum with $1 \times 10^7$ SK-OV-3 cells, which were resuspended in PBS 1: 1 mixed with matrigel (0.1 mL/mouse). The growth of the tumors was observed periodically. When the mean volume of the tumors reached 129.98 $mm^3$, the mice were randomly grouped for administration according to the tumor size and the mouse weight. The day of grouping was defined as day 0 and the administration was started at day 0. A single administration by intravenous injection was adopted. The tumor volume and weight were measured twice a week, and the data were recorded.

Test results: The tumor volume changed as shown in Table 2 and FIG. 2. The test results show that ADC1 and ADC2 have significantly greater efficacy than ADC3 after a single administration. There was no apparent abnormality or weight loss in the groups of mice during the experiment.

Table 2

| Experimental group | Mean tumor volume (day 0)[a] | Mean tumor volume (day 13)[a] | TGI (%)[b] | P value (relative to control group) [c] |
|---|---|---|---|---|
| Vehicle, 0 mg/kg | 130.12±10.29 | 2224.22±358.85 | - | - |
| ADC3, 5 mg/kg | 129.89±8.09 | 903.31±227.77 | 63.07 | 3.14e-01[ns] |
| ADC2, 5 mg/kg | 129.99±9.92 | 160.77±93.32 | 98.53 | 2.05e-05*** |

(continued)

| Experimental group | Mean tumor volume (day 0)[a] | Mean tumor volume (day 13)[a] | TGI (%)[b] | P value (relative to control group) [c] |
|---|---|---|---|---|
| ADC1, 5 mg/kg | 129.89±7.59 | 8.53±5.96 | 105.80 | 7.92e-09*** |

Note: a. Data were expressed as "mean ± standard error";
b. TGI%=[1-(Ti-T0)/(Ci-C0)]×100, where T0 and C0 are the mean tumor volumes of the administration group and vehicle control group at the day of grouping (day 0), respectively, and Ti and Ci are the mean tumor volumes of the administration group and vehicle control group at day 28, respectively
c. *P<0.05, ***P*<0.01 and ****P*<0.001 compared to the tumor volume of the vehicle control group.

**Example 14: Clinical Study on ADC1**

Preclinical pharmacokinetic study:

The results of the pharmacokinetic study on ADC1 administered by single intravenous infusion in cynomolgus monkeys showed that no significant gender differences (P > 0.05) were observed in the pharmacokinetic parameters of ADC1 and total antibody in the experimental groups.

The low-dose, medium-dose, and high-dose groups were administered at a dose ratio of 1:3:10. The ratios of mean serum $C_{max}$ (peak concentration) to $AUC_{last}$ to $AUC_{INF_obs}$ for the ADC in the animals of the groups were 1:3.10:10.16, 1:4.42:16.04, and 1:4.41:16.01, respectively. The ratios of mean serum $C_{max}$ to $AUC_{last}$ to $AUC_{INF_obs}$ for the total antibody in the animals of the groups were 1:3.14:10.52, 1:4.46:16.97, and 1:4.43:16.86, respectively. The ratios of mean plasma $C_{max}$ to $AUC_{last}$ for the small molecule in the animals of the groups were 1:2.34:8.09 and 1:3.62:18.90, respectively. The results showed that after ADC1 was administered to cynomolgus monkeys at doses of 1 mg/kg, 3 mg/kg, and 10 mg/kg by intravenous infusion, the plasma concentrations of the ADC and the total antibody in the animals increased with the increase in dose, and the systemic exposure ($C_{max}$ and $AUC_{0-t}$) of the small molecule metabolite Exatecan in the cynomolgus monkeys increased with the increase in dose.

Fitting was performed by using a power function model to obtain relationships between the exposure level parameters ($C_{max}$, $AUC_{last}$, and $AUC_{INF_obs}$) of the ADC, the total antibody, and the small molecule drug and the dose for groups 1-3. According to the slope (parameter β) of the linear fitting and its 90% confidence interval, it was indicated that within the dose range of 1-10 mg/kg, the increase in $C_{max}$ of the ADC and the total antibody in the serum and the small molecule in the plasma was directly proportional to the increase in dose, and the increase in $AUC_{last}$ and $AUC_{inf}$ was higher than the increase in dose.

**Hemolysis test:** The *in vitro* hemolysis test of ADC1 was performed using erythrocytes from New Zealand white rabbits. The test results showed that the test sample ADC1 for injection at a concentration of 5 mg/mL did not cause hemolysis or agglutination of rabbit erythrocytes and was injectable.

**Immunogenicity study:** After a single intravenous injection of 1 mg/kg, 3 mg/kg, and 10 mg/kg of ADC1 in cynomolgus monkeys, the production of anti-drug antibody (ADA) was observed in the low-dose (1 mg/kg) and high-dose (10 mg/kg) groups, with individual positive rates of 16.7% (1/6), 0% (0/6), and 33.3% (2/6), respectively

**Example 15: Clinical Study on HER2-ADC**

This study was a multicenter, open-label phase I clinical study of safety, tolerability, pharmacokinetics (PK) characteristics, and preliminary clinical efficacy of ADC1 for injection in patients with HER2-positive solid tumors. The maximum tolerated dose (MTD) or maximum administered dose (MAD) was explored to provide recommended doses for subsequent clinical trials.

**Primary objective:** The safety and tolerability of ADC1 in patients with HER2-positive solid tumors were evaluated, and the maximum tolerated dose (MTD) was explored to provide recommended doses for subsequent clinical trials.

**Secondary objectives:** The pharmacokinetic (PK) characteristics, the immunogenicity, and the anti-tumor efficacy of ADC 1 after single and multiple doses in patients with HER2-positive solid tumors were evaluated.

The clinical study of ADC1 was divided into two stages. In the first stage, a "3 + 3" dose escalation rule was used to explore the safety and the tolerability of the study drug, with 7 dose groups set, namely A (1.2 mg/kg), B (2.4 mg/kg), C (3.6 mg/kg), D (4.8 mg/kg), E (6.0 mg/kg), F (7.2 mg/kg), and G (8.4 mg/kg). In the second stage, based on the preliminary safety and efficacy results of the previous stage, proper dose, administration regimen, and tumor type were selected for extension study to further explore the safety and clinical efficacy of ADC1 and provide a basis for subsequent clinical studies.

The dose escalation pattern of the "3 + 3" rule is as follows:

| Number of DLT (dose-limiting toxicity) cases/number of subjects in the current dose group | Dose escalation/de-escalation decision |
|---|---|
| 0/3 | 3 new subjects receive the trial in the next high-dose group |
| 1/3 | 3 new subjects are enrolled in the current dose group |
| 1/3+0/3 (i.e.1/6) | 3 new subjects receive the trial in the next high-dose group |
| ≥2/3 or ≥2/6 | It is indicated that this dose has exceeded the MTD (maximum tolerated dose), and dose escalation should be stopped. If there are already 6 subjects in the previous dose group, the previous dose is used as the MTD. If there are only 3 subjects in the previous dose group, 3 more subjects are enrolled in the previous dose group. After 3 more subjects are enrolled, if there are a total of ≥ 2 DLTs in the previous dose group, the MTD continued to be deescalated. |

During the tolerability exploration according to the "3+3" dose escalation rule, all subjects in the previous dose group must complete a DLT observation before the enrollment for the next dose group (cohort). In each dose group, a time interval of at least 24 hours between the doses of the first and second subjects enrolled should be guaranteed, and if the tolerability is good, no 24-hour interval is required subsequently.

**Administration regimen:** Based on the regimen, the dose groups were subjected to administration in a mg/kg administration mode by intravenous infusion. The recommended duration of infusion for the first dose was 90 (± 5) minutes. If no infusion reaction occurred during the first dose, subsequent doses could be administered within 30-120 minutes at the earliest, which was determined by the investigator in the context of the clinical practice. With one dose every 3 weeks (Q3W) counted as one cycle, the administration was performed on the first day of each cycle, until the occurrence of progressive disease/death, or intolerable toxicity, or voluntary withdrawal such as withdrawal of informed consent and other reasons, or loss to follow-up, or the decision to discontinue treatment based on the investigator's evaluation, or receipt of new anti-tumor treatment, or the end of the study, whichever came first.

The body weight of the subjects measured at screening was used to calculate the dose administered, and if the weight varied by ± 10% from the baseline body weight, the dose was recalculated.

**Evaluation of tolerability and safety:**

Tolerability evaluation indexes: dose-limiting toxicity (DLT) events and their incidence.

Safety evaluation indexes: Vital signs and physical examination, laboratory examinations (complete blood count, blood biochemistry, troponin, coagulation function, urinalysis, and pregnancy test), ECOG score, electrocardiogram, cardiac color ultrasound/radionuclide angiography, and adverse events (including SAE, TEAE, ADR, etc.). The nature, frequency, severity, occurrence time, etc., of the adverse events were evaluated; the changes in clinical laboratory examination results before, during, and after the treatment were evaluated.

**Pharmacokinetic evaluation:**

Blood samples were collected at specified time points during the treatment, and pharmacokinetic characteristics were studied. The drug serum concentrations ofADC 1, the anti-HER2 total antibody, and Exatecan were observed at the time points specified in the protocol.

The following PK (pharmacokinetic) parameters were calculated from the actual administration doses and actual sampling time by the non-compartmental model. For patients with accidental and/or severe AEs, additional PK (pharmacokinetic) blood samples should be collected. PK-related parameters include: Cmax, Tmax, T1/2, CL, Vd, Ke, MRT, AUC(0-τ), and AUC(0-∞) at single administration; and Cmax,ss, Cavg,ss, Cmin,ss, AUC(0-τ),ss, AUC(0-oo),ss, Tmax,ss, T1/2,ss, CL, Vss, Ke, MRT, accumulation index (Rac), and fluctuation index (DF) at multiple administration.

**Immunogenicity evaluation indexes involve:** the sample positive rate and individual positive rate for the anti-drug antibody (ADA), the titer of the ADA-positive sample, and whether the ADA-positive sample will continue to be tested for a neutralizing antibody (NAb)

Samples for immunogenicity evaluation will be collected prior to each administration of the first 6 doses and subsequently at a frequency of every 12 weeks. In addition, samples for immunogenicity evaluation will be collected once

more in a safety follow-up 28 days after the last dose. The effect of anti-drug and neutralizing antibodies on PK (pharmacokinetics), and efficacy/safety (as applicable) will be evaluated. If subjects experience adverse events such as infusion reaction, additional samples should be collected at the onset of (toxic) events, when the (toxic) reaction subsides, and about 30 ± 7 days after the reaction for immunogenicity analysis. Blood samples of 5 mL were collected at each time point and tested for anti-drug antibodies and/or neutralizing antibodies in the serum.

The immunogenicity of ADC1 for injection was studied, and the immunogenicity evaluation data were summarized by dose groups, including data on subjects who had at least one positive ADA/NAb detection result at any time point, the frequency of positive ADA/NAb detection, and the frequency of increase in ADA/NAb levels from baseline.

**Clinical efficacy evaluation:**

Evaluation method: Anti-tumor efficacy evaluation is divided into tumor imaging evaluation (CT/MRI), physical examination, and survival evaluation. Throughout the trial, the anti-tumor efficacy was evaluated by using the RECIST 1.1 evaluation standard.

The anti-tumor efficacy indexes include: objective response rate (ORR), duration of response (DOR), disease control rate (DCR), progression-free survival (PFS), and overall survival (OS) based on the RECIST evaluation standard with CT/MRI as the primary means of imaging evaluation.

**DLT definition:** Adverse events (AEs) will be evaluated based on CTCAE v5.0. Dose-limiting toxicity (DLT) is defined as AE that occurs during the DLT observation period and is considered to be at least possibly related to the study drug. After the first dose, if the administration of the second cycle is delayed due to an AE, the DLT observation period needs to be prolonged accordingly. The definition of DLT in this study is specifically as follows:

· Grade 5 toxicity.

· Definition of hepatotoxicity DLT:

Grade 4 elevation of AST (aspartate transaminase) or ALT (alanine transaminase);

subjects with hepatocellular carcinoma or liver metastasis, e.g., AST or ALT ≤ 3-fold ULN (upper limit of normal) at baseline, AST or ALT elevation > 5-fold ULN in a DLT evaluation period and lasting > 7 days;

subjects with hepatocellular carcinoma or liver metastasis, e.g., AST or ALT > 3-fold ULN at baseline, AST or ALT elevation > 8-fold ULN in a DLT evaluation period and lasting > 7 days;

subjects without liver metastasis, AST or ALT elevation > 5-fold ULN and lasting > 7 days;

AST or ALT > 5-fold upper limit of normal (ULN), with ≥ Grade 2 blood bilirubin elevation.

· Definition of hematological toxicity DLT:

Grade 4 neutropenia lasting > 7 days;

≥ Grade 3 neutropenia with fever (single body temperature > 38.3 °C or sustained body temperature ≥ 38 °C for more than 1 hour);

Grade 4 anemia;

Grade 4 thrombocytopenia;

≥ Grade 3 thrombocytopenia lasting > 7 days;

≥ Grade 3 thrombocytopenia with bleeding.

· Definition of non-hepatotoxicity and non-hematological toxicity DLT:
Other ≥ Grade 3 non-hepatotoxicity and non-hematological toxicity.

In the determination of DLT (dose-limiting toxicity), some apparent and indisputable AEs caused by progressive disease or causes other than DLT should not be included in DLT. During the DLT observation period, subjects who

discontinue the study within the DLT evaluation window due to non-DLT causes (such as adverse events clearly related to underlying diseases, progressive disease, concomitant medications, or concomitant diseases, and dropout) will be considered as non-evaluable for DLT. Subjects who received < 80% of the planned study dose during the DLT evaluation period will be considered non-evaluable for DLT. A subject is considered non-evaluable for DLT if the symptomatic and supportive treatment (excluding the symptomatic and supportive treatment described in the definition of DLT) that the subject receives during the DLT evaluation period interferes with the judgment of DLT and makes DLT difficult to interpret.

A subject who is non-evaluable for DLT will be replaced by another patient at the same dose level. If a subject voluntarily withdraws from the study, the last visit must be completed as soon as possible and as required. The replaced subject is included in the SS (safety data set) for statistical purposes, but is not counted in the total number of the group when calculating the incidence of DLT events. After agreement between the sponsor and the investigator, repeated screening of some subjects is allowed, and the screening numbers needs to be reassigned.

② The following AEs were not considered DLTs:

Grade 3 nausea, vomiting, diarrhea, anorexia, or fatigue that is controlled by standard supportive treatment and relieved to ≤ Grade 2 within 3 days;

Grade 3 or higher rash that can be relieved within ≤ 7 days after the conventional treatment;

abnormalities in laboratory examinations without clinically relevant symptoms or signs, including elevated ALP, elevated uric acid, elevated blood amylase, elevated lipase, or decreased lymphocyte to Grade 3 or 4, with Grade 1 hyponatremia at baseline escalating to Grade 3 but lasting < 72 hours;

Grade 3 infusion reaction that can be relieved within 6 hours after treatment.

**Example 16: Combination of HER2-ADC with HER2-Targeted Antibody**

BAT0303F is an anti-HER2 antibody with an enhanced ADCC effect for use in targeting cells or tissues of a patient to enhance the anti-tumor efficacy of the drug. BAT0303F has a fucosylation level of 0%-5%.

Preparation and purification of the antibody BAT0303F: the expression vector for the antibody BAT0303F (with amino acid sequences shown in Table 3) was constructed using conventional molecular biology methods. BAT0303F was expressed by CHO cells in which the α-(1,6)-fucosyltransferase gene FUT8 was knocked out. The method for knocking out the α-(1,6)-fucosyltransferase gene FUT8 of CHO was referred to Patent WO2019029713A. Cell culture supernatant was subjected to Protein A chromatography, anion chromatography, cation chromatography, and other steps to obtain the purified antibody.

Table 3. Amino acid sequence of antibody BAT0303F

| Name | No. | Amino acid sequence |
|---|---|---|
| HCDR1 | SEQ ID NO:1 | GFTFTDYT |
| HCDR2 | SEQ ID NO:2 | VNPNSGGSIYNQRFK |
| HCDR3 | SEQ ID NO:3 | LGPSFYFDY |
| LCDR1 | SEQ ID NO.4 | KASQDVSIGVA |
| LCDR2 | SEQ ID NO:5 | ASYRYTG |
| LCDR3 | SEQ ID NO:6 | QQYYIYPYTF |
| VH | SEQ ID NO: 7 | EVQLVESGGGLVQPGGSLRLSCAASGFTFTDYTMDWVRQ APGKGLEWVADVNPNSGGSIYNQRFKGRFTLSVDRSKNT LYLQMNSLRAEDTAVYYCARNLGPSFYFDYWGQGTLVTV SS |
| VL | SEQ ID NO:8 | DIQMTQSPSSLSASVGDRVTITCKASQDVSIGVAWYQQKP GKAPKLLIYSASYRYTGVPSRFSGSGSGTDFTLTISSLQPED FATYYCQQYYIYPYTFGQGTKVEIKR |

(continued)

| Name | No. | Amino acid sequence |
|---|---|---|
| Heavy chain full length | SEQ ID NO:9 | EVQLVESGGGLVQPGGSLRLSCAASGFTFTDYTMDWVRQ APGKGLEWVADVNPNSGGSIYNQRFKGRFTLSVDRSKNT LYLQMNSLRAEDTAVYYCARNLGPSFYFDYWGQGTLVTV SSASTKGPSVFPLAPSSKSTSGGTAALGCLVKDYFPEPVTV SWNSGALTSGVHTFPAVLQSSGLYSLSSVVTVPSSSLGTQT YICNVNHKPSNTKVDKKVEPKSCDKTHTCPPCPAPELLGG PSVFLFPPKPKDTLMISRTPEVTCVVVDVSHEDPEVKFNW YVDGVEVHNAKTKPREEQYNSTYRVVSVLTVLHQDWLN GKEYKCKVSNKALPAPIEKTISKAKGQPREPQVYTLPPSRE EMTKNQVSLTCLVKGFYPSDIAVEWESNGQPENNYKTTPP VLDSDGSFFLYSKLTVDKSRWQQGNVFSCSVMHEALHNH YTQKSLSLSPGK |
| Light chain full length | SEQ ID NO:10 | DIQMTQSPSSLSASVGDRVTITCKASQDVSIGVAWYQQKP GKAPKLLIYSASYRYTGVPSRFSGSGSGTDFTLTISSLQPED FATYYCQQYYIYPYTFGQGTKVEIKRTVAAPSVFIFPPSDEQ LKSGTASVVCLLNNFYPREAKVQWKVDNALQSGNSQESV TEQDSKDSTYSLSSTLTLSKADYEKHKVYACEVTHQGLSS PVTKSFNRGEC |

Table 4. Percentage content of part of glycoforms of antibody BAT0303F

| Antibody sample | G0F-GN | G0-GN | G0 | G0F | Man5 | G1 | G1' | G1F | G1F' | G2 | G2F |
|---|---|---|---|---|---|---|---|---|---|---|---|
| BAT0303F | ND | 5.35 | 72.56 | ND | 2.27 | 8.94 | 8.94 | ND | ND | 0.76 | ND |
| Note: ND: not detectable. | | | | | | | | | | | |

**Claims**

**1.** A method for treating a HER2-positive solid tumor, comprising administering to a patient with a HER2-positive solid tumor an effective amount of an antibody-drug conjugate, wherein, the antibody-drug conjugate has a structure shown as formula I, or a stereoisomer, or a pharmaceutically acceptable salt or solvate thereof:

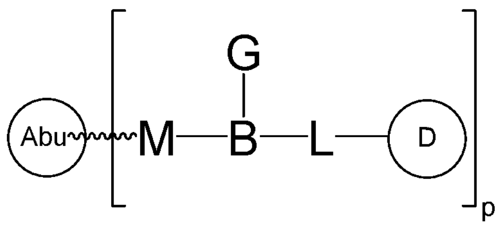

formula I

wherein,

Abu is an antibody or an antigen-binding unit thereof that binds to HER2;
D is drug,
M is

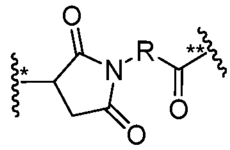

,

wherein * links to Abu, ** links to B, R is selected from: $-(CH_2)_r-$, - $(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8carbocyclyl-)-, -(C3-C8 carbocyclyl)-$(CH_2)_r-$, C3-C8 carbocyclyl, $-(CH_2)_r$-(C3-C8 heterocyclyl)-, - (C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, - $(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$ and - $(CH_2CH_2O)_rC(O)NR^m(CH_2)_r-$; wherein each $R^m$ is independently H, C1-C6 alkyl, C3-C8 carbocyclyl, phenyl or benzyl; and each r is independently 1, 2, 3, 4, 5, 6, 7, 8, 9 and 10; or R is $-(CH_2)_r-$, or r is 1 or 5;
B is

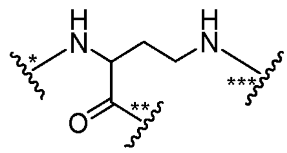

,

or is

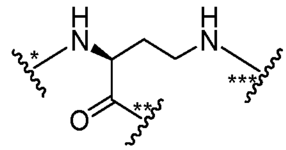

;

wherein * links to M, ** links to L, *** links to G;
L is $-(AA)_i-(FF)_f-$, wherein AA is amino acid or polypeptide, i is 1, 2, 3, 4, 5, 6, 7, 8, 9, 10, 11, 12, 13, 14, 15, 16, 17, 18, 19 or 20; or AA is selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly; or AA is Val-Cit, i is 1;
FF is

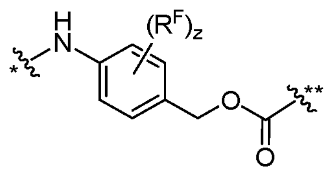

,

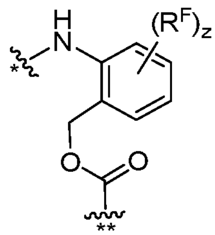

or

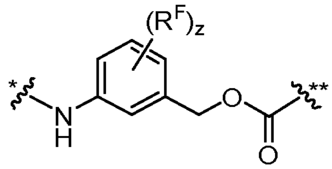

,

wherein $R^F$ is C1-C6 alkyl, C1-C6 alkoxy, $-NO_2$ or halogen; z is 0, 1, 2, 3 or 4, wherein * links to AA, ** links to D; or FF is

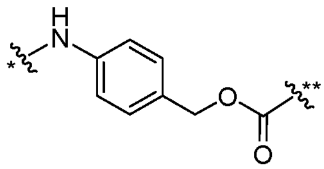,

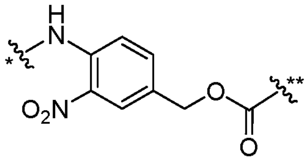, 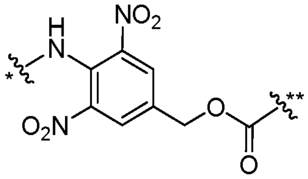, 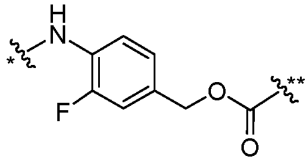,

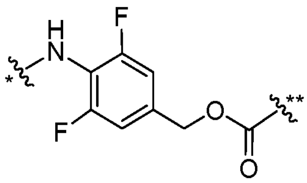, 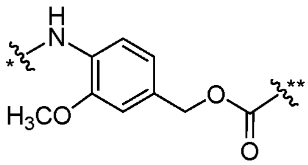, 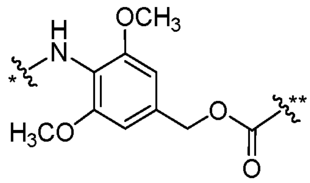, 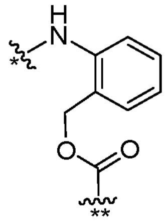,

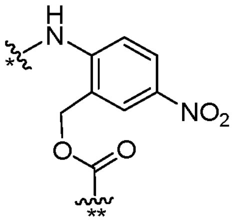, 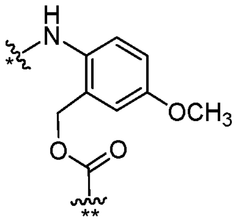, 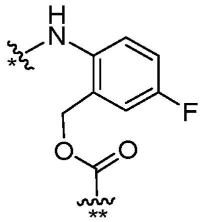, 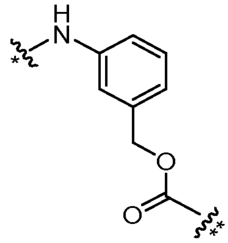,

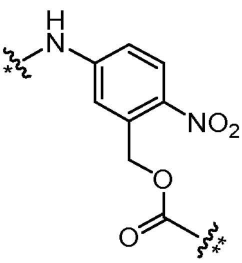, 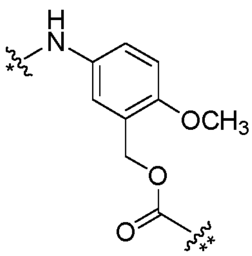, or 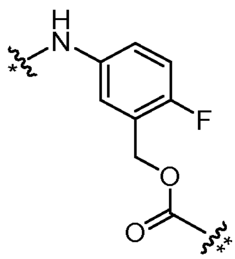;

f is 1, 2, 3, 4, 5, 6, 7, 8, 9 or 10,
wherein * links to AA, ** links to D; or FF is

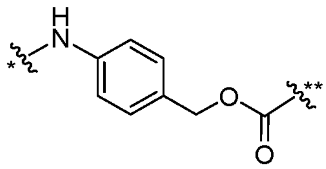,

f is 1; wherein * links to AA, ** links to D; or L is

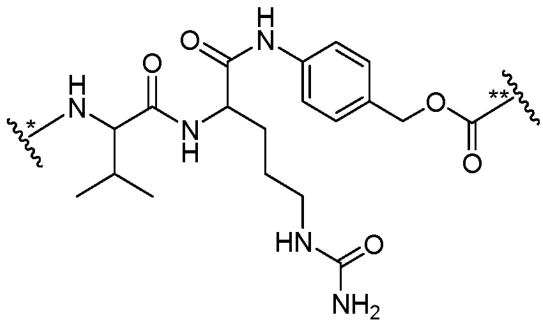

or

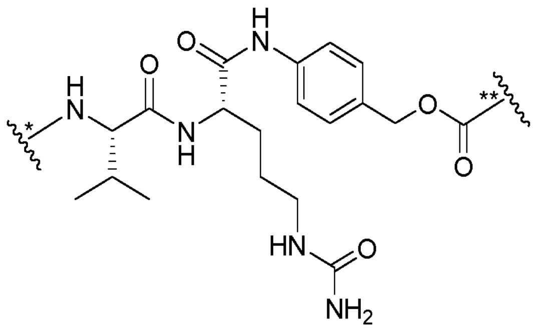

,

wherein * links to B, ** links to D;
G is

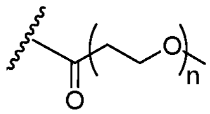

,

wherein n is 1-24; or n is 4-12; or n is 4-8; or n is 4 or 8;
p is 1-10; or p is 2-8; or p is 4-8; or p is 6-8; or p is 7-8.

**2.** A method for treating a HER2-positive solid tumor, comprising administering to a patient with a HER2-positive solid tumor an effective amount of an antibody-drug conjugate, wherein, the antibody-drug conjugate has a structure shown as formula I-1, I-2, I-3, I-4, I-5, I-6, I-7, I-8, I-9, I-10, or I-11, or a stereoisomer, or a pharmaceutically acceptable salt or solvate thereof, wherein

the formula 1-1 is:

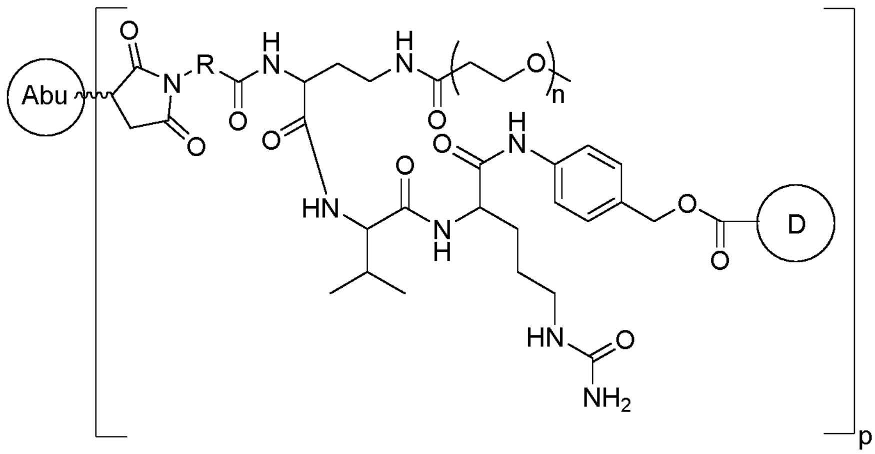

formula I-1

the formula I-2 is:

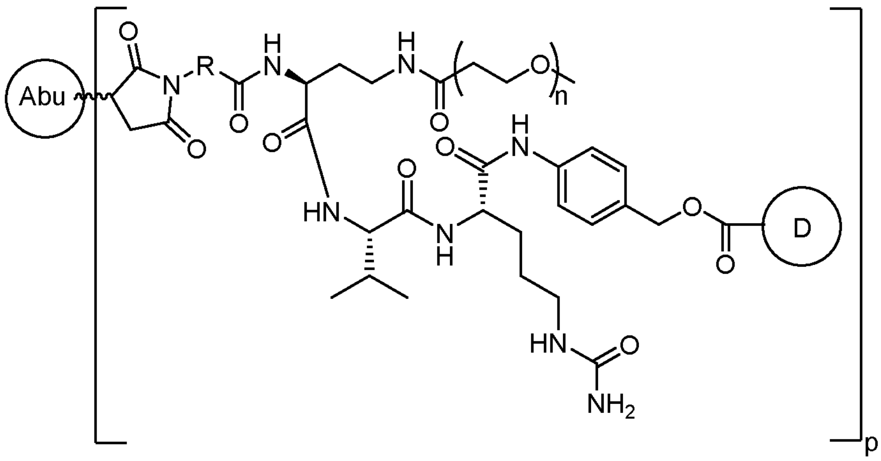

formula I-2

the formula I-3 is:

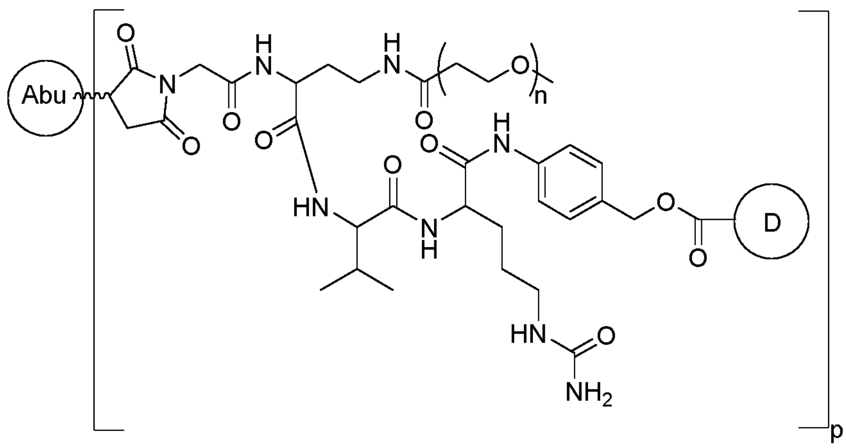

formula I-3

the formula I-4 is:

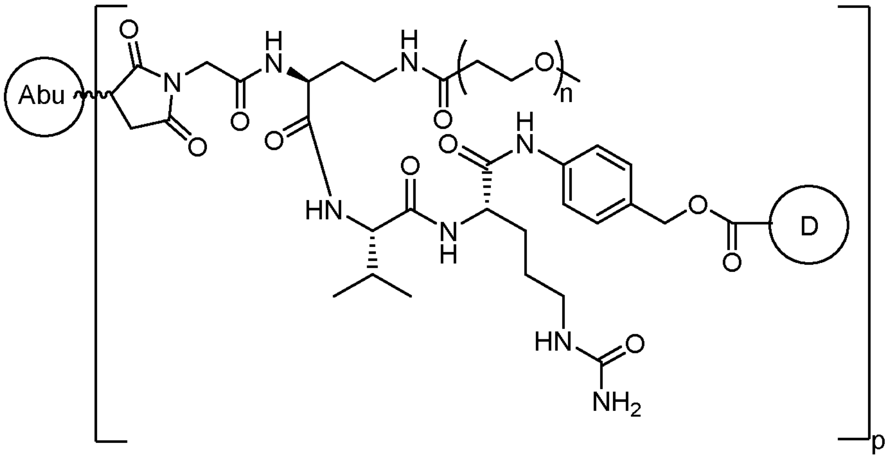

formula I-4

the formula I-5 is:

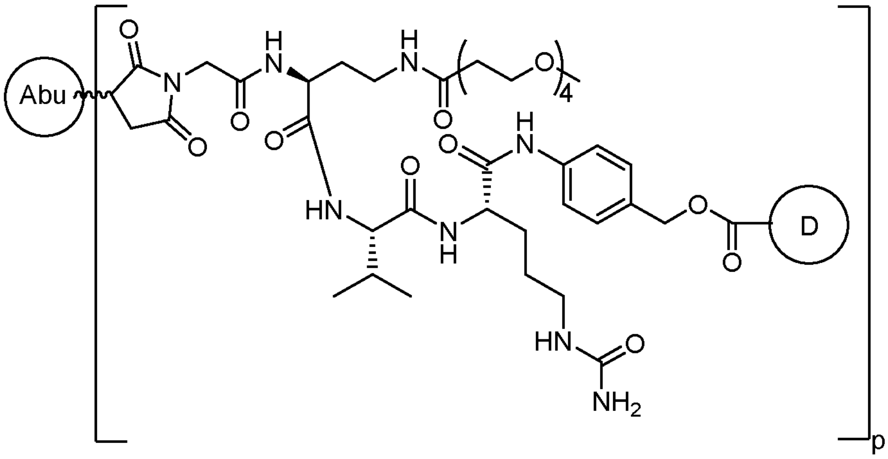

formula I-5

the formula I-6 is:

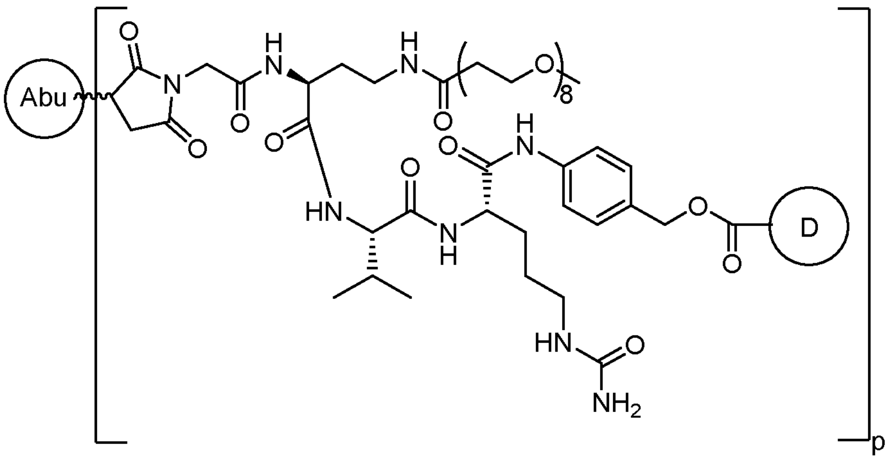

formula I-6

the formula I-7 is:

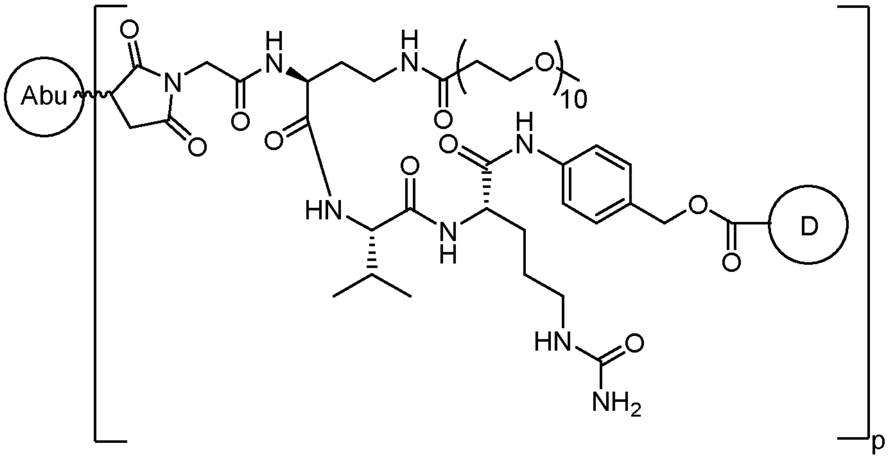

formula I-7

the formula I-8 is:

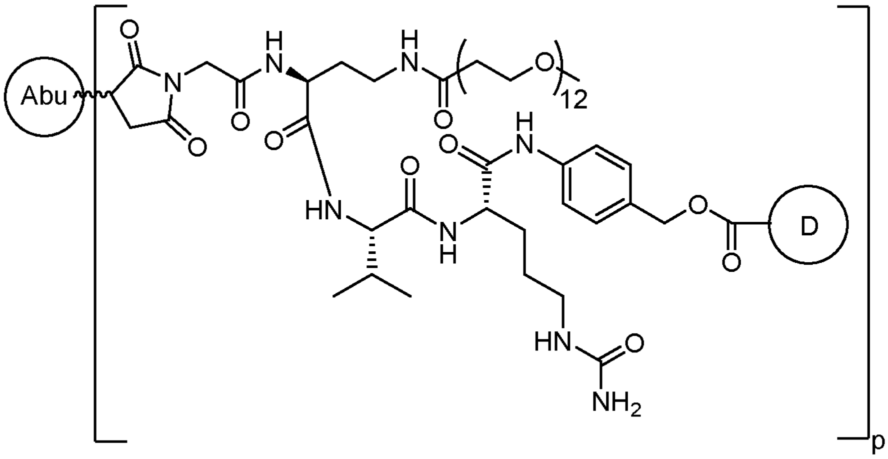

formula I-8

the formula I-9 is:

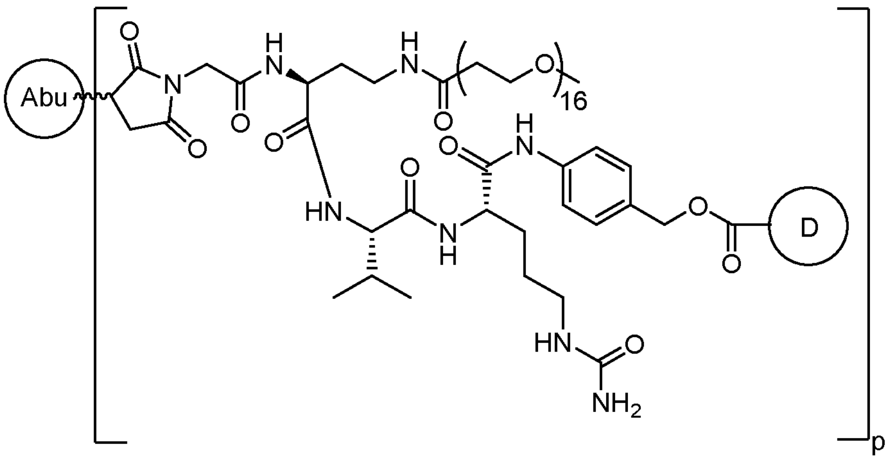

formula I-9

the formula I-10 is:

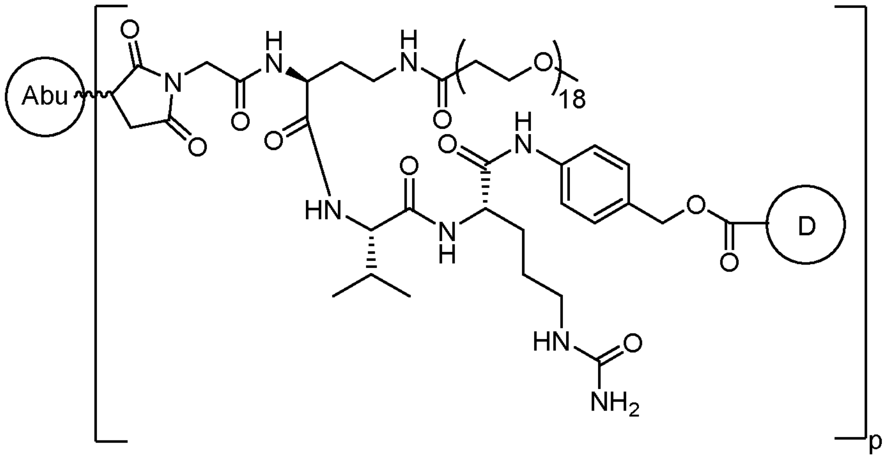

or

formula I-10

the formula I-11 is:

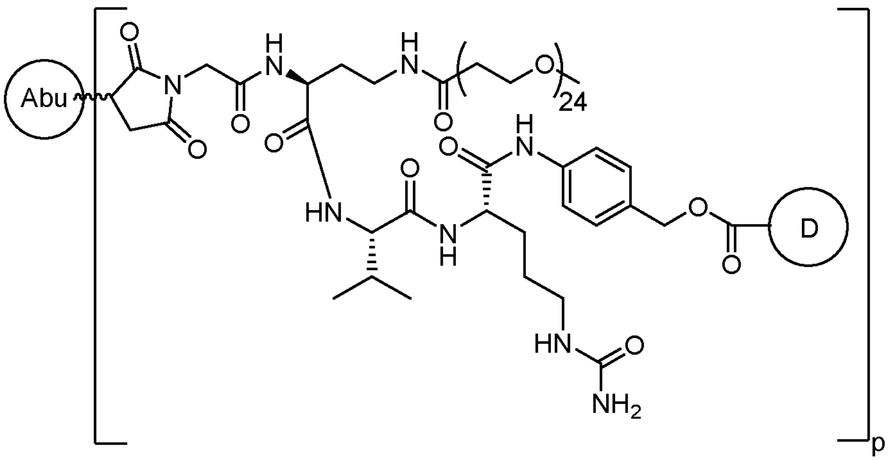

formula I-11.

wherein

Abu is an antibody or an antigen-binding unit thereof that binds to HER2;

R is selected from: $-(CH_2)_r-$, $-(CHR^m)_r-$, C3-C8 carbocyclyl, $-O-(CH_2)_r-$, arylene, $-(CH_2)_r$-arylene-, - arylene-$(CH_2)$r-, $-(CH_2)_r$-(C3-C8 carbocyclyl)-, -(C3-C8 carbocyclyl)-$(CH_2)_r-$, C3-C8 heterocyclyl, - $(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, - $(CH_2CH_2O)_{r-}C(O)NR^m(CH_2CH_2O)_r-$, $-(CH_2CH_2O)_rC(O)NR^m(CH_2CH_2O)_r-CH_2-$ and-$(CH_2CH_2O)_rC(O)NR^m(CH_2)_r-$; wherein each $R^m$ is independently H, C1-C6 alkyl, C3-C8 carbocyclyl, phenyl or benzyl; and each r is independently 1, 2, 3, 4, 5, 6, 7, 8, 9 and 10; or R is $-(CH_2)_r-$, or r is 1 or 5;

D is drug;

n is an integer from 1-24; or n is 4-12; or n is 4-8; n is 4 or 8;

p is 1-10; or p is 2-8; or p is 4-8; or p is 6-8; or p is 7-8.

**3.** The method according to claim 1 or 2, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases; or the drug is an anti-cancer drug; or the drug is a tubulin inhibitor, a DNA damaging agent, or a DNA topoisomerase inhibitor; or the tubulin inhibitor is selected from dolastatin, auristatins and maytansinoids; or the drug is an auristatin, selected from MMAE, MMAF, or AF; or the drug is a DNA damaging agent, selected from calicheamicin, duocarmycin, the anthramycin derivative PBD; or the drug is DNA topoisomerase inhibitor or a salt

thereof, selected from irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, or N-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine, or exatecan; or a pharmaceutically acceptable salt or solvate thereof.

**4.** The method according to claim 1-3, the characteristic is that, the drug is

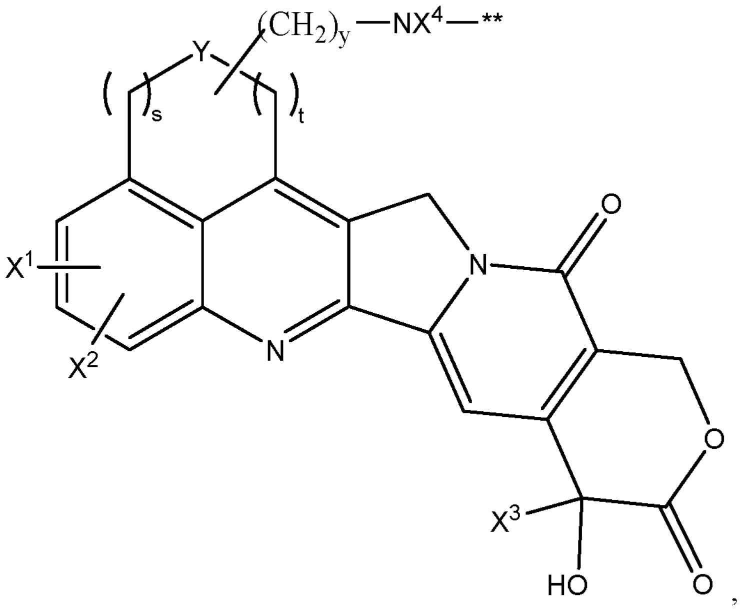

wherein
$X^1$ and $X^2$ are each independently:

H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro,

cyano or protecting group,
amino-substituted heterocyclyl, which is optionally substituted at a nitrogen atom of the heterocyclyl moiety or at the amino moiety with a protecting group or one or more C1-C6 alkyl groups, heterocyclylamino, which is optionally substituted at a nitrogen atom of the heterocyclic moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
$X^3$ is C1-C6 alky;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10; or ,$X^4$ is H or C1-C6 alkyl;
* * is point of connection;
y is 0, 1 or 2;
Y is O, S or $CR^1R^2$, wherein $R^1$ and $R^2$ are each independently H or C1-C6 alkyl; s and t are each independently 0, 1 or 2, but not both 0;
or the drug is

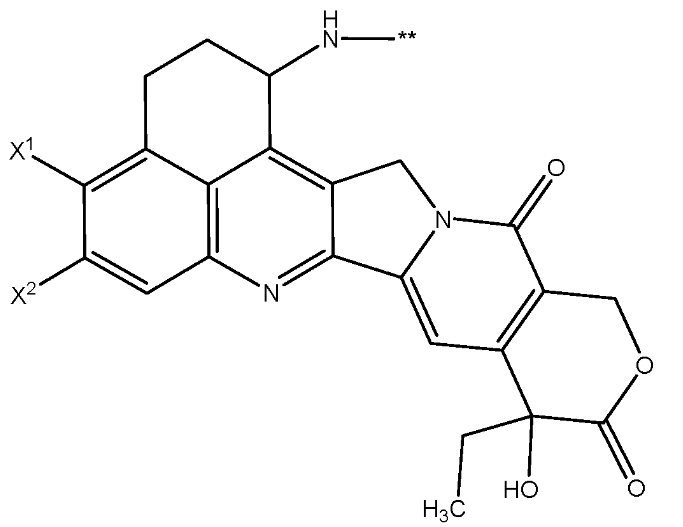

,

wherein $X^1$ and $X^2$ are each independently C1-C6 alkyl, halogen, or -OH; or C1-C6 alkyl is $-CH_3$; or halogen is F; ** is point of connection;
or the drug is

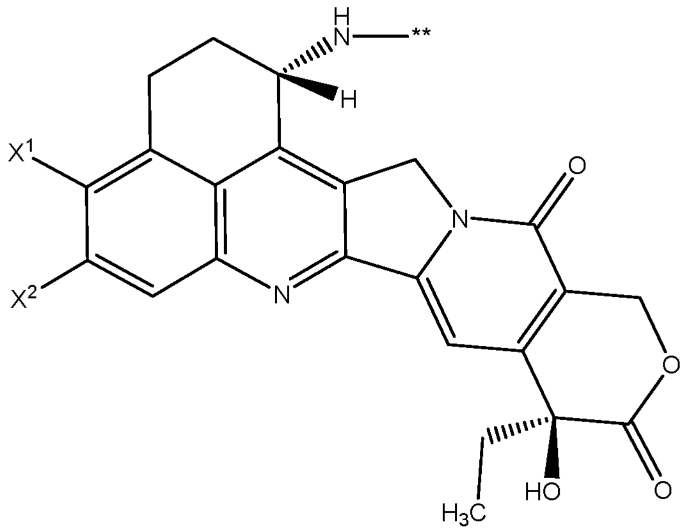

,

wherein $X^1$ and $X^2$ are each independently C1-C6 alkyl, halogen, or -OH; or C1-C6 alkyl is $-CH_3$; or halogen is F; ** is point of connection.

**5.** A method for treating a HER2-positive solid tumor, comprising administering to a patient with a HER2-positive solid tumor an effective amount of an antibody-drug conjugate, wherein, the antibody-drug conjugate has a structure shown as formula I-12, I-13, I-14, I-15, I-16, I-17, I-18, I-19, I-20, I-21, I-22, I-23, I-24, or I-25, or a stereoisomer, or a pharmaceutically acceptable salt or solvate thereof, wherein the formula 1-12, I-13, I-14, I-15, I-16, I-17, I-18, I-19, I-20, I-21, I-22, I-23, I-24, or I-25 is:

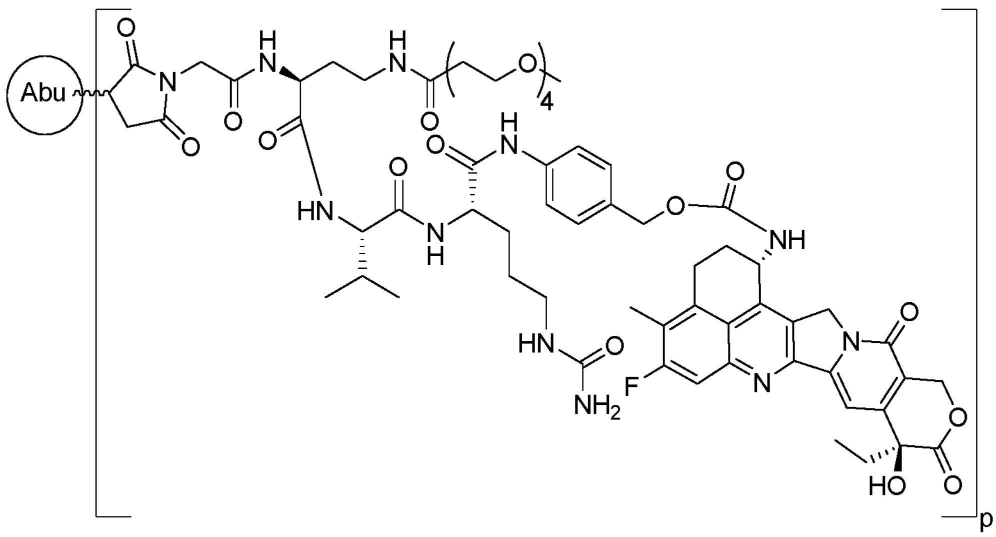

formula I-12

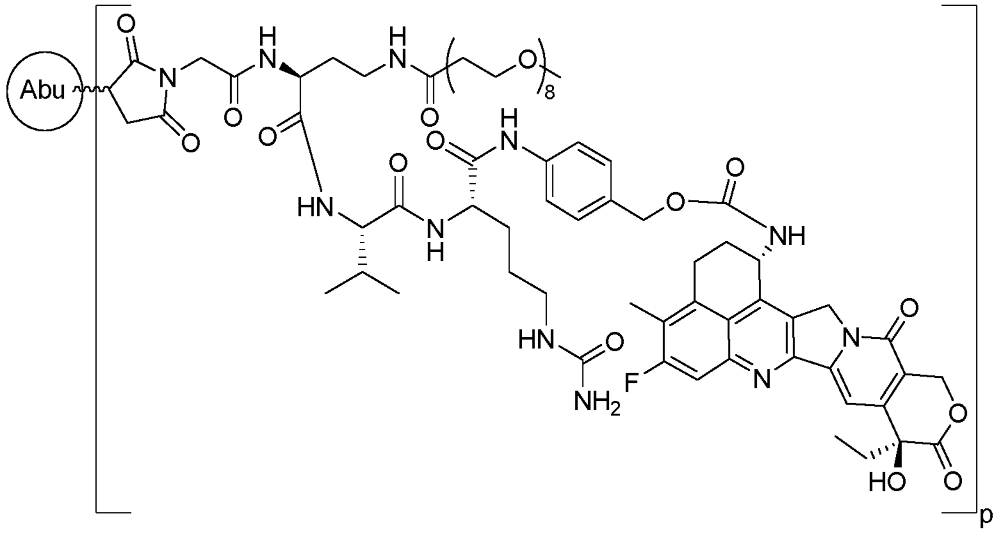

formula I-13

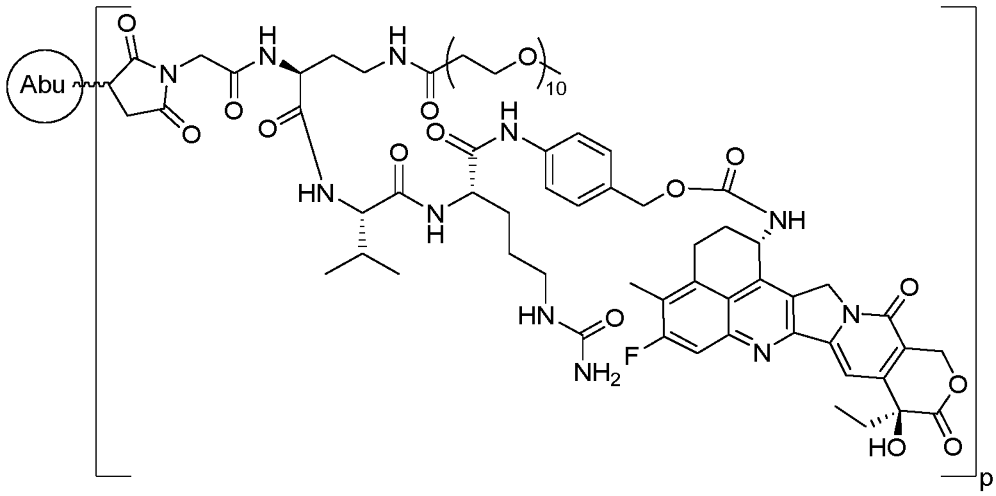

formula I-14

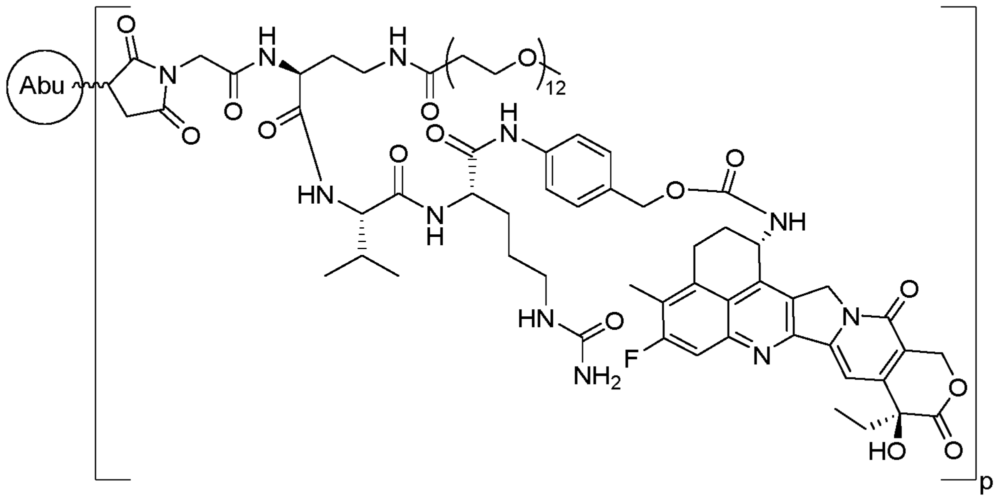

formula I-15

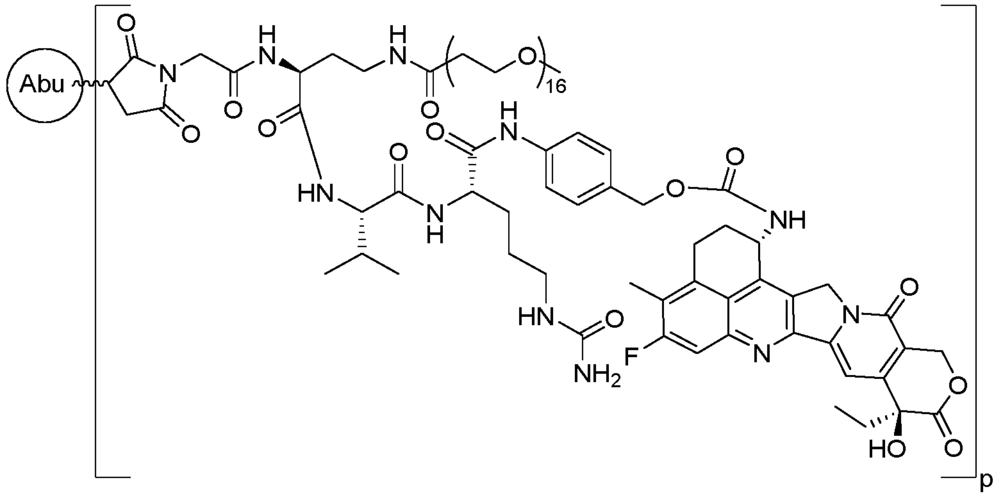

formula I-16

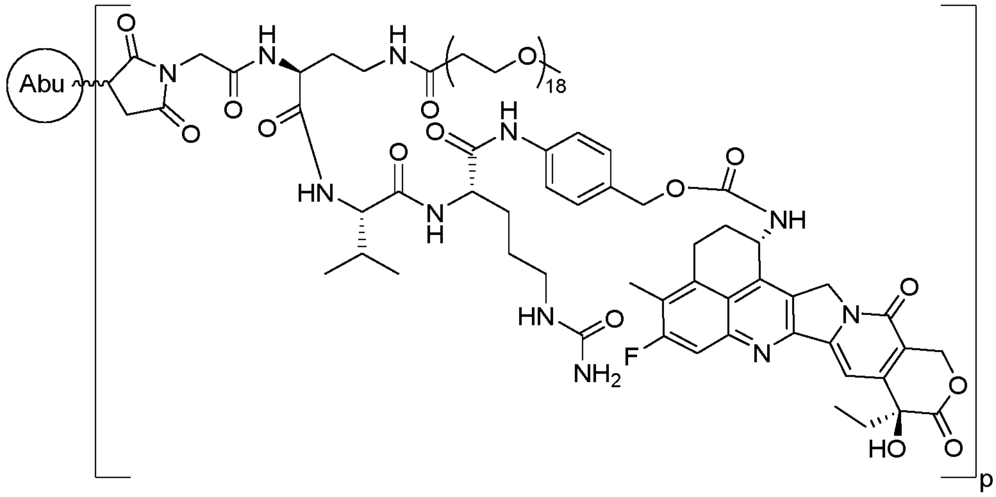

formula I-17

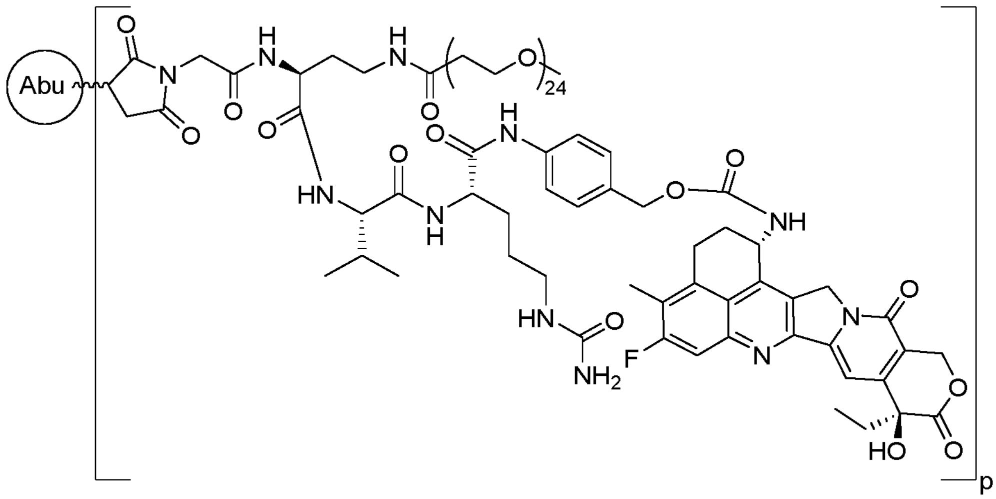

formula I-18

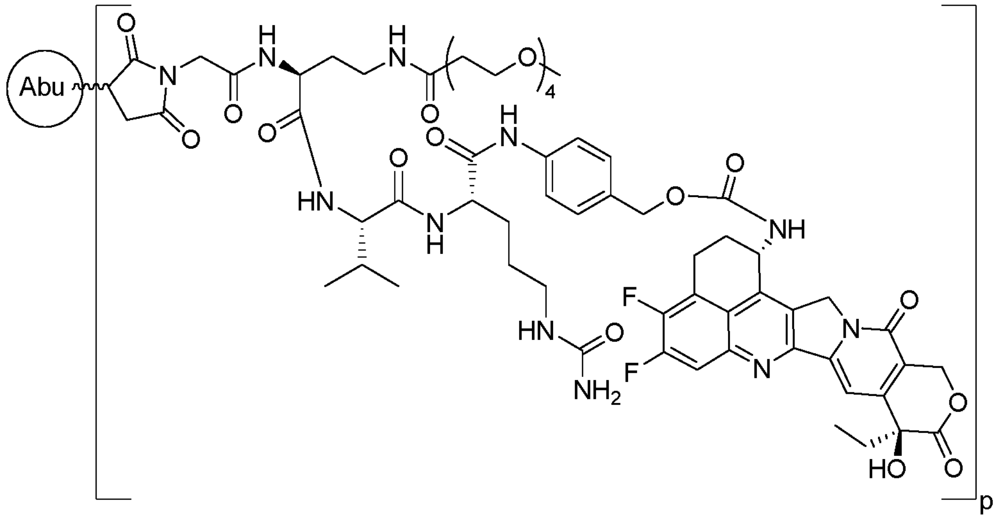

formula I-19

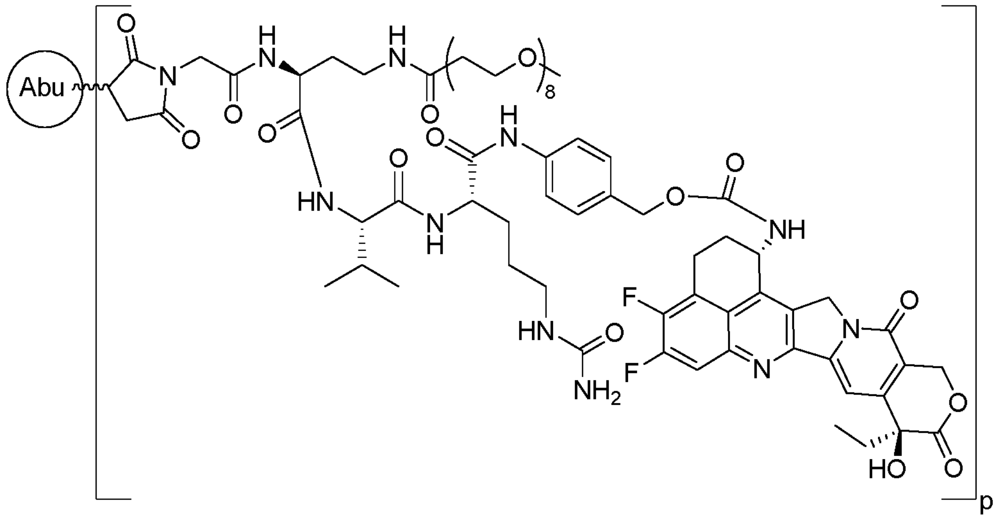

formula I-20

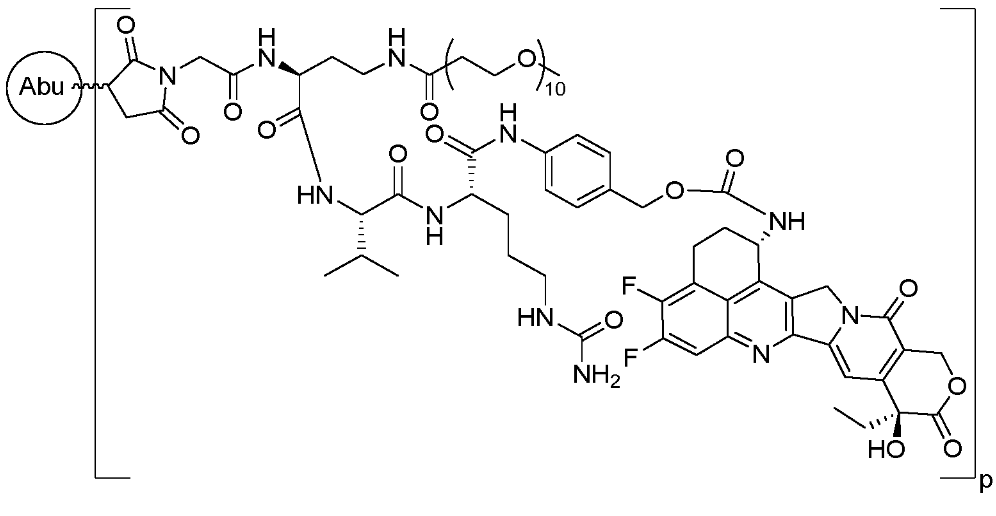

formula I-21

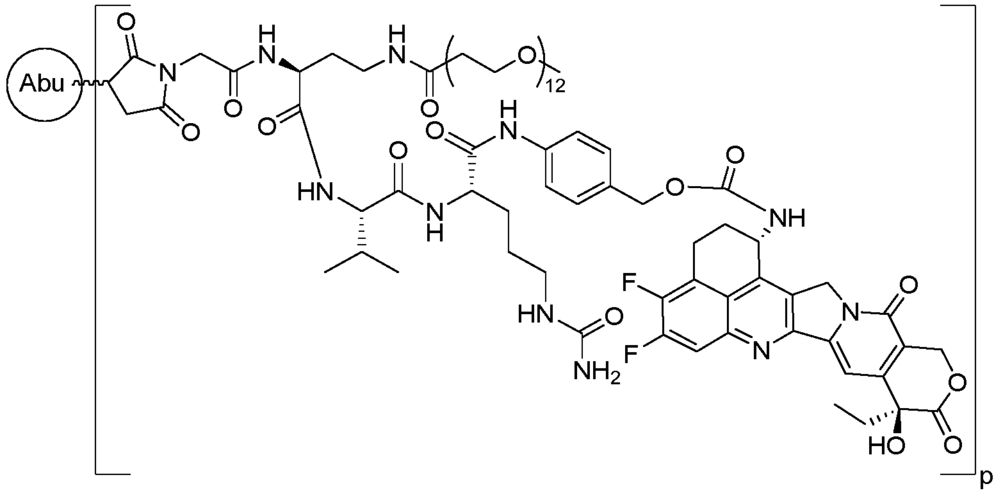

formula I-22

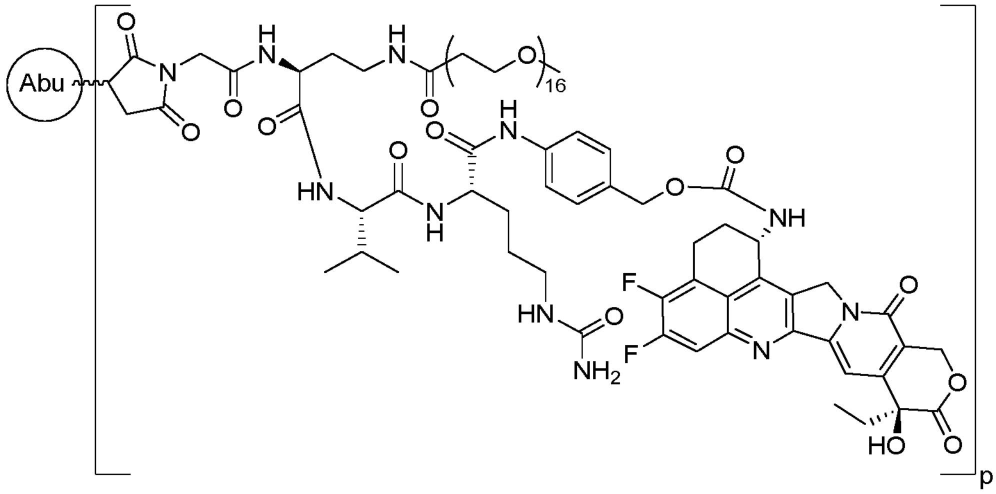

formula I-23

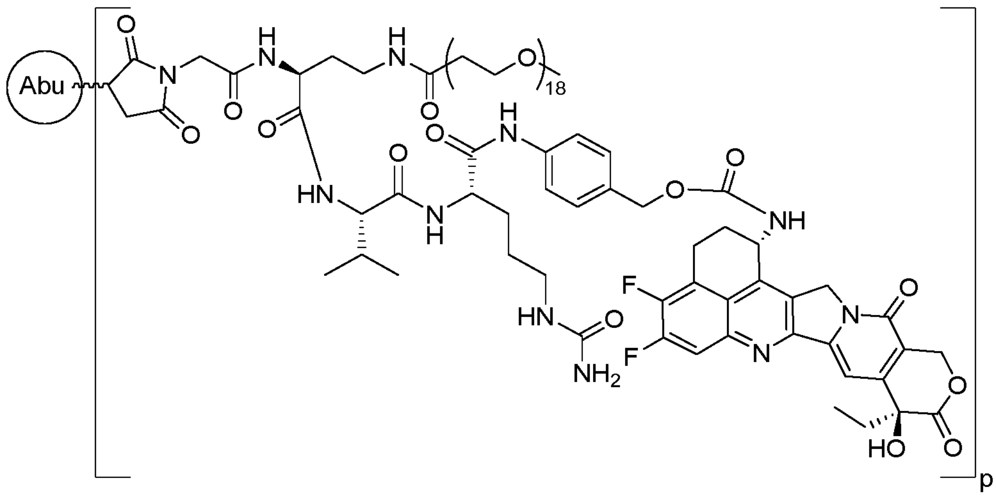

formula I-24

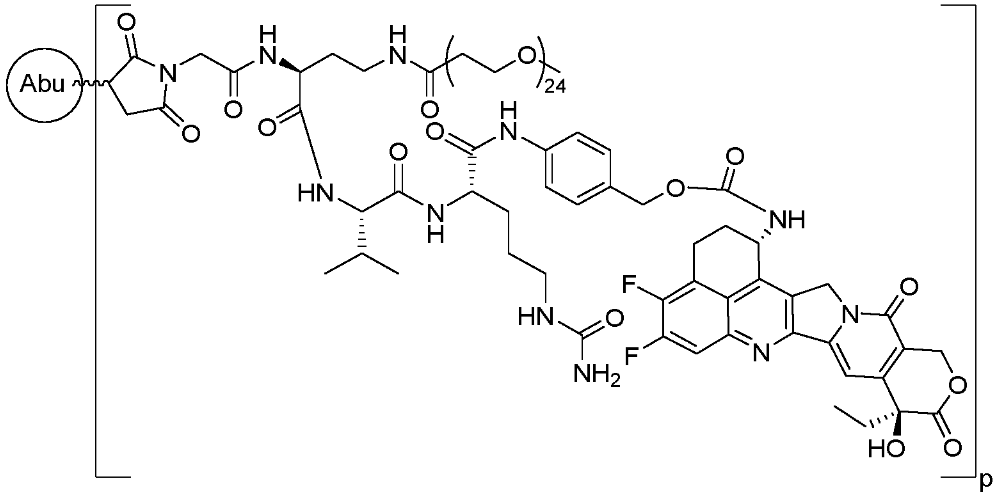

formula I-25

wherein

Abu is an antibody or an antigen-binding unit thereof that binds to HER2;
p is 1-10; or p is 2-8; or p is 4-8; or p is 6-8; or p is 7-8.

**6.** The method according to any one of claims 1-5, wherein Abu is trastuzumab.

**7.** The method according to any one of claims 1-6, wherein the antibody-drug conjugate is administered once every 1, 2, 3, 4, 5, 6, or 7 weeks at a dose of 1-30 mg/kg each time.

**8.** The method according to any one of claims 1-7, wherein the antibody-drug conjugate is administered to the patient in combination with a second drug.

**9.** The method according to claim 8, wherein the second drug is an antibody; for example, the second drug is a HER2-targeted antibody, a heavy chain variable region of the HER2-targeted antibody comprises an HCDR1 set forth in SEQ ID NO: 1, an HCDR2 set forth in SEQ ID NO: 2, and an HCDR3 set forth in SEQ ID NO: 3, and/or a light chain variable region of the HER2-targeted antibody comprises an LCDR1 set forth in SEQ ID NO: 4, an LCDR2 set forth in SEQ ID NO: 5, and an LCDR3 set forth in SEQ ID NO: 6;

or a heavy chain variable region of the HER2-targeted antibody comprises a sequence set forth in SEQ ID NO: 7, a sequence having at least 80% identity to the sequence set forth in SEQ ID NO: 7, or a sequence having one or more conservative amino acid substitutions as compared to the sequence set forth in SEQ ID NO: 7; and/or a light chain variable region of the HER2-targeted antibody comprises a sequence set forth in SEQ ID NO: 8, a sequence having at least 80% identity to the sequence set forth in SEQ ID NO: 8, or a sequence having one or more conservative amino acid substitutions as compared to the sequence set forth in SEQ ID NO: 8;
or a heavy chain of the HER2-targeted antibody comprises a sequence set forth in SEQ ID NO: 9, a sequence having at least 80% identity to the sequence set forth in SEQ ID NO: 9, or a sequence having one or more conservative amino acid substitutions as compared to the sequence set forth in SEQ ID NO: 9; and/or a light chain of the HER2-targeted antibody comprises a sequence set forth in SEQ ID NO: 10, a sequence having at least 80% identity to the sequence set forth in SEQ ID NO: 10, or a sequence having one or more conservative amino acid substitutions as compared to the sequence set forth in SEQ ID NO: 10; or, the HER2-targeted antibody has a fucosylation level of 0%-5%, and the antibody comprises no less than 60% of G0;
or, the HER2-targeted antibody is pertuzumab.

**10.** The method according to claim 9, wherein the HER2-targeted antibody has a content of G0-GN glycoform of 3%-7%, a content of G1 glycoform of 6%-12%, a content of G1' glycoform of 6%-12%, and a content of G2 glycoform of no more than 3%; or the HER2-targeted antibody is an antibody with enhanced ADCC.

**11.** The method according to any one of claims 8-10, wherein the antibody-drug conjugate and the second drug are

administered to the patient sequentially in any order, or simultaneously; or, the administration mode is injection; or the administration mode is intravenous injection, subcutaneous injection, or intraperitoneal injection; or the administration mode is intravenous infusion or subcutaneous injection.

**12.** The method according to any one of claims 9-12, wherein the second drug is administered in a cycle of one dose every 1, 2, 3, 4, 5, 6, or 7 weeks at a dose of 1-15 mg/kg each time; or, the second drug is administered in a cycle of one dose every 2 weeks or every 3 weeks at a dose of 3-15 mg/kg each time; or the second drug is administered in a cycle of one dose every 1, 2, 3, 4, 5, 6, or 7 weeks at a dose of 50-1000 mg each time; or, the second drug is administered in a cycle of one dose every three weeks at a first dose of 840 mg followed by a dose of 420 mg each time

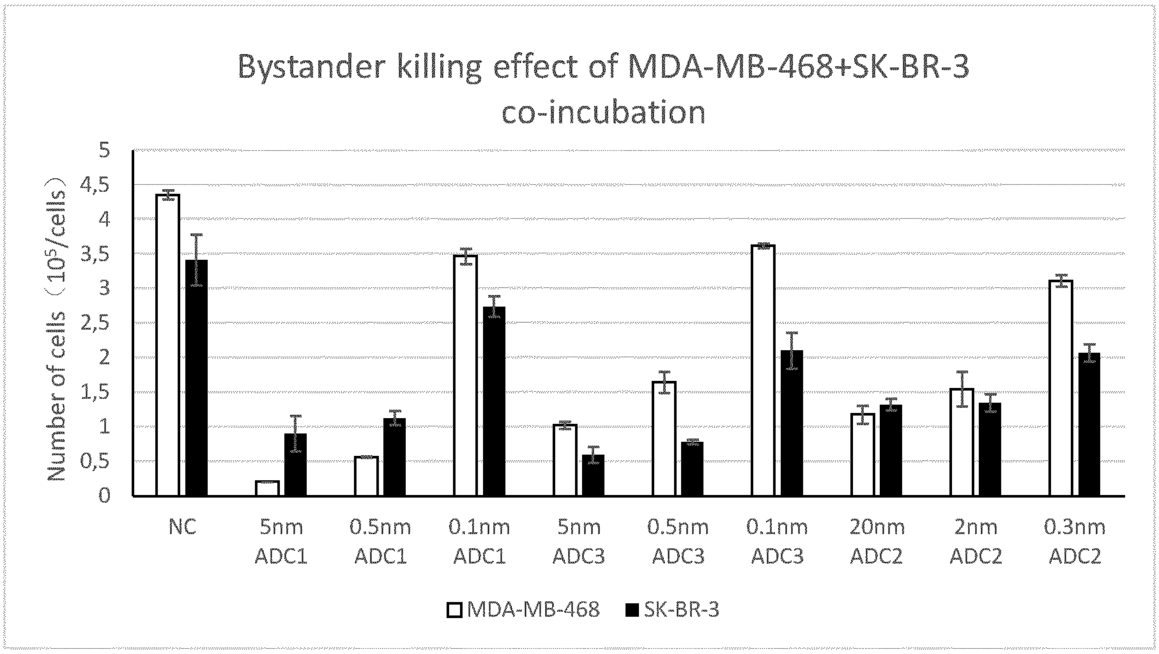
Bystander killing effect of MDA-MB-468+SK-BR-3 co-incubation
Number of cells （10⁵/cells）
5
4,5
4
3,5
3
2,5
2
1,5
1
0,5
0
NC
5nm ADC1
0.5nm ADC1
0.1nm ADC1
5nm ADC3
0.5nm ADC3
0.1nm ADC3
20nm ADC2
2nm ADC2
0.3nm ADC2
MDA-MB-468
SK-BR-3

FIG. 1

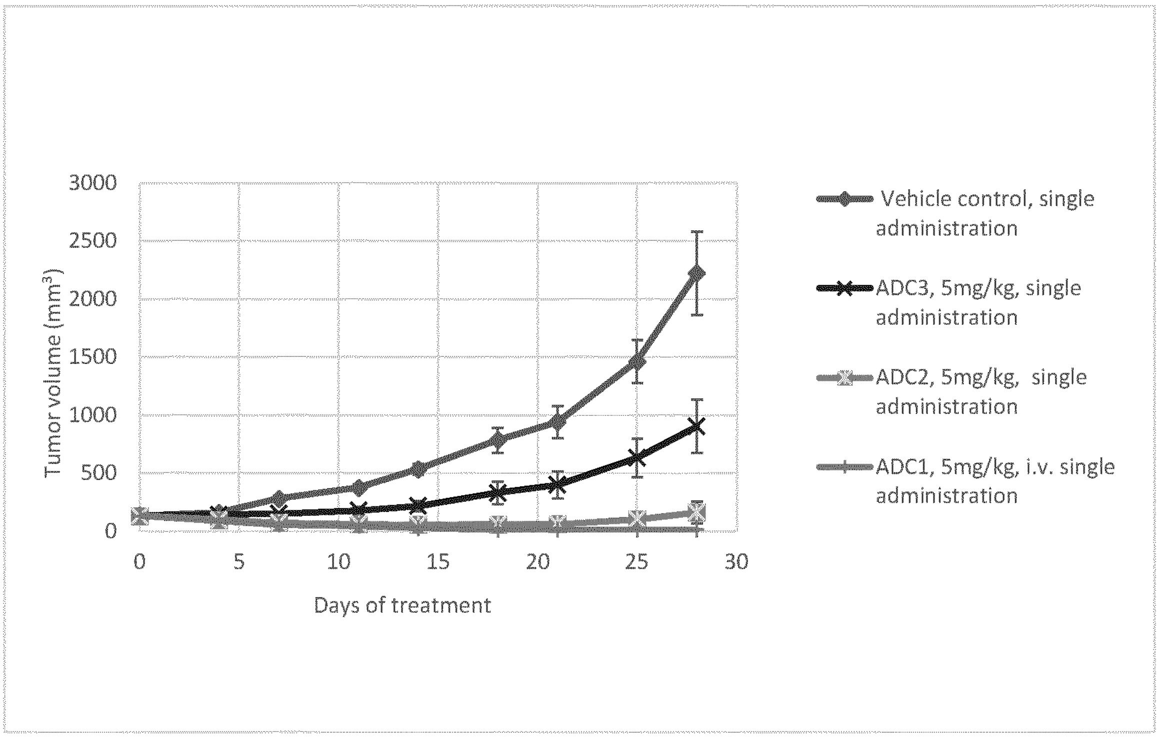
3000
2500
2000
1500
1000
500
0
Tumor volume (mm³)
0
5
10
15
20
25
30
Days of treatment
Vehicle control, single administration
ADC3, 5mg/kg, single administration
ADC2, 5mg/kg, single administration
ADC1, 5mg/kg, i.v. single administration

FIG. 2

**INTERNATIONAL SEARCH REPORT**

International application No.
**PCT/CN2023/087691**

**A. CLASSIFICATION OF SUBJECT MATTER**

A61K47/60(2017.01)i； A61K47/68(2017.01)i； A61K47/65(2017.01)i； C07K16/00(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： A61K C07K A61P

Documentation searched other than minimum documentation to the extent that such documents are included in the fields searched

Electronic data base consulted during the international search (name of data base and, where practicable, search terms used)

CNTXT; DWPI; ENTXTC; PUBMED; ELSEVIER; Patentics; NCBI; Web of Science; STN; 中国专利生物序列检索系统, China Patent Biological Sequence Search System: 百奥泰, 抗体偶联, 连接子, 喜树碱, 聚乙二醇, structural formula search, 序列检索, sequence search, ADC, antibody drug conjudate, linker, Camptothecin, CPT, PEG

**C. DOCUMENTS CONSIDERED TO BE RELEVANT**

| Category* | Citation of document, with indication, where appropriate, of the relevant passages | Relevant to claim No. |
|---|---|---|
| PX | CN 115429893 A (BIO-THERA SOLUTIONS, LTD.) 06 December 2022 (2022-12-06) claims 1-5 | 1-12 |
| X | US 2021093733 A1 (OBI PHARMA INC.) 01 April 2021 (2021-04-01) claims 53-61, description, paragraphs [0309]-[0329] | 1-12 |
| X | WO 2019243825 A1 (CURADEV PHARMA LTD.) 26 December 2019 (2019-12-26) description, pages 128, 133 | 1-12 |
| A | CN 108883198 A (EISAI INC.) 23 November 2018 (2018-11-23) description, paragraphs [0007]-[0031] | 1-12 |
| A | CN 113853219 A (NOVARTIS AG) 28 December 2021 (2021-12-28) claims 1-48 | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 June 2023** | **20 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/087691**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No.
**PCT/CN2023/087691**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **1-12**
because they relate to parts of the international application that do not comply with the prescribed requirements to such an extent that no meaningful international search can be carried out, specifically:

The subject matter of claims 1-12 is: a method for treating HER2-positive solid tumors, which relates to a treatment method, and thus falls within subject matter for which no search is required by the International Searching Authority (PCT Rule 39.1(4)).

With regard to claims 1-12, the reasonably expected amendment is made as follows:

the use of an antibody-drug conjugate in preparation of a drug for treating HER2-positive solid tumors.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/087691**

| Patent document cited in search report | Publication date (day/month/year) | Patent family member(s) | Publication date (day/month/year) |
|---|---|---|---|
| CN 115429893 A | 06 December 2022 | WO 2022253284 A1 | 08 December 2022 |
| US 2021093733 A1 | 01 April 2021 | None | |
| WO 2019243825 A1 | 26 December 2019 | TW 202016081 A | 01 May 2020 |
| CN 108883198 A | 23 November 2018 | JOP 20210074 A1 | 30 January 2023 |
| | | UA 125024 C2 | 29 December 2021 |
| | | TW 202241524 A | 01 November 2022 |
| | | CA 3013791 A1 | 08 September 2017 |
| | | JP 2020019787 A | 06 February 2020 |
| | | JP 6870051 B2 | 12 May 2021 |
| | | EP 3824909 A1 | 26 May 2021 |
| | | US 2017252458 A1 | 07 September 2017 |
| | | US 10548986 B2 | 04 February 2020 |
| | | US 2020297860 A1 | 24 September 2020 |
| | | SG 10202007520 WA | 29 September 2020 |
| | | IL 261428 A | 31 October 2018 |
| | | LT 3423105 T | 10 September 2021 |
| | | AR 121302 A2 | 04 May 2022 |
| | | CO 2018008667 A2 | 31 August 2018 |
| | | SG 11201806515 RA | 27 September 2018 |
| | | BR 112018067379 A2 | 15 January 2019 |
| | | RS 62108 B1 | 31 August 2021 |
| | | KR 20220101204 A | 19 July 2022 |
| | | KR 20180115330 A | 22 October 2018 |
| | | KR 102445255 B1 | 22 September 2022 |
| | | HUE 054726 T2 | 28 September 2021 |
| | | CL 2021000048 A1 | 28 May 2021 |
| | | JP 2020128413 A | 27 August 2020 |
| | | TW 201800110 A | 01 January 2018 |
| | | TWI 772288 B | 01 August 2022 |
| | | HRP 20211125 T1 | 15 October 2021 |
| | | JP 2019516664 A | 20 June 2019 |
| | | JP 6599019 B2 | 30 October 2019 |
| | | ES 2880402 T3 | 24 November 2021 |
| | | JOP 20210073 A1 | 30 January 2023 |
| | | PL 3423105 T3 | 25 October 2021 |
| | | MD 3423105 T2 | 31 October 2021 |
| | | EP 3423105 A1 | 09 January 2019 |
| | | EP 3423105 B1 | 05 May 2021 |
| | | US 2018193478 A1 | 12 July 2018 |
| | | US 10322192 B2 | 18 June 2019 |
| | | IL 292946 A | 01 July 2022 |
| | | AR 107787 A1 | 06 June 2018 |
| | | WO 2017151979 A1 | 08 September 2017 |
| | | JOP 20170053 B1 | 17 August 2021 |
| | | SI 3423105 T1 | 31 December 2021 |
| | | AU 2017225982 A1 | 16 August 2018 |
| | | PH 12018501847 A1 | 15 May 2019 |
| | | PE 20181953 A1 | 17 December 2018 |
| | | AR 121301 A2 | 04 May 2022 |
| | | DK 3423105 T3 | 26 July 2021 |

Form PCT/ISA/210 (patent family annex) (July 2022)

**INTERNATIONAL SEARCH REPORT**
**Information on patent family members**

International application No.
**PCT/CN2023/087691**

| Patent document cited in search report | Publication date (day/month/year) | Patent family member(s) | Publication date (day/month/year) |
|---|---|---|---|
| | | MX 2018010562 A | 20 February 2019 |
| | | CL 2018002456 A1 | 21 December 2018 |
| | | KR 20220101203 A | 19 July 2022 |
| | | KR 102456433 B1 | 19 October 2022 |
| | | RU 2018134331 A | 02 April 2020 |
| | | RU 2018134331 A3 | 14 August 2020 |
| | | RU 2754369 C2 | 01 September 2021 |
| | | PT 3423105 T | 19 July 2021 |
| | | JP 2021185176 A | 09 December 2021 |
| | | JP 7254861 B2 | 10 April 2023 |
| | | MA 45280 A | 09 January 2019 |
| | | MA 45280 B1 | 31 August 2021 |
| | | CL 2021000049 A1 | 28 May 2021 |
| | | RU 2021125492 A | 05 April 2022 |
| CN 113853219 A | 28 December 2021 | IL 287596 A | 01 December 2021 |
| | | KR 20220010527 A | 25 January 2022 |
| | | AU 2020279731 A1 | 06 January 2022 |
| | | CA 3140063 A1 | 26 November 2020 |
| | | WO 2020236841 A2 | 26 November 2020 |
| | | WO 2020236841 A3 | 14 January 2021 |
| | | JP 2022533215 A | 21 July 2022 |
| | | US 2023091510 A1 | 23 March 2023 |
| | | EP 3972650 A2 | 30 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022253284 A
- WO 2019029713 A


### Non-patent literature cited in the description


- **ALLEN, T.M.** ; **CULLIS, P.R.** *Science*, 2004, vol. 303 (5665), 1818-22
- **HU, Q.Y et al.** *Chem Soc Rev*, 2016, vol. 45 (6), 1691-1719
- **THOMAS, A et al.** *Lancet Oncol*, 2016, vol. 17 (6), e254-e262
- **CHARI, R.V.** *Acc Chem Res*, 2008, vol. 41 (1), 98-107
- **SINGH, S.K. et al.** *Pharm Res*, 2015, vol. 32 (11), 3541-3571
- **HAMILTON, G.S.** *Biologicals*, 2015, vol. 43 (5), 318-332
- **OUYANG**. *J. Methods Mol Biol*, 2013, vol. 1045, 275-83
- McGraw-Hill Dictionary of Chemical Terms. McGraw-Hill, 1985
- **WOOD et al.** *J Immunol.*, 1990, vol. 145, 3011
- *CHEMICAL ABSTRACTS*, 143655-49-6